# EUROPEAN PATENT SPECIFICATION

(11) **EP 4 265 608 B1**
(45) Date of publication and mention of the grant of the patent: **17.06.2026**
(21) Application number: 21905840.1
(22) Date of filing: 17.12.2021
(51) Int. Cl.: C07D 401/14, C07D 417/14, A61K 31/4725, A61P 9/04, A61P 13/12

(54) **BENZOHETEROCYCLE SUBSTITUTED TETRAHYDROISOQUINOLINE COMPOUND**
BENZOHETEROCYCLISCH SUBSTITUIERTE TETRAHYDROISOCHINOLINVERBINDUNG
COMPOSÉ DE TÉTRAHYDROISOQUINOLÉINE SUBSTITUÉ PAR UN BENZOHÉTÉROCYCLE

(30) Priority: 18.12.2020 CN 202011508096; 11.03.2021 CN 202110266745; 13.12.2021 CN 202111523273
(43) Date of publication of application: 25.10.2023
(73) Proprietor: Shanghai Jeyou Pharmaceutical Co., Ltd., Shanghai 201315 (CN)
(72) Inventor: GUO, Shuchun, Shanghai 201203 (CN); FAN, Jun, Shanghai 201203 (CN); WU, Nan, Shanghai 201203 (CN); FANG, Zhihua, Shanghai 201203 (CN); SHI, Wenqiang, Shanghai 201203 (CN); LIU, Yang, Shanghai 201203 (CN); PENG, Jianbiao, Shanghai 201203 (CN); GUO, Haibing, Shanghai 201203 (CN)
(74) Representative: Haseltine Lake Kempner LLP
(86) International application number: PCT/CN2021/139314
(87) International publication number: WO 2022/127917

(56) References cited:
- EP-A1- 3 351 248
- WO-A1-2018/129556
- WO-A1-2018/129556
- WO-A1-2018/129557
- CN-A- 1 617 856
- CN-A- 102 333 759
- CN-A- 102 333 759
- CN-A- 103 819 403
- CN-A- 104 837 503
- CN-A- 104 837 503
- CN-A- 110 291 082
- CN-A- 110 291 082
- US-A1- 2015 299 131

## Description

The present application claims the right of the following priorities:
application number: CN202011508096.4, filed on December 18, 2020;
application number: CN202110266745.2, filed on March 11, 2021;
application number: CN202111523273.0, filed on December 13, 2021.

### TECHNICAL FIELD

The present disclosure relates to the field of medicinal chemistry, and in particular, relates to benzoheterocycle substituted tetrahydroisoquinoline compounds and use thereof.

### BACKGROUND

Phosphate is an important mineral that regulates various metabolic processes including signal transduction, energy production, and mineral metabolism, and is primarily absorbed in the small intestine and filtered by the kidneys, and subsequently reabsorbed or excreted via the renal tubules. Thus, serum phosphate concentrations remain within a physiological range despite differences in daily phosphate intake. In patients with advanced chronic kidney disease (CKD), the renal phosphorus metabolism function is basically lost, which leads to hyperphosphatemia. Studies have shown that hyperphosphatemia is associated with multiple adverse clinical outcomes in CKD patients, including: induction of vascular calcification, increased incidence and risk of death of cardiovascular disease, secondary hyperparathyroidism, metabolic bone disease or ectopic calcification resulted from renal osteodystrophy, promotion of renal failure and progression of cardiovascular disease.

Currently, the main treatment measures for hyperphosphatemia are low phosphate diet, hemodialysis treatment and taking phosphate binder drugs with meals. Clinical experience shows that it is difficult to control phosphate intake through diet; the efficiency of hemodialysis is limited; therefore, the use of phosphate binder drugs is an important treatment method for lowering serum phosphorus currently. At present, there are two main kinds of phosphate binder drugs commonly used in clinic: phosphate binders containing metal ions (calcium/magnesium/iron/lanthanum) and ion-exchange resin-type binders (sevelamer or sevelamer carbonate). For the former phosphate binders containing metal ions, patients need to strengthen the management of metal ions in the drug, and the drug is affected by pH and has a weak phosphorus binding effect, which is prone to cause diarrhea and is not tolerated by patients. The latter binding phosphorus through ion exchange is not absorbed by the gastrointestinal tract which can reduce accumulation, and has fewer side effects than the former. However, the dosage of the two kinds of drugs is large and the price of which is high, the patient's compliance is poor.

It is known that there are two main ways for the intestinal tract to absorb phosphate: passive cell bypass transport and active transport dependent on transfer proteins, and passive cell bypass phosphate transport is considered to be the main reason of phosphate absorption in humans. The cell bypass phosphate transport is primarily driven by a concentration gradient of phosphate, which is absorbed by tight junction complex formed between cells, and it has been shown in the literature that such tight conjunction complex has permeability specificity for specific ions through the regulation of signal transduction. Sodium-hydrogen antiporter 3 (NHE3/SLC9A3) is a kind of gastrointestinal transfer protein expressed in the apical part of intestinal epithelial cells and mainly responsible for maintaining sodium ion balance, which can affect sodium absorption in the intestinal tract by inhibiting NHE3 activity in the intestinal tract, thus altering the concentration of hydrogen ions in the intestinal epithelial cells and thus affecting the change of local pH; decrease the permeability of the tight junction complex formed between cells to phosphate and reduce phosphate absorption by cell bypass. In clinical practice, the need for serum phosphorus control in patients with advanced CKD has not been met, so it is necessary to further develop drugs with different mechanisms to lower serum phosphorus.

Among the drugs marketed in our country, the diagnostic method for hyperphosphatemia in CKD patients is relatively single; therefore, it is necessary to further develop more effective and safer drugs to lower serum phosphorus levels.

EP 3 351 248 A1, US 2015/299131 A1, WO 2018/129557 A1, and WO 2018/129556 A1 all describe compounds that inhibit NHE-mediated antiport and methods for the treatment of disorders associated with fluid retention or salt overload, such as chronic kidney disease, and end-stage renal disease.

### CONTENT OF THE PRESENT INVENTION

The present invention is defined in the appended claims.

In one aspect of the present disclosure, the present disclosure provides a compound represented by formula (I), a stereoisomer thereof and a pharmaceutically acceptable salt thereof, 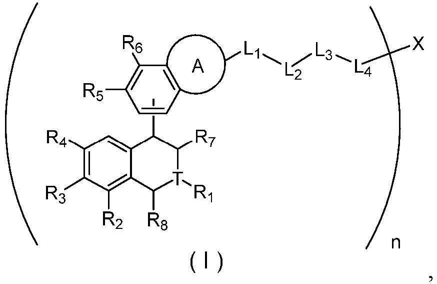 wherein,
R₁ is selected from CH₃, 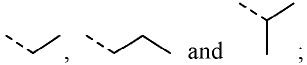
R₂, R₃, R₄ are each independently selected from H, F, Cl, Br, OH, NH₂, CN, CH₃, 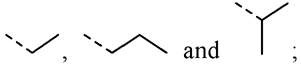
R₇, R₈ are each independently selected from H;
T is selected from N and CH;
the structural moiety 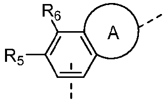 is selected from 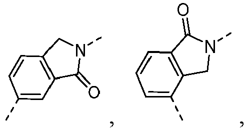 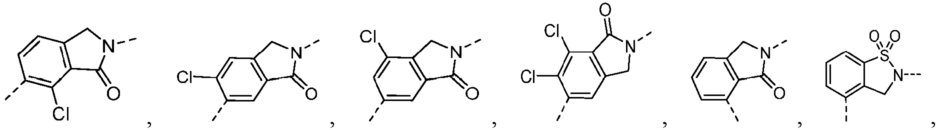 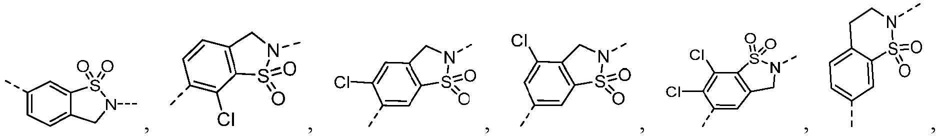 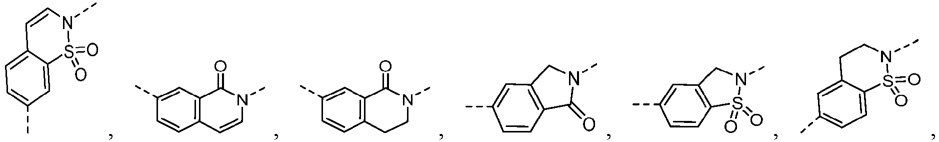 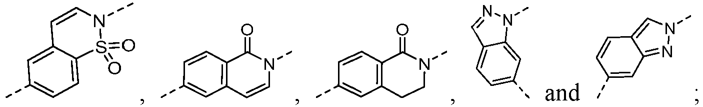
n is selected from 2 and 3;
the structural unit 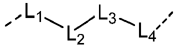 is selected from 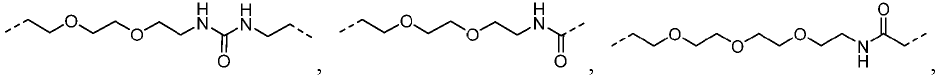 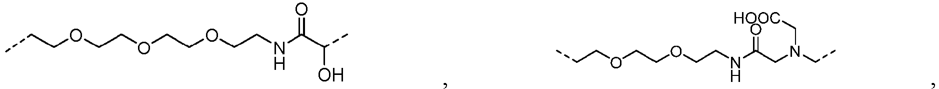 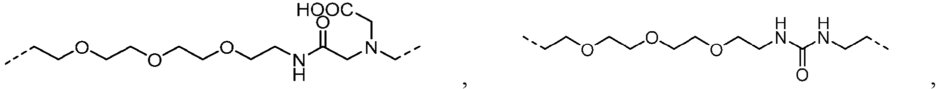 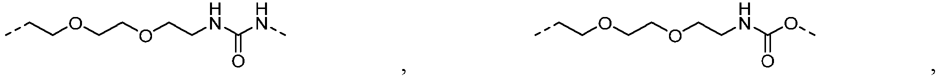 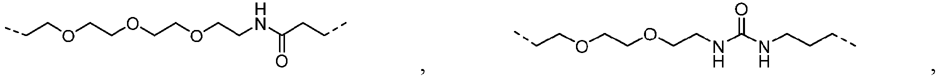 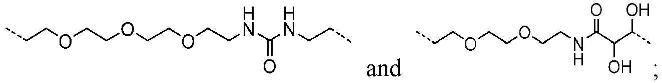
X is selected from a single bond, N, NH, O,

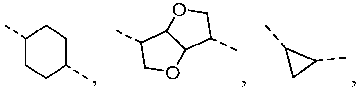

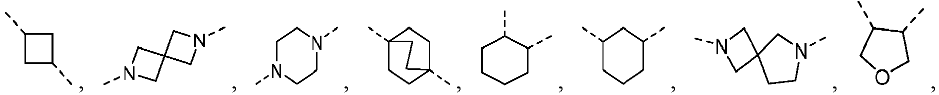

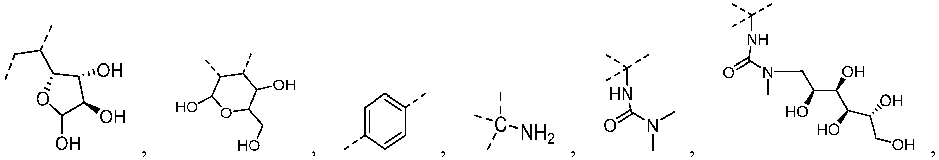

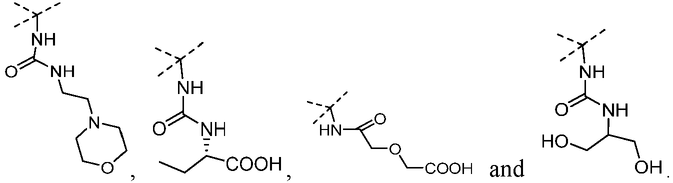

In another aspect of the present disclosure, the present disclosure also provides a compound represented by the following formula, a stereoisomer thereof and a pharmaceutically acceptable salt thereof, selected from:

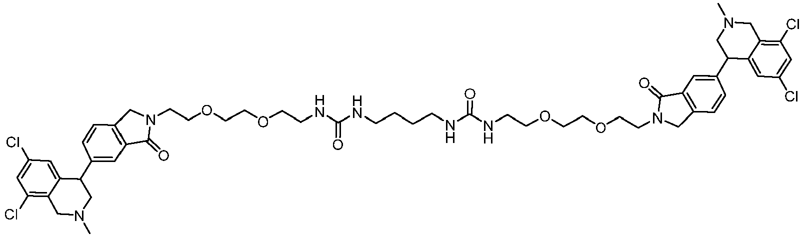

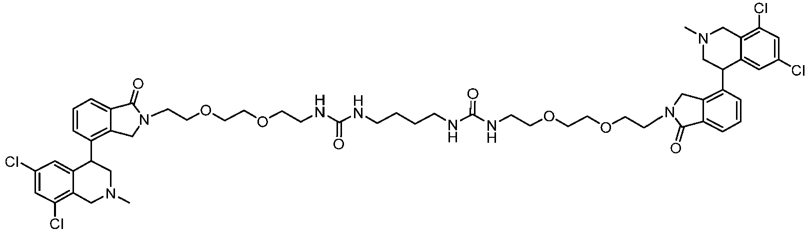

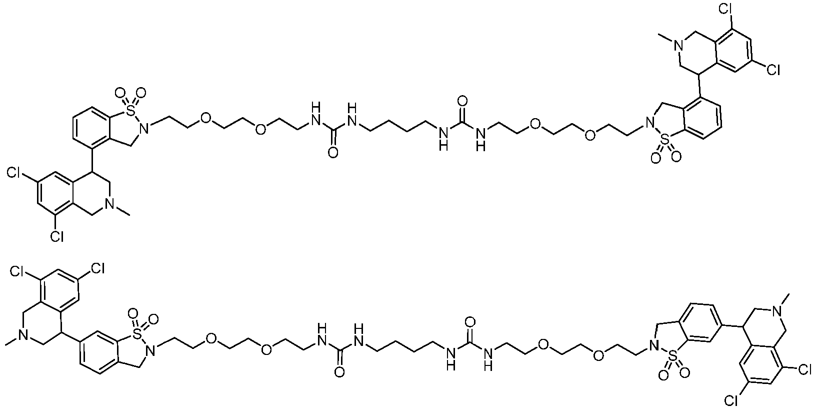

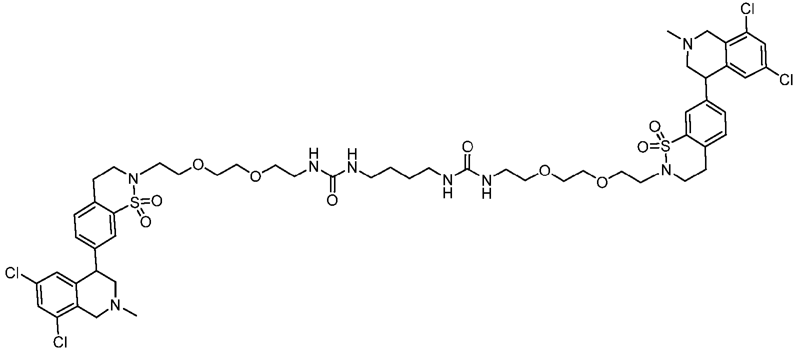

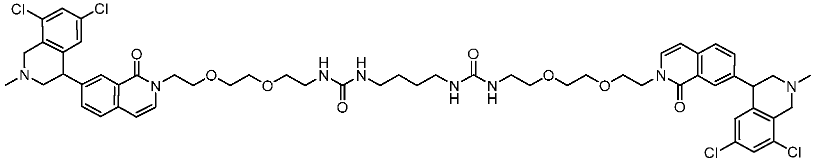

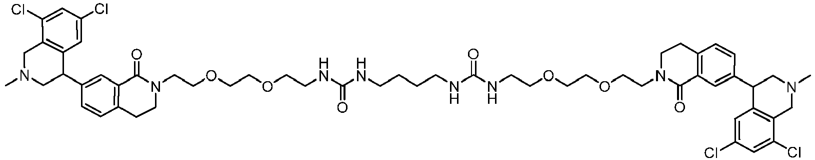

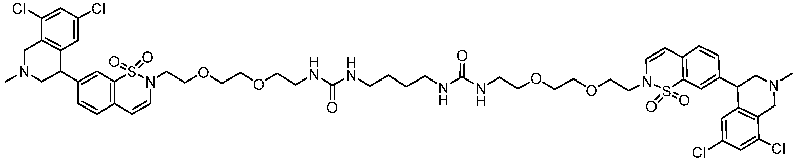

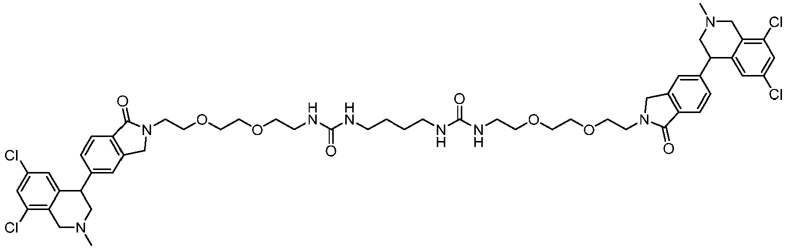

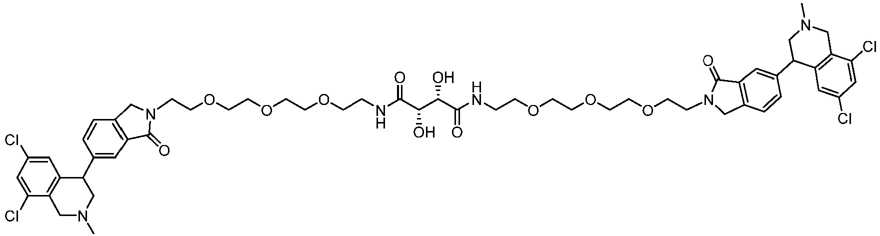

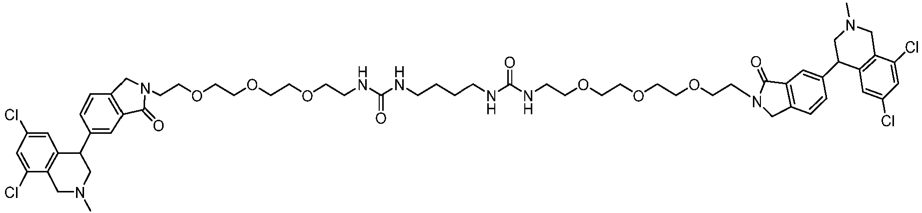

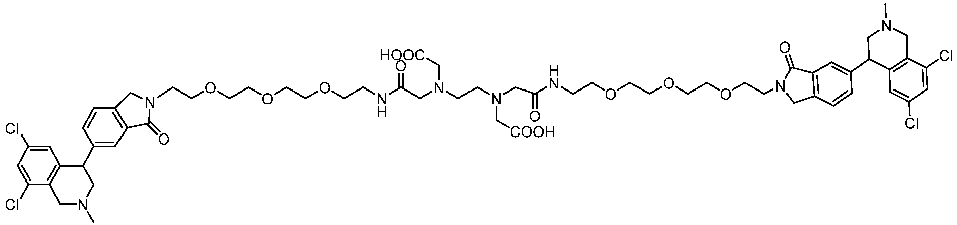

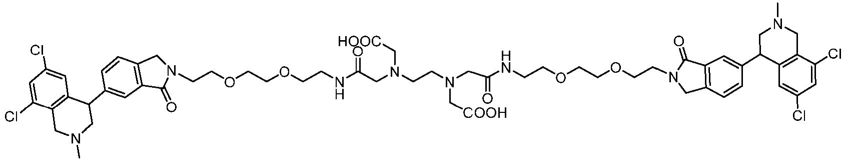

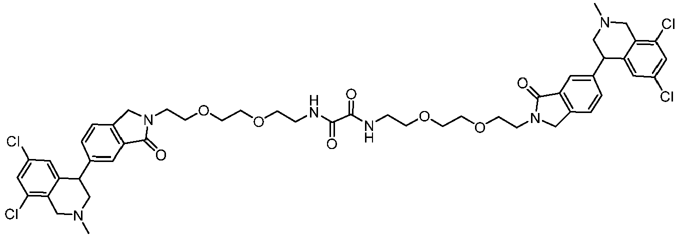

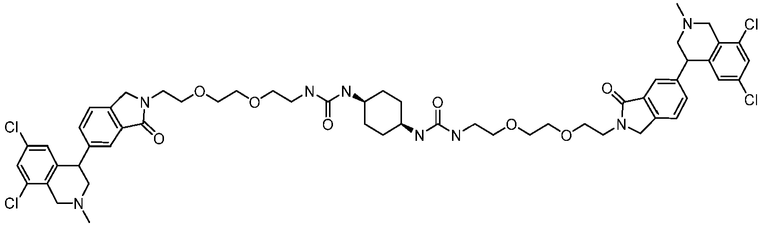

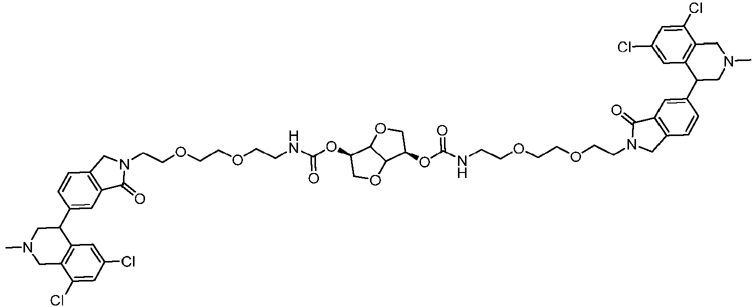

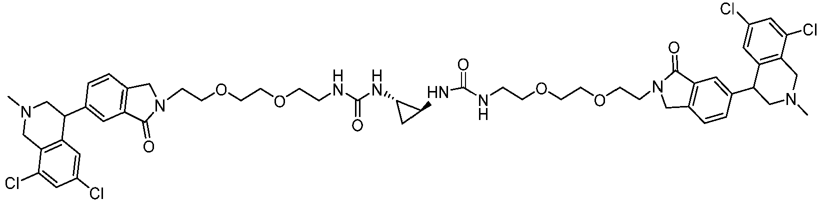

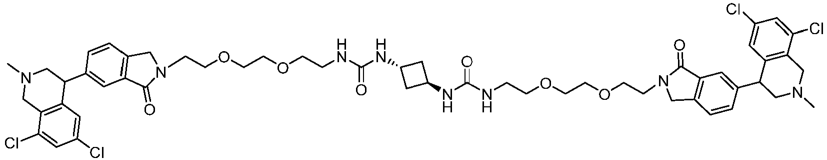

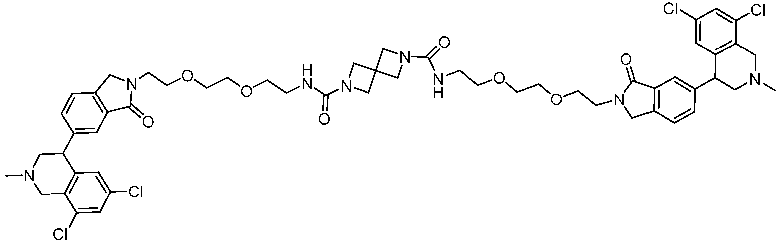

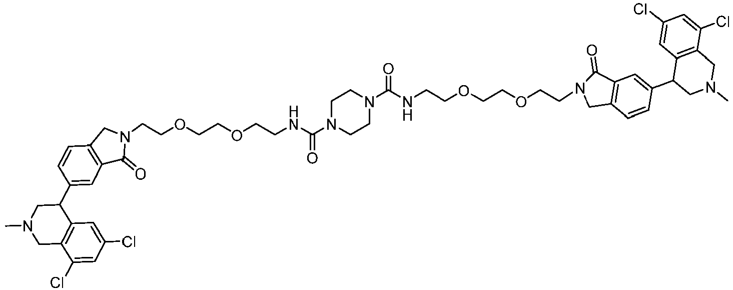

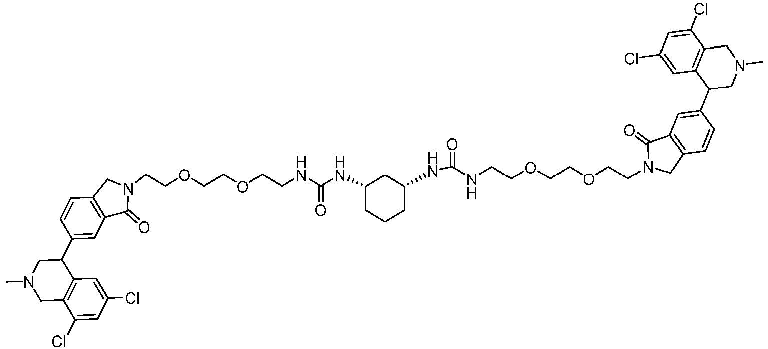

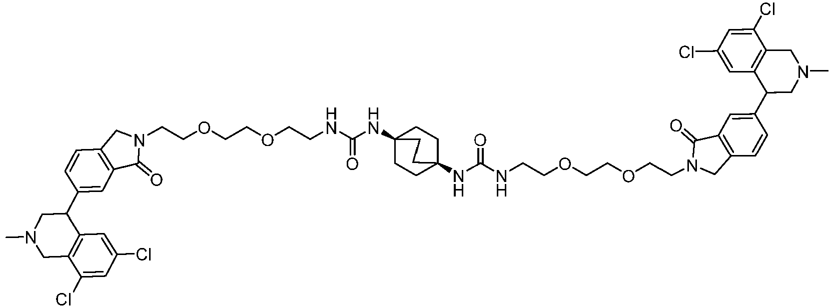

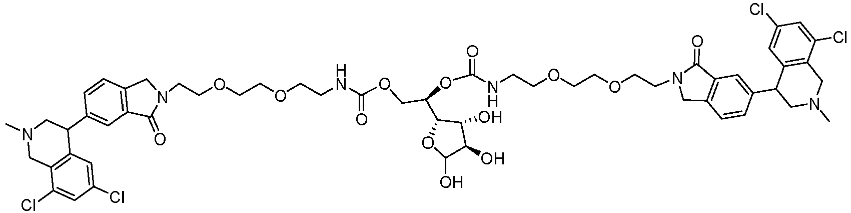

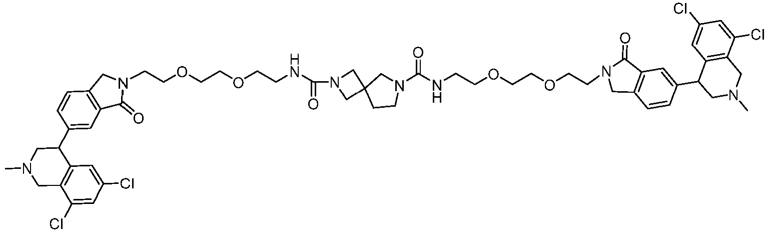

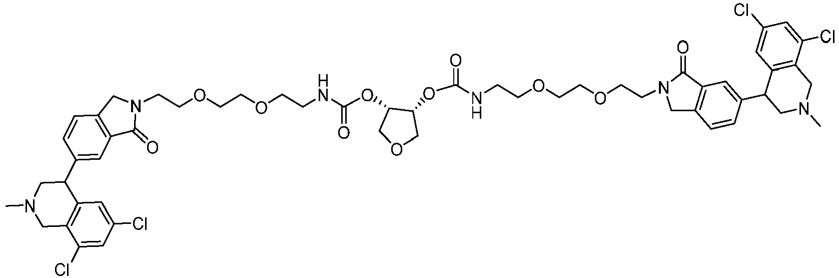

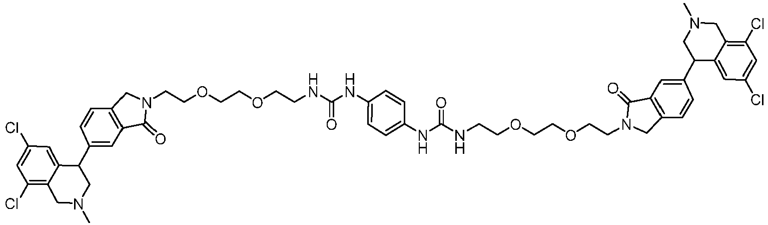

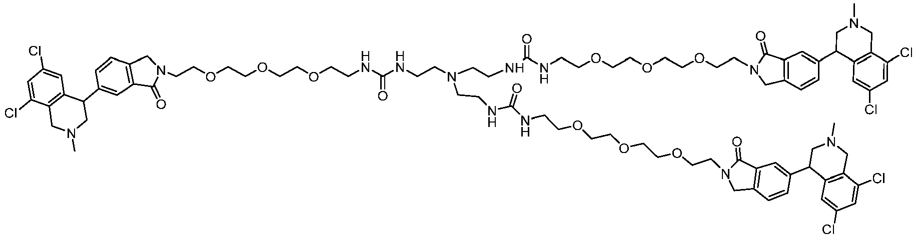

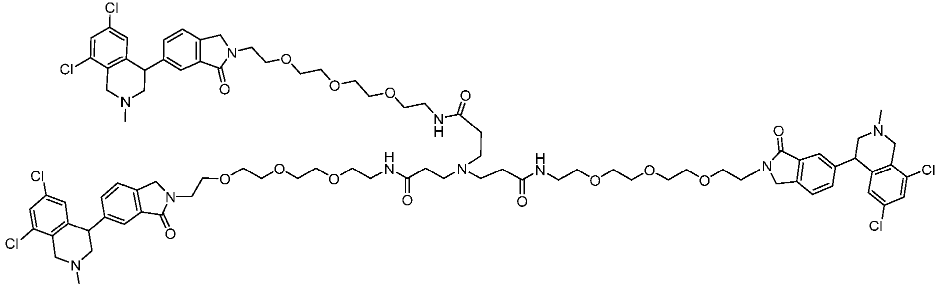

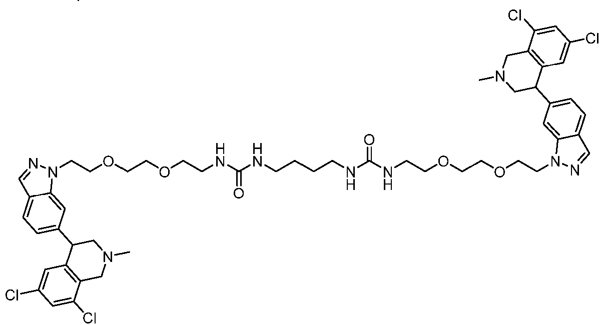

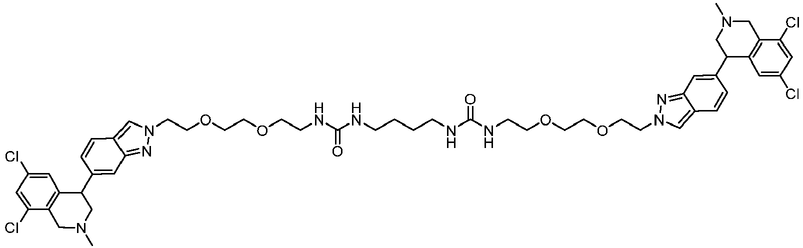

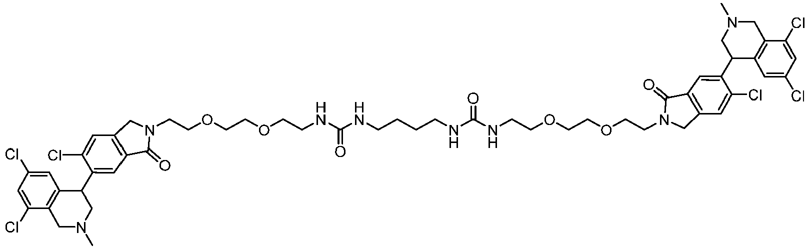

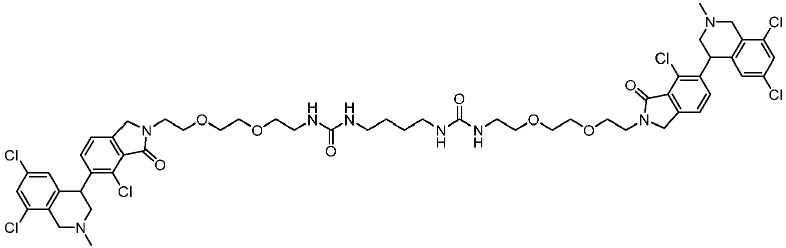

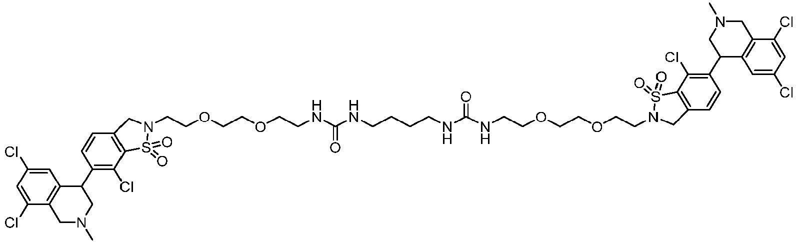

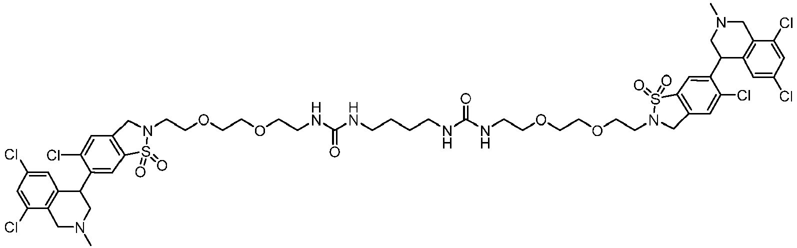

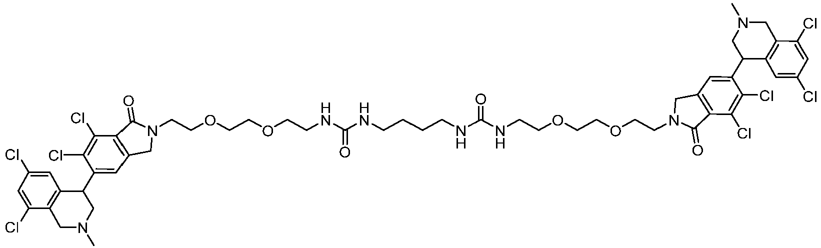

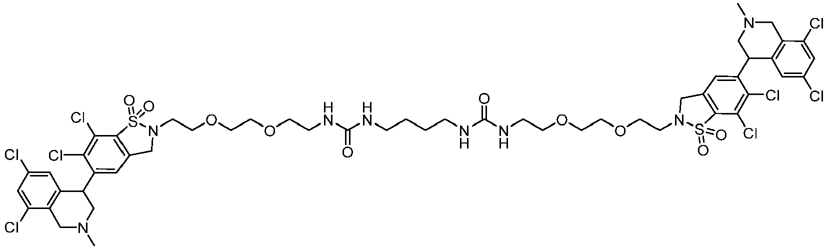

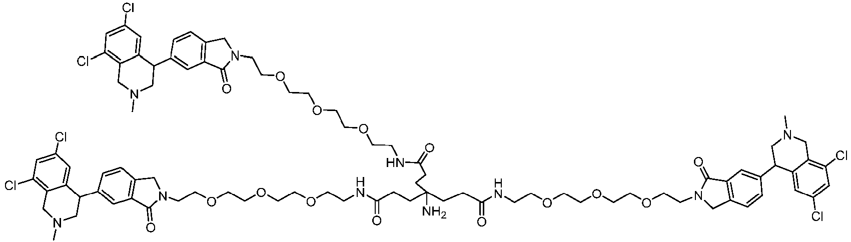

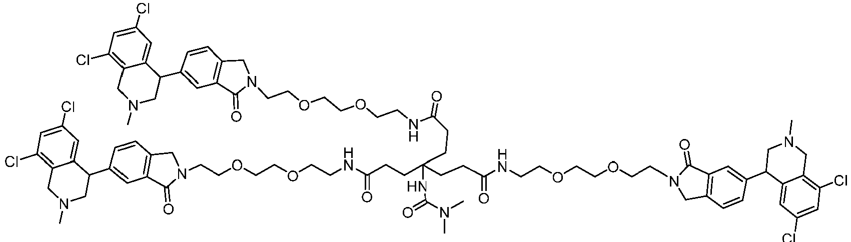

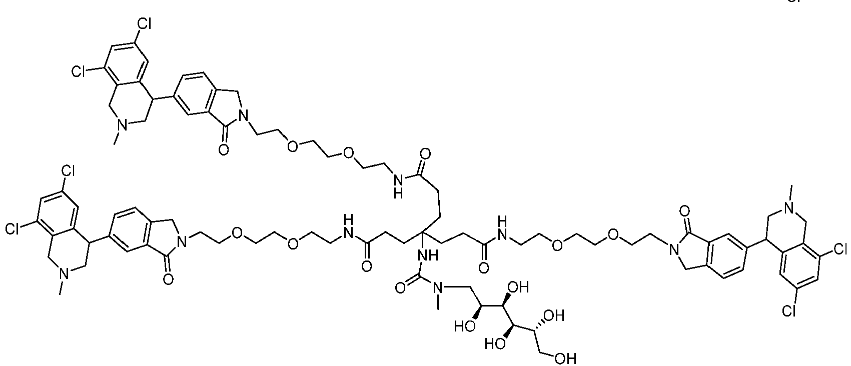

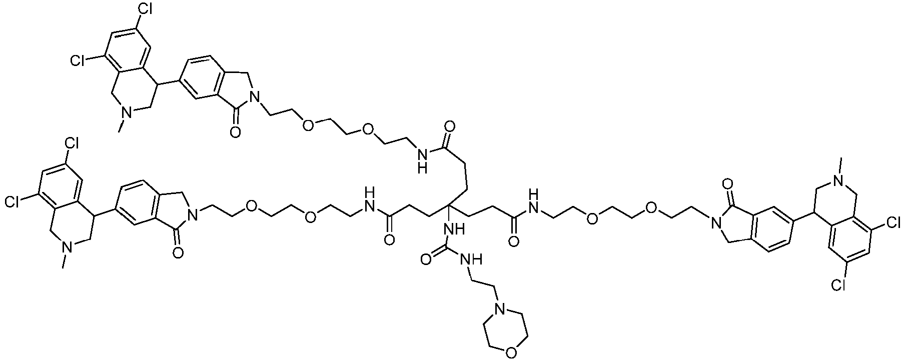

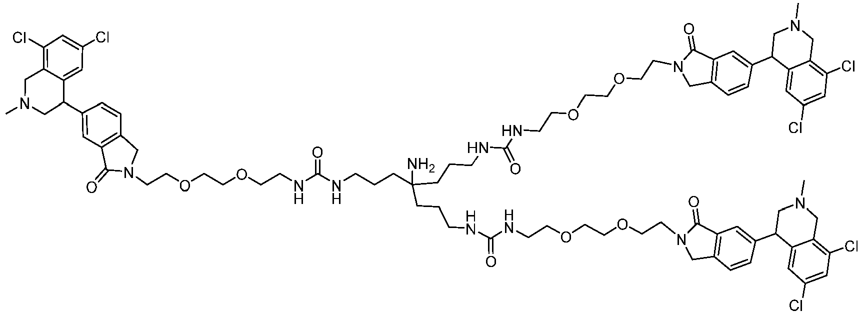

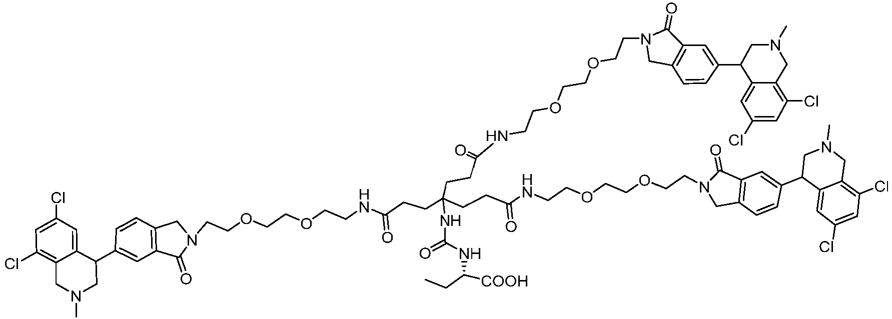

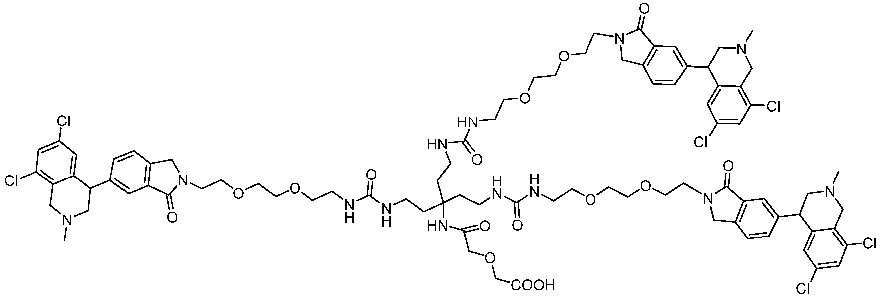

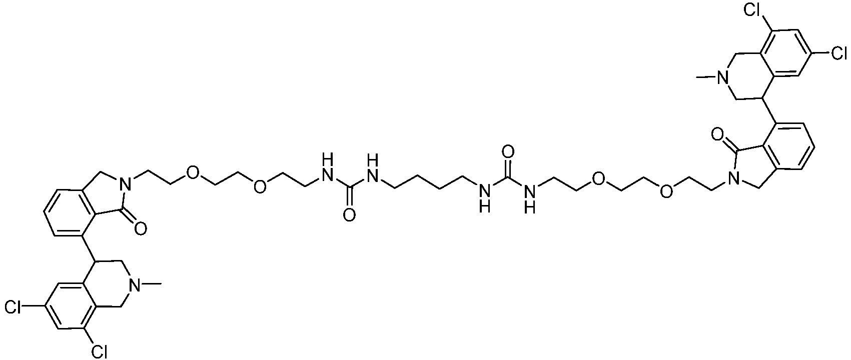

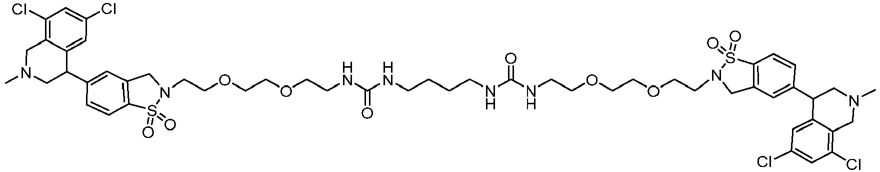

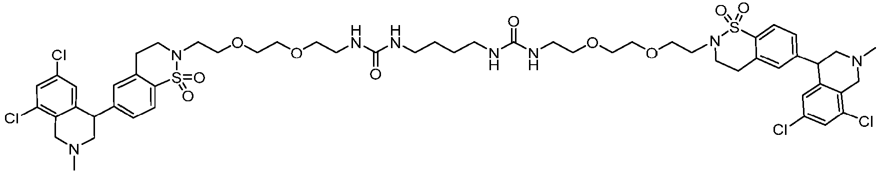

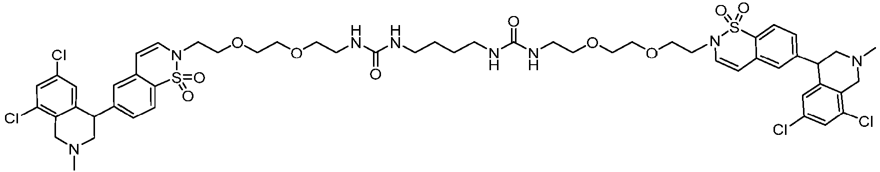

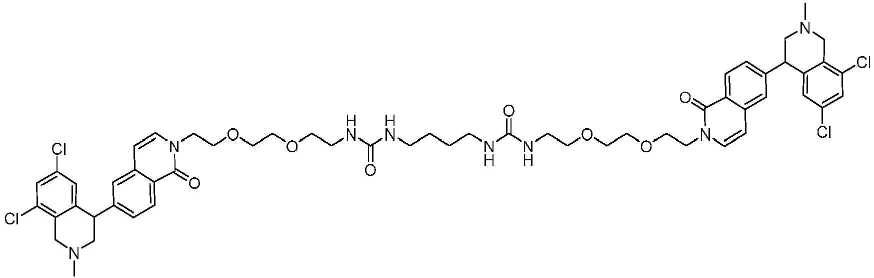

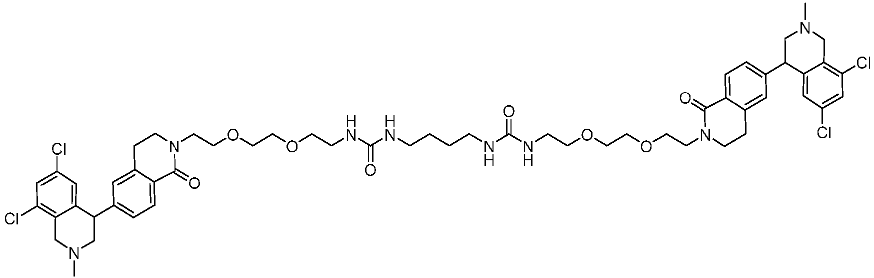

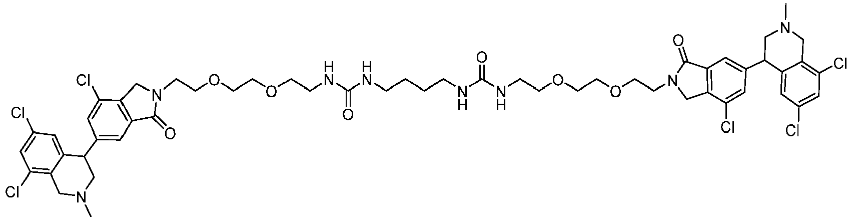

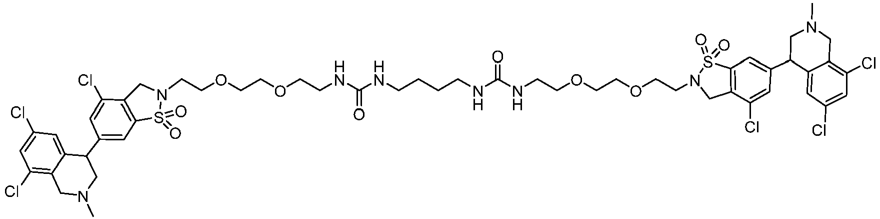

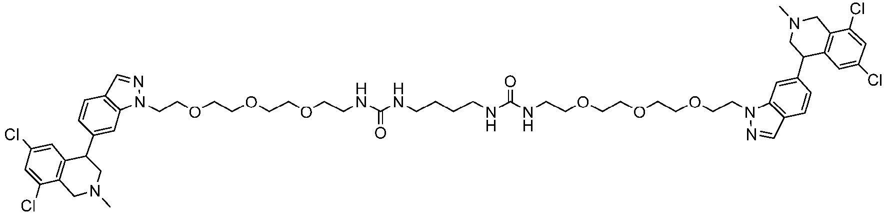

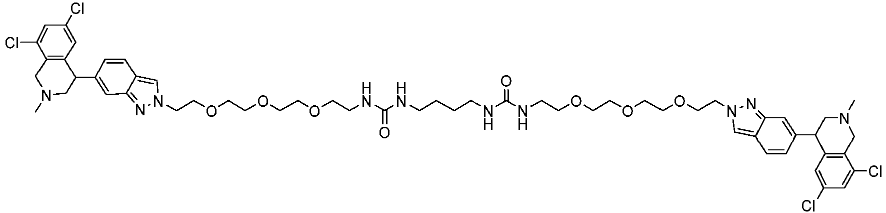

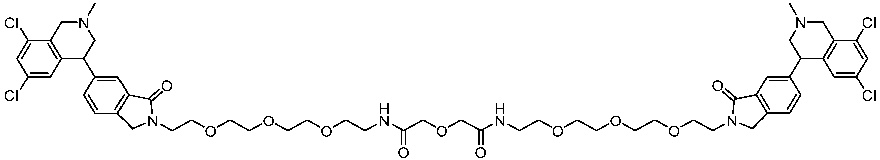

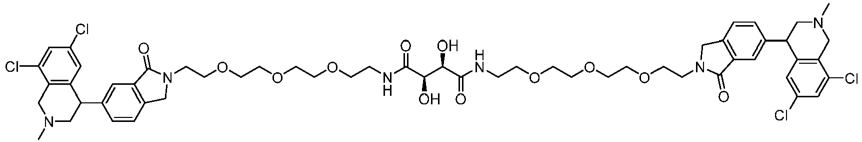

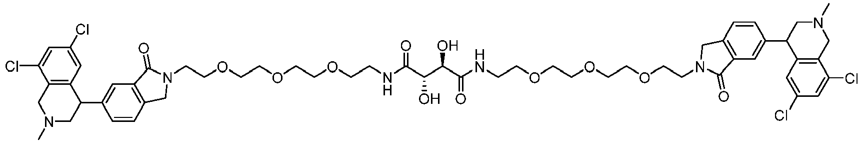

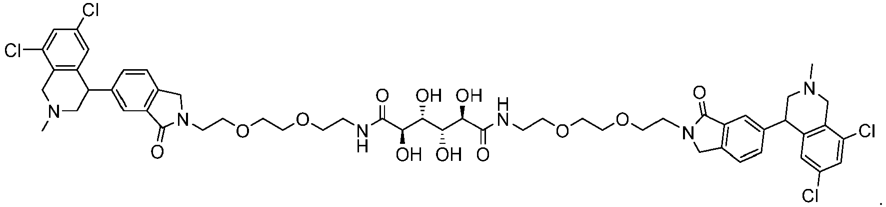

In another aspect of the present disclosure, the present disclosure also provides a use of the above compound, a stereoisomer thereof and a pharmaceutically acceptable salt thereof in the preparation of a medicament for treating irritable bowel syndrome, heart failure, chronic kidney disease, end-stage renal disease or liver disease.

### Definition and description

Unless otherwise specified, the following terms and phrases used herein have the following meanings. A specific term or phrase should not be considered indefinite or unclear in the absence of a particular definition, but should be understood according to the common meaning. When a trade name appears herein, it is intended to refer to its corresponding commercial product or active ingredient thereof.

The term "pharmaceutically acceptable" is used herein in terms of those compounds, materials, compositions, and/or dosage forms, which are suitable for use in contact with human and animal tissues within the scope of reliable medical judgment, without excessive toxicity, irritation, anaphylactic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt can be obtained by bringing the neutral form of the compound into contact with a sufficient amount of acid in a solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, trifluoroacetic acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific stereoisomeric forms. The present disclosure contemplates all such compounds, including (-)-and (+)-enantiomers, (R)-and (*S*)-enantiomers, diastereoisomers, (D)-isomers, (L)-isomers, and racemic and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound. For example, 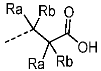 can be selected from 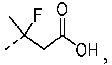 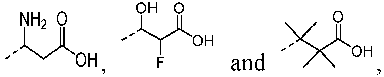 and the like.

A dash ("-") that is not between two letters or symbols indicates the connection site of the substituent. For example, C₁₋₆ alkylcarbonyl- refers to a C₁₋₆ alkyl that is attached to the rest of the molecule through a carbonyl. However, when the connection site of the substituent is obvious to those skilled in the art, for example, a halogen substituent, the "-" may be omitted.

Unless otherwise indicated, when the valence bond of a group is marked with a dashed line " 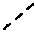 ", for example, in 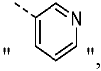 the dashed line indicates the connection site between the group and the other part of the molecule.

When one of the variables is selected from a single bond, it means that the two groups connected are directly connected, for example, when L₁ in 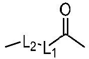 represents a single bond, it means that the structure is actually

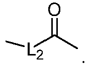

When the enumerative substituent does not indicate by which atom it is linked to the group to be substituted, such substituent can be bonded by any atom thereof, for example, when pyridyl acts as a substituent, it can be linked to the group to be substituted by any carbon atom on the pyridine ring.

When the enumerative linking group does not indicate the direction for linking, the direction for linking is arbitrary, for example, the linking group L in 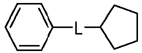 is 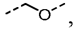 then 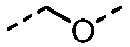 can link phenyl and cyclopentyl to form 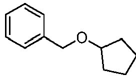 in the direction same as left-to-right reading order, and can also link phenyl and cyclopentyl to form 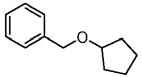 in the direction contrary to left-to-right reading order. A combination of the linking groups, substituents and/or variables thereof is allowed only when such combination can result in a stable compound.

Unless otherwise specified, the number of atoms in a ring is generally defined as the number of ring members, for example, "4-6 membered ring" refers to a "ring" in which 4 to 6 atoms are arranged around.

Those skilled in the art should understand that some compounds of the formula (I) may have one or more chiral centers, so there are two or more stereoisomers. Therefore, the compounds of the present disclosure may exist in the form of a single stereoisomer (e.g., enantiomers, diastereomers) and mixtures thereof in any ratios such as racemate.

The term "stereoisomer" used herein refers to the compound with the same chemical composition but different in the spatial arrangement of atoms or groups. Stereoisomers include enantiomers, diastereomers and conformational isomers.

The term "enantiomer" used herein refers to two stereoisomers of compound that are non-superimposable mirror images of each other.

The term "diastereomer" used herein refers to stereoisomers with two or more chiral centers and the molecules are not mirror images of each other. Diastereomers have different physical properties, such as melting point, boiling point, spectral properties or biological activity. Mixtures of diastereomers can be separated by high-resolution analytical methods such as electrophoresis and chromatography such as HPLC.

The definition and practice of stereochemistry can be followed in S. P. Parker, McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. Many organic compounds exist in optically active form, that is, they have the ability to rotate the plane of plane-polarized light. When describing optically active compounds, the prefixes D and L or R and S are used to indicate the absolute configuration of the molecule with respect to its chiral center. The prefixes d and 1 or (+) and (-) are used to refer to the symbol of the compound rotating plane-polarized light, where (-) or 1 refers to that the compound is levorotatory. Compounds prefixed with (+) or D are dextral. For a given chemical structure, the stereoisomers are the same except that they mirror each other. Specific stereoisomers can also be called enantiomers, and mixtures of such isomers are usually called enantiomer mixtures. The 50:50 mixture of enantiomers is called racemic mixture or racemate, which can occur in the absence of stereoselectivity or stereospecificity in chemical reactions or methods. The terms "racemic mixture" and "racemate" refer to an equimolar mixture of two enantiomers that do not have optical activity.

Racemic mixtures can be used in their own form or split into individual isomers. Through resolution, stereochemically pure compounds or mixtures enriched in one or more isomers can be obtained. Methods for separating isomers are well known (see Allinger n. L. and Eliel E. L., "topics in stereochemistry", Vol. 6, Wiley Interscience, 1971), including physical methods, such as chromatography using chiral adsorbents. Individual isomers in chiral form may be prepared from chiral precursors. Alternatively, the individual isomer may be obtained by chemical separation from the mixture of diastereoisomeric salts formed by chiral acids (e.g., single enantiomer of 10-camphorsulfonic acid, camphoric acid, α-bromocamphoric acid, tartaric acid, diacetyltartaric acid, malic acid, pyrrolidone-5-carboxylic acid, etc.), crystallizing the salt in stages, and then freeing one or two by the separated bases, optionally repeating the process, thereby obtaining one or two isomers substantially free of the other isomer, i.e., the desired stereoisomer with an optical purity of, for example, at least 91%, 92%, 93%, 94%, 95%, 96%, 97%, 98%, 99% or 99.5% by weight. Alternatively, as well known to those skilled in the art, the racemate can be covalently attached to a chiral compound (auxiliary) to obtain diastereomers.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred implementations include but are not limited to the embodiments of the present disclosure.

The technical and scientific terms used herein that are not specifically defined have the meanings commonly understood by those skilled in the art to which the present disclosure belongs.

### BRIEF DESCRIPTION OF THE DRAWINGS

Fig. 1 is a standardized correction value (nP) of urinary phosphorus excretion amount against dietary phosphorus intake amount according to embodiments of the present disclosure;
Fig. 2 is a standardized correction value (nNa) of urinary sodium excretion amount against dietary sodium intake amount according to embodiments of the present disclosure;
Fig. 3 is fecal form score according to embodiments of the present disclosure;
Fig. 4 is a diagram of an experimental procedure according to embodiments of the present disclosure;
Fig. 5 is a serum phosphorus concentration in rats according to embodiments of the present disclosure;
Fig. 6 is a standardized correction value (nP) for 24h urinary phosphorus excretion amount and dietary phosphorus intake amount according to embodiments of the present disclosure;
Fig. 7 is a standardized correction value (nNa) for 24h urinary sodium excretion amount and dietary sodium intake amount according to embodiments of the present disclosure;
Fig. 8 is fecal form score according to embodiments of the present disclosure.

### DETAILED DESCRIPTION OF THE PREFERRED EMBODIMENT

The present application is described in detail by the embodiments below. The present application has been described in detail herein, wherein specific embodiments thereof are also disclosed.

### Synthesis of embodiment A1

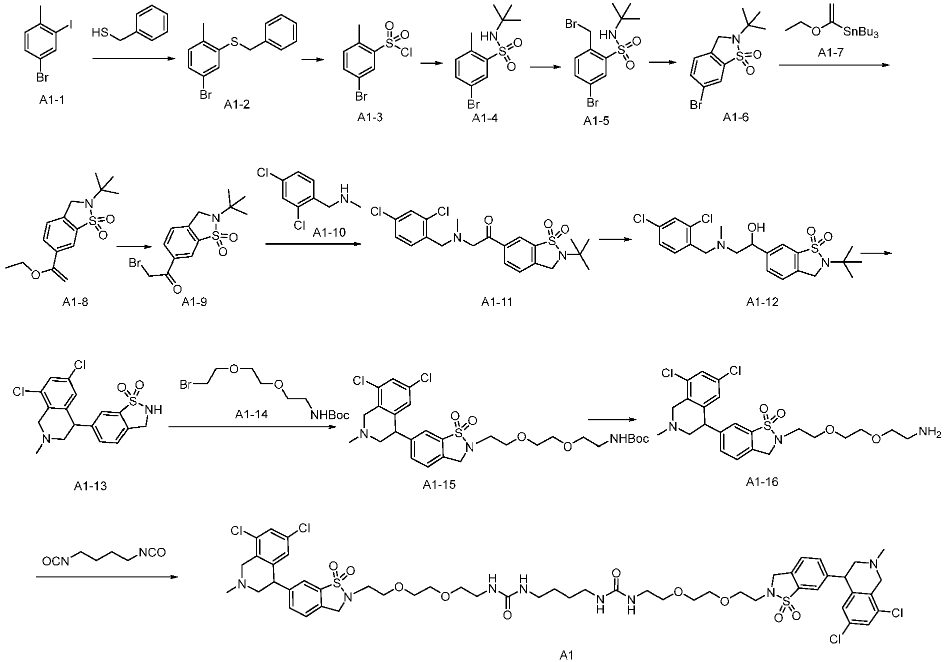

### Step 1: preparation of compound A1-2

Compound A1-1 (6.53 g, 22.0 mmol), tris(dibenzylideneacetone)dipalladium (700 mg, 0.76 mmol), xantphos (700 mg, 1.21 mmol), *N*,*N*-diisopropylethylamine (3.87 g, 30.0 mmol) were dissolved in 1,4-dioxane (50 mL) solution, then the system was heated under nitrogen atmosphere to 70°C, and the compound benzyl mercaptan (2.48 g, 20.0 mmol) was added dropwise to the reaction system, after the dropwise addition, the system was stirred at 70°C for 3 hours. The reaction mixture was cooled to room temperature (25°C), filtered with celite, and the filtrate was concentrated under vacuum to obtain the crude product, which was purified by silica gel column chromatography (ethyl acetate/petroleum ether (v/v) = 0-6%) to obtain compound A1-2.

¹H NMR (400 MHz, DMSO-*d*₆) δ 7.44 (d, *J* = 2.0 Hz, 1H), 7.41 - 7.36 (m, 2H), 7.35 - 7.29 (m, 2H), 7.29 - 7.23 (m, 2H), 7.13 (dd, J = 8.0, 0.9 Hz, 1H), 4.27 (s, 2H), 2.18 (s, 3H).

### Step 2: preparation of compound A1-3

Compound A1-2 (12.9 g, 43.87 mmol) was dissolved in a mixed solution of acetic acid (120 mL) and water (30 mL), to which *N*-chlorosuccinimide (23.30 g, 175.5 mmol) was added in batches at room temperature (25°C), after the addition, the system was stirred at room temperature (25°C) for 2 hours. The reaction mixture was concentrated under vacuum at low temperature (<30°C) to obtain compound A1-3, which was directly used in the next step without further purification.

### Step 3: preparation of compound A1-4

*Tert*-butylamine (6.3 g, 86 mmol) and triethylamine (13 g, 129 mmol) were dissolved in anhydrous dichloromethane (100 mL), then the system was cooled to 0°C, to which compound A1-3 was slowly added. After the addition, the system was heated to room temperature (25°C) and reacted for 2 hours. Water (100 mL) was added to the reaction mixture, followed by liquid separation. The aqueous phase was extracted with dichloromethane (80 mL x 2). The organic phases were combined, washed with saturated aqueous sodium chloride solution (100 mL), dried over anhydrous sodium sulfate, filtered, the filtrate was concentrated under vacuum to obtain the crude product. The crude product was purified by silica gel column chromatography (ethyl acetate/petroleum ether (v/v) = 0-10%) to obtain compound A1-4.

¹H NMR (400 MHz, Chloroform-*d*) δ 8.17 (d, *J* = 2.2 Hz, 1H), 7.55 (dd, *J =* 8.1, 2.2 Hz, 1H), 7.17 (dd, *J =* 8.1, 0.8 Hz, 1H), 4.44 (s, 1H), 2.59 (d, *J =* 0.6 Hz, 3H), 1.24 (s, 9H).

### Step 4: preparation of compound A1-5

Compound A1-4 (7 g, 22.8 mmol) was dissolved in carbon tetrachloride (100 mL), and then N-bromosuccinimide (4.46 g, 25 mmol) and 2, 2'-azobis(2-methylpropionitrile) (370 mg, 2.28 mmol) were added. After the addition, the reaction system was heated to 80°C and stirred for 16 hours. The reaction mixture was cooled to room temperature (25°C) and concentrated under vacuum to obtain the crude product. The crude product was purified by silica gel column chromatography (ethyl acetate/petroleum ether (v/v) = 0-10%) to obtain compound A1-5.

¹H NMR (400 MHz, Chloroform-d) δ 8.20 (d, *J =* 2.1 Hz, 1H), 7.66 (dd, *J =* 8.2, 2.1 Hz, 1H), 7.43 (d, *J =* 8.2 Hz, 1H), 4.92 (s, 2H), 1.29 (s, 9H).

### Step 5: preparation of compound A1-6

Compound A1-5 (5.65 g, 14.67 mmol), potassium carbonate (4 g, 29.3 mmol), tetrabutylammonium iodide (1 g, 3 mmol) were dissolved in acetonitrile (100 mL). After the addition, the system was heated to 50°C and stirred for 16 hours. The reaction mixture was cooled to room temperature (25°C), to which water (200 mL) was added, extracted with ethyl acetate (100 mL × 3), and the organic phases were combined, dried over anhydrous sodium sulfate, filtered, and the filtrate was concentrated to obtain the crude product. The crude product was purified by silica gel column chromatography (ethyl acetate/petroleum ether (v/v) = 0-30%) to obtain compound A1-6.

MS (ESI) m/z [(M+H)⁺-56] = 248.0.

### Step 6: preparation of compound A1-8

Compound A1-6 (4.07 g, 13.39 mmol), compound A1-7 (5.54 g, 15.40 mmol), tetrakis(triphenylphosphine)palladium (775 mg, 0.67 mmol) were dissolved in *N,N-*dimethylformamide (50 mL). Under nitrogen atmosphere, the system was heated to 100°C and stirred for 4 hours. The reaction mixture was cooled to room temperature (25°C), to which water (100 mL) was added, extracted with ethyl acetate (100 mL × 2), and the organic phases were combined, dried over anhydrous sodium sulfate, filtered, and the filtrate was concentrated to obtain the crude product. The crude product was purified by silica gel column chromatography (ethyl acetate/petroleum ether (v/v) = 0-20%) to obtain compound A1-8.

MS (ESI) m/z [(M+H)⁺-56] = 240.0.

### Step 7: preparation of compound A1-9

Compound A1-8 (3.54 g, 12 mmol) was dissolved in a mixed solution of THF (50 ml) and water (20 mL). Under the condition of 0°C, to which N-bromosuccinimide (2.13 g, 12 mmol) was added, the system was stirred at 0°C for 1 hour. The reaction mixture was concentrated to obtain the crude product, which was purified by silica gel column chromatography (ethyl acetate/petroleum ether (v/v) = 0-30%) to obtain compound A1-9.

MS (ESI) m/z [(M+H)⁺-56] = 291.9.

### Step 8: preparation of compound A1-11

Compound A1-9 (3.5 g, 10.1 mmol), compound A1-10 (1.93 g, 10.1 mmol) were dissolved in 1,4-dioxane (100 mL), to which triethylamine (2.05 g, 20.2 mmol) was added, after the addition, the system was stirred at room temperature (25°C) for 2 hours. The reaction mixture was filtered, and the filtrate A1-11 was directly used in the next step.

MS (ESI) m/z (M+H)⁺ = 455.0.

### Step 9: preparation of compound A1-12

Compound A1-11 (the filtrate obtained in step 8) was dissolved in methanol (100 mL), to which sodium borohydride (740 mg, 20 mmol) was added in batches at 0°C, and the system was heated to 5°C and stirred for 2 hours. Acetone (5 mL) was added to the system, and the system was concentrated under vacuum. The concentrated residue was dissolved in dichloromethane (50 mL), and washed with saturated aqueous sodium chloride solution. The organic phases were combined, dried over anhydrous sodium sulfate, filtered, and the filtrate was concentrated to obtain compound A1-12, which was used directly in the next step without further purification.

MS (ESI) m/z (M+H)⁺ = 457.1.

### Step 10: preparation of compound A1-13

Compound A1-12 (4.5 g, 9.82 mmol) was dissolved in anhydrous dichloromethane (20 mL), to which concentrated sulfuric acid (20 mL) was added dropwise at 0°C, after the addition, the system was heated to 90°C and stirred for 1 hour. After the reaction mixture was cooled to room temperature (25°C), it was poured into ice water (200 g), and the pH of which was adjusted to 9 with 2N aqueous sodium hydroxide solution. The reaction mixture was extracted with dichloromethane (100 mL × 3), and the organic phases were combined, dried over anhydrous sodium sulfate, filtered, and the filtrate was concentrated to obtain the crude product. The crude product was separated and purified to obtain compound A1-13.

¹H NMR (400 MHz, Methanol-*d*₄) δ 7.55 (d, *J =* 1.4 Hz, 1H), 7.39 (qd, *J =* 8.0, 1.2 Hz, 2H), 7.33 - 7.20 (m, 1H), 6.72 (dd, *J =* 2.1, 0.9 Hz, 1H), 4.51 (s, 1H), 4.34 (s,2H), 3.66 - 3.60 (m, 2H), 2.92 (dd, *J =* 11.8, 5.4 Hz, 1H), 2.61 (dd, *J =* 11.7, 7.4 Hz, 1H), 2.38 (s, 3H).

Similar to the synthesis of intermediate A1-13, the following intermediate A2-13 was synthesized, as shown in Table 1 below:

**Table 1: Structural formula and analysis data of intermediate A2-13**

| Intermediate | Structural formula | Analysis data |
|---|---|---|
| A2-13 | 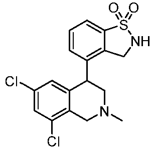 | ¹H NMR (400 MHz, DMSO-*d*₆) δ 7.84 (t, *J* = 4.5 Hz, 1H), 7.69 (dd, *J =* 7.8, 0.9 Hz, 1H), 7.53 (d, *J* = 2.1 Hz, 1H), 7.46 (t, *J =* 7.7 Hz, 1H), 7.17 (dd, *J* = 7.6, 1.0 Hz, 1H), 6.99 (d, *J =* 2.3 Hz, 1H), 4.71 (dd, *J =* 15.3, 4.4 Hz, 1H), 4.40 (d, *J =* 4.8 Hz, 1H), 4.31 (dd, *J =* 15.2, 4.4 Hz, 1H), 3.76 (d, *J =* 16.2 Hz, 1H), 3.43 (d, *J =* 16.3 Hz, 1H), 2.82 (dd, *J* = 11.7, 5.1 Hz, 1H), 2.63 (dd, *J* = 11.7, 5.1 Hz, 1H), 2.34 (s, 3H). |
| | | MS (ESI) m/z (M+H)⁺ = 382.8. |

### Step 11: preparation of compound A1-15

Compound A1-13 (246 mg, 0.642 mmol), compound A1-14 (290 mg, 0.942 mmol), cesium carbonate (416 mg, 1.280 mmol), sodium iodide (10 mg, 0.071 mmol) were dissolved in N,N-dimethylformamide (4 mL), and the system was heated to 60°C and stirred for 1 hour. The reaction mixture was cooled to room temperature, filtered, and the filtrate was purified by C18 reversed-phase silica gel column chromatography (acetonitrile/0.1% aqueous ammonium bicarbonate (v/v) = 5-65%) to obtain compound A1-15.

MS (ESI) m/z (M+H)⁺ = 614.2.

### Step 12: preparation of compound A1-16

Compound A1-15 (240 mg, 0.390 mmol) was dissolved in anhydrous methanol (3 mL), to which concentrated hydrochloric acid (2 mL) was added, after the addition, the system was stirred at room temperature (25°C) for 1 hour. The reaction mixture was concentrated under vacuum to obtain the crude product, dissolved with dichloromethane (20 mL), to which water (20 mL) was added, and the pH of which was adjusted to 9 with saturated aqueous sodium bicarbonate solution, and separated, and the aqueous phase was extracted with dichloromethane (20 mL × 2), and the organic phases were combined, dried over anhydrous sodium sulfate, filtered, and the filtrate was concentrated to obtain compound A1-16.

MS (ESI) m/z (M+H)⁺ = 514.0.

### Step 13: preparation of compound A1

Compound A1-16 (165 mg, 0.320 mmol) was dissolved in N,N-dimethylformamide (2 mL), to which 1,4-diisocyanatobutane (20 mg, 0.128 mmol) was added, after the addition, the system was stirred at room temperature (25°C) for 1 hour. The reaction mixture was purified by high performance preparative liquid chromatography (separation conditions: column: Agilent 10 Prep-C18 250×21.2mm; column temperature: 25°C; mobile phase: water (10 mM/L ammonium bicarbonate)-acetonitrile; acetonitrile: 60%-80% 12min, flow rate: 30 mL/min) to obtain compound A1.

¹H NMR (400 MHz, Methanol-*d*₄) δ 7.58 (d, J = 1.7 Hz, 2H), 7.44 - 7.35 (m, 4H), 7.24 (d, J = 2.0 Hz, 2H), 6.71 (d, J = 2.1 Hz, 2H), 4.45 (s, 4H), 4.32 (t, J = 6.3 Hz, 2H), 3.70 (t, J = 5.2 Hz, 4H), 3.60 (s, 4H), 3.58 - 3.50 (m, 8H), 3.38 (dt, *J =* 15.5, 5.1 Hz, 8H), 3.15 (t, *J =* 5.2 Hz, 4H), 3.04 (dd, *J =* 11.8, 5.3 Hz, 2H), 3.01 - 2.91 (m, 4H), 2.82 - 2.71 (m, 2H), 2.48 (d, *J =* 3.5 Hz, 6H), 1.40 - 1.27 (m, 4H).

MS (ESI) m/z (M+H)⁺ = 1169.20.

HPLC 100% purity; retention time was 7.680 min.

Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min.

Similar to the synthesis of embodiment A1, embodiments A2 was synthesized from A2-13, as shown in Table 2 below:

**Table 2: Structural formula and analysis data of embodiment A2**

| | |
|---|---|
| Structural formula | 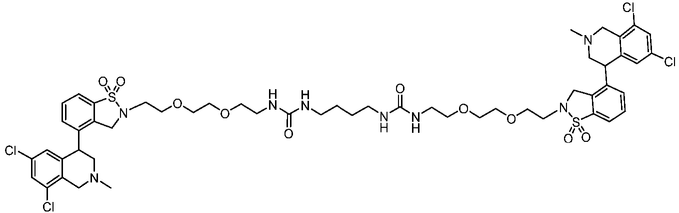 |
| Analysis data | ¹H NMR (400 MHz, Methanol-*d*₄) δ 7.61 (dd, *J =* 7.8, 1.0 Hz, 2H), 7.44 (t, *J* = 7.7 Hz, 2H), 7.30 - 7.22 (m, 4H), 6.73 (d, *J =* 2.1 Hz, 2H), 4.64 (d, *J =* 14.5 Hz, 2H), 4.34 (t, *J =* 6.6 Hz, 2H), 4.29 (d, *J =* 14.4 Hz, 2H), 3.73 - 3.69 (m, 4H), 3.65 (s, 2H), 3.59 (s, 2H), 3.57 - 3.51 (m, 8H), 3.42 - 3.37 (m, 8H), 3.15 (t, *J =* 5.4 Hz, 4H), 2.97 (q, *J =* 2.9 Hz, 4H), 2.94 - 2.90 (m, 2H), 2.55 (dd, *J =* 11.8, 7.5 Hz, 2H), 2.37 (s, 6H), 1.34 (t, *J =* 3.1 Hz, 4H). |
| | MS (ESI) m/z (M+H)⁺ = 1169.20. |
| | HPLC 100% purity; retention time was 7.114 min. |
| | Separation conditions: column: Waters XBridge 4.6*100mm, 3.5um; column temperature: 40°C; mobile phase: water (10mM NH₄HCO₃)-acetonitrile; acetonitrile: 5%-95% 7 min, 95% 8min; flow rate: 1.2 mL/min. |

### Synthesis of embodiment B1

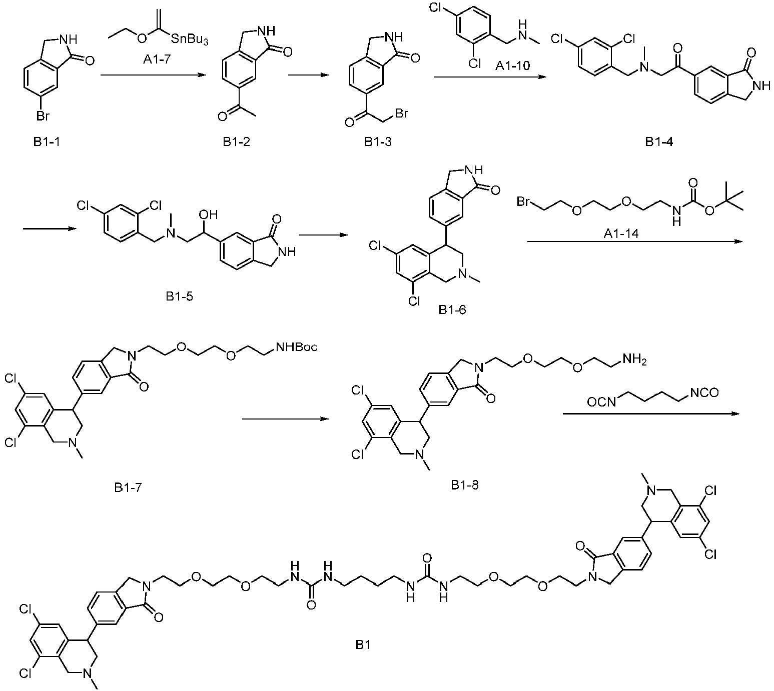

### Step 1: preparation of compound B1-2

Under nitrogen atmosphere, compound B1-1 (8.48 g, 40 mmol), compound A1-7 (17.32 g, 48.0 mmol) and tetrakis(triphenylphosphine)palladium (2.31 g, 2 mmol) were dissolved in *N*,*N*-dimethylformamide (80 mL), and system was heated to 100°C and stirred for 4 hours. The reaction mixture was cooled to room temperature (25°C), quenched by adding saturated aqueous ammonium chloride (200 mL) and extracted with ethyl acetate (200 mL × 3), and the organic phases were combined, washed by saturated sodium chloride (200 mL × 2), dried over anhydrous sodium sulfate, filtered, and the filtrate was concentrated to obtain the crude product. The crude product was dissolved in tetrahydrofuran (60 mL), to which aqueous hydrochloric acid (2 *N,* 8 mL) was added, and the system was stirred for 30 minutes. The system was concentrated, slurried with petroleum ether, and filtered to obtain compound B1-2, which was directly used for the next step without further purification.

MS (ESI) *m*/*z* (M+H)⁺ = 175.8.

### Step 2: preparation of compound B1-3

Compound B1-2 (200 mg, 1.14 mmol) was dissolved in chloroform (30 mL), to which liquid bromine (1.21 g, 7.66 mmol) was added dropwise, after the addition, the system was heated to 70°C and stirred for 30 minutes. The reaction mixture was filtered to obtain compound B1-3, which was directly used in the next step without further purification.

MS (ESI) *m*/*z* (M+H)⁺ = 256.0.

### Step 3: preparation of compound B1-4

Compound B1-3 (1.4 g, 5.53 mmol), compound A1-10 (1.25 g, 5.53 mmol) and triethylamine (2.8 g, 27.65 mmol) were dissolved in 1,4-dioxane (20 mL), and the system was reacted at room temperature (25 °C) for 3 hours. The reaction mixture was concentrated, to which water (40 mL) was added, extracted by ethyl acetate (50 mL x 3), and the organic phase was dried over anhydrous sodium sulfate, filtered and concentrated to obtain compound B1-4, which was used directly in the next step without further purification.

MS (ESI) *m*/*z* (M+H)⁺ = 363.0.

### Step 4: preparation of compound B1-5

Compound B1-4 (1.4 g, 3.87 mmol) was dissolved in methanol (20 mL), to which sodium borohydride (300 mg, 7.74 mmol) was added in batches at 0°C. After the addition, the system was reacted at 0°C for 2 hours. The reaction mixture was quenched by adding acetone (5 mL), and concentrated to obtain the crude product, which was purified by silica gel column chromatography (dichloromethane/methanol (v/v) = 10-1%) to obtain compound B1-5.

MS (ESI) *m*/*z* (M+H)⁺ = 365.0.

### Step 5: preparation of compound B1-6

Compound B1-5 (1.9 g, 5.2 mmol) was dissolved in dichloromethane (5 mL), to which concentrated sulfuric acid (3 mL) was added dropwise at 0°C. After the addition, the system was heated to room temperature (25°C) and reacted for 5 hours. The reaction mixture was poured into ice water, and the pH of which was adjusted to 7-8 with 2N aqueous sodium hydroxide solution. The reaction mixture was extracted with dichloromethane (80 mL × 3), and the organic phases were combined, dried over anhydrous sodium sulfate, filtered, and the filtrate was concentrated to obtain the crude product. The crude product was separated and purified to obtain compound B1-6.

¹H NMR (400 MHz, DMSO-*d*₆) δ 8.54 (s, 1H), 7.54 - 7.40 (m, 4H), 6.86 (d, *J =* 2.1 Hz, 1H), 4.41 (t, *J =* 5.4 Hz, 1H), 4.34 (s, 2H), 3.73 (d, *J =* 16.2 Hz, 1H), 3.47 (d, *J =* 16.1 Hz, 1H), 2.84 (dd, *J =* 11.6, 5.1 Hz, 1H), 2.71 - 2.62 (m, 1H), 2.36 (s, 3H).

HPLC 100%. Retention time was 5.598 min.

Separation conditions: column: Waters XBridge 4.6*100mm, 3.5µm; column temperature: 40°C; mobile phase: water (10mM ammonium bicarbonate)-acetonitrile; acetonitrile: 5%-95% 7min; flow rate: 1.2 mL/min.

Similar to the synthesis of intermediate B1-6, the following intermediate B2-6 - B5-6 were synthesized, as shown in Table 3 below:

**Table 3: Structural formula and analysis data of intermediate B2-6 - B5-6**

| **Intermediates** | Structural formula | Analysis data |
|---|---|---|
| **B2-6** | 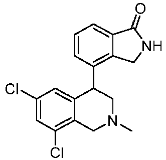 | ¹H NMR (400 MHz, DMSO-*d*₆) δ 8.57 (s, 1H), 7.55 (d, *J* = 7.3 Hz, 1H), 7.51 (d, *J =* 2.0 Hz, 1H), 7.38 (t, *J =* 7.5 Hz, 1H), 7.16 (d, *J =* 7.1 Hz, 1H), 6.94 (d, *J =* 1.7 Hz, 1H), 4.56 (d, *J =* 17.6 Hz, 1H), 4.40 (t, *J =* 5.4 Hz, 1H), 4.15 (d, *J =* 17.6 Hz, 1H), 3.70 (d, *J =* 16.2 Hz, 1H), 3.50 (d, *J =* 16.2 Hz, 1H), 2.87 (dd, *J =* 11.6, 5.2 Hz, 1H), 2.65 (dd, *J =* 11.6, 6.0 Hz, 1H), 2.35 (s, 3H) |
| | | MS (ESI) *m*/*z* (M+H)⁺ = 346.8 |
| | | LCMS 100%. Retention time was 0.98 min. |
| | | Separation conditions: column: Infinitylab Poroshell HPH-C18 3.0*30mm, 1.9um; column temperature: 40°C; mobile phase: water (10mM ammonium bicarbonate)-acetonitrile: 5%-95% 0.7 min, 95% 0.8min, 5% 0.5 min; flow rate: 1.2 ml/min. |
| **B3-6** | 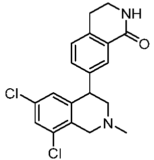 | ¹H NMR (400 MHz, DMSO-*d*₆) δ 7.93 (d, *J =* 2.8 Hz, 1H), 7.63 (d, *J =* 1.9 Hz, 1H), 7.49 (d, *J =* 2.2 Hz, 1H), 7.33 (dd, *J =* 7.8, 2.0 Hz, 1H), 7.23 (d, *J =* 7.8 Hz, 1H), 6.84 (d, *J* = 2.1 Hz, 1H), 4.32 (t, *J =* 5.6 Hz, 1H), 3.72 - 3.46 (m, 2H), 3.36 (dd, *J =* 6.7, 2.8 Hz, 2H), 2.85 (dt, *J =* 16.2, 5.8 Hz, 3H), 2.61 (dd, *J =* 11.5*,* 6.2 Hz, 1H), 2.36 (s, 3H) |
| | | MS (ESI) m/z (M+H)⁺ = 360.8 |
| | | HPLC 100.0% purity; retention time was 6.264 min. |
| | | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |
| **B4-6** | 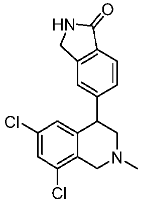 | ¹H NMR (400 MHz, DMSO-*d*₆) δ 8.50 (s, 1H), 7.60 (d, *J* = 7.8 Hz, 1H), 7.50 (s, 1H), 7.40 (s, 1H), 7.35 (d, *J =* 8.9 Hz, 1H), 6.87 (s, 1H), 4.40 (t, *J =* 5.3 Hz, 1H), 4.32 (s, 2H), 3.69 (d, *J =* 16.2 Hz, 1H), 3.49 (d, *J =* 16.1 Hz, 1H), 2.86 (dd, *J =* 11.5, 5.2 Hz, 1H), 2.69 (dd, *J =* 11.5, 5.8 Hz, 1H), 2.37 (s, 3H). |
| | | MS (ESI) *m*/*z* (M+H)⁺ = 346.8. |
| | | HPLC 100%. Retention time was 6.02 min. |
| | | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |
| **B5-6** | 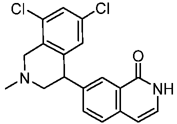 | ¹H NMR (400 MHz, DMSO-*d*₆) δ 11.25 (d, *J =* 5.6 Hz, 1H), 7.96 (d, *J =* 1.6 Hz, 1H), 7.62 - 7.54 (m, 2H), 7.50 (d, *J =* 2.0 Hz, 1H), 7.14 (dd, *J =* 7.1, 5.7 Hz, 1H), 6.88 (d, *J =* 2.0 Hz, 1H), 6.52 (d, *J =* 7.0 Hz, 1H), 4.43 (t, *J =* 5.5 Hz, 1H), 3.71 (d, *J =* 16.2 Hz, 1H), 3.50 (d, *J* = 16.2 Hz, 1H), 2.87 (dd, *J =* 11.6, 5.1 Hz, 1H), 2.68 (dd, *J =* 11.5, 6.0 Hz, 1H), 2.36 (s, 3H). MS (ESI) m/z (M+H)⁺ = 359.0 |
| | | HPLC retention time was 6.880 min |
| | | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |

### Step 6: preparation of compound B1-7

Compound B1-6 (250 mg, 0.72 mmol), compound A1-14 (335 mg, 1.08 mmol), cesium carbonate (585 mg, 1.8 mmol), 18-crown-6 (19 mg, 0.072 mmol) were added into acetonitrile (25 ml), and the system was heated to 90°C for 10 hours. The reaction mixture was concentrated, to which water (30 mL) was added, extracted with ethyl acetate (50 mL x 3), the organic phases were combined, dried over anhydrous sodium sulfate, filtered, and the filtrate was concentrated to obtain the crude product, which was purified by reverse phase C18 column chromatography (acetonitrile/water (5% aqueous ammonium bicarbonate solution) (v/v) = 30-40%) to obtain compound B1-7.

MS (ESI) *m*/*z* (M+H)⁺ = 578.2.

### Step 7: preparation of compound B1-8

Compound B1-7 (134 mg, 0.2 mmol) was dissolved in dichloromethane (2 mL) at room temperature (25°C), to which a solution of dioxane with hydrochloric acid (2 mL) was added, and the reaction was stirred at room temperature (25°C) for 2 hours. The reaction mixture was concentrated, and the crude product was dissolved in saturated aqueous sodium bicarbonate solution, extracted with dichloromethane, and the organic phases were combined, dried over anhydrous sodium sulfate, filtered, and the filtrate was concentrated to obtain compound B1-8.

MS (ESI) *m*/*z* (M+H)⁺ = 478.0.

### Step 8: preparation of compound B1

Compound B1-8 (120 mg, 0.25 mmol) was dissolved in N,N-dimethylformamide (1 mL), to which 1,4-diisocyanatobutane (14 mg, 0.09 mmol) was added at 0°C, and the reaction was stirred at room temperature (25°C) for 0.5 hour. The reaction mixture was purified directly by high performance preparative liquid chromatography (separation conditions: column: Welch Xtimate C18 250×21.2mm; column temperature: 25°C; mobile phase: water (10 mM/L ammonium bicarbonate)-acetonitrile; acetonitrile: 50%-70% 12min, flow rate: 30 mL/min) to obtain compound B1.

¹H NMR (400 MHz, Methanol-*d*₄) δ 7.48 - 7.40 (m, 4H), 7.37 (d, *J =* 9.4 Hz, 2H), 7.23 (d, *J =* 1.7 Hz, 2H), 6.76 - 6.64 (m, 2H), 4.51 (s, 4H), 4.35 - 4.28 (m, 2H), 3.75 - 3.61 (m, 10H), 3.54 - 3.50 (m, 6H), 3.48-3.44 (m, 4H), 3.33 (t, *J =* 5.4 Hz, 4H), 3.09 (t, *J =* 5.3 Hz, 4H), 3.03 - 2.89 (m, 6H), 2.55 (dd, *J =* 11.7, 8.1 Hz, 2H), 2.37 (s, 6H), 1.35 (t, *J =* 4.7 Hz, 4H).

MS (ESI) m/z (M+H)⁺ = 1097.4.

HPLC 100% purity; retention time was 6.908 min.

Separation conditions: column: Waters XBridge 4.6*100mm, 3.5um; column temperature: 40°C; mobile phase: water (10mM ammonium bicarbonate)-acetonitrile; acetonitrile: 5%- 95% 7 min; flow rate: 1.2 mL/min.

### Synthesis of embodiment B3

### Step 1: preparation of compound B3-7

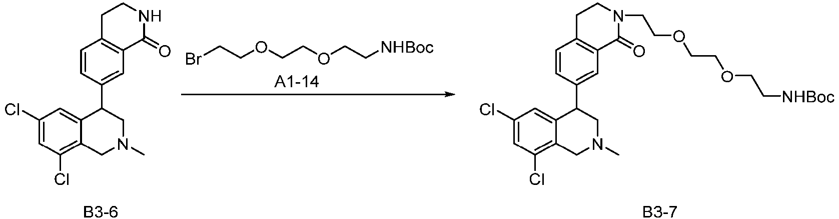

Compound B3-6 (144 mg, 0.398 mmol) was dissolved in tetrahydrofuran (2 mL), to which sodium hydride (29.6 mg, 0.74 mmol) was added under argon protection, and the system was stirred at room temperature (25°C) for 0.5 hour. 18-Crown ether-6 (101.6 mg, 0.39 mmol) and compound A1-14 (186 mg, 0.589 mmol) were dissolved in tetrahydrofuran (2 mL), which were slowly added into the above reaction system under argon protection, and the system was stirred at room temperature (25°C) for 16 hours. The reaction mixture was purified by C18 reversed-phase silica gel column chromatography (acetonitrile/0.1% aqueous ammonium bicarbonate (v/v) = 5-65% to obtain compound B3-7.

MS (ESI) m/z (M+H)⁺ = 592.40.

Similar to the synthesis of embodiment B1, embodiments B2 - B6 were synthesized from B2-6 - B3-6, as shown in Table 4 below:

**Table 4: Structural formula and analysis data of embodiments B2 - B6**

| | |
|---|---|
| **Embodiment B2** | 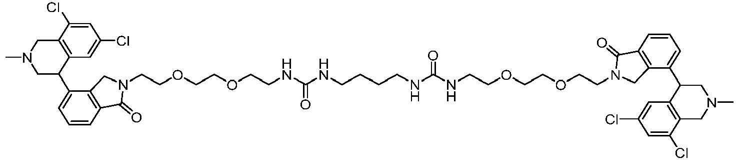 |
| | ¹H NMR (400 MHz, Methanol-*d*₄) δ 7.62 - 7.57 (m, 2H), 7.37 (t, *J =* 7.6 Hz, 2H), 7.27 (d, *J* = 2.0 Hz, 2H), 7.22 (d, *J =* 7.6 Hz, 2H), 6.71 (d, *J =* 2.0 Hz, 2H), 4.57 (d, *J =* 17.9 Hz, 2H), 4.43 - 4.35 (m, 2H), 4.16 (d, *J =* 17.9 Hz, 2H), 3.80 - 3.59 (m, 10H), 3.56 - 3.43 (m, 10H), 3.35 (t, *J =* 5.4 Hz, 4H), 3.11 (t, *J =* 5.4 Hz, 4H), 2.97 (dt, *J =* 11.7, 5.6 Hz, 6H), 2.57 (dd, *J* = 11.7, 8.3 Hz, 2H), 2.38 (s, 6H), 1.41 - 1.32 (m, 4H). |
| | MS (ESI) m/z (M+H)⁺ = 1097.4 |
| | HPLC 100% purity; retention time was 6.929 min. |
| | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |
| **Embodiment B3** | 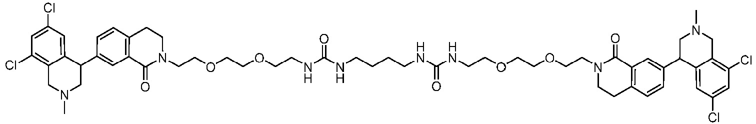 |
| | ¹H NMR (400 MHz, DMSO-*d*₆) δ 7.65 (d, *J =* 1.9 Hz, 2H), 7.49 (d, *J =* 2.1 Hz, 2H), 7.31 (dd, *J =* 7.9, 2.0 Hz, 2H), 7.21 (d, *J =* 7.8 Hz, 2H), 6.86 - 6.80 (m, 2H), 5.92 (t, *J =* 5.7 Hz, 2H), 5.78 (t, *J* = 5.7 Hz, 2H), 4.32 (t, *J* = 5.3 Hz, 2H), 3.71 - 3.43 (m, 24H), 3.33 (d, *J =* 5.5 Hz, 4H), 3.09 (q, *J =* 5.7 Hz, 4H), 2.97 - 2.88 (m, 8H), 2.83 (s, 2H), 2.63 (s, 2H), 2.36 (s, 6H), 1.30 (t, *J* = 4.1 Hz, 4H). |
| | MS (ESI) m/z (M+H)⁺ = 1123.60. |
| | HPLC 100% purity; retention time was 7.717 min. |
| | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |
| **Embodiment B4** | 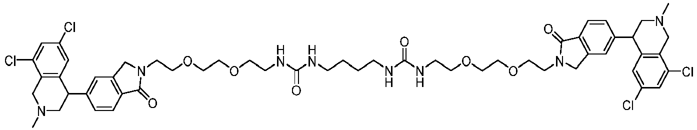 |
| | ¹H NMR (400 MHz, DMSO-*d*₆) δ 7.60 (d, *J =* 7.8 Hz, 2H), 7.47 (d, *J =* 2.1 Hz, 2H), 7.43 (s, 2H), 7.33 (dd, *J =* 7.9, 1.5 Hz, 2H), 6.85 (d, *J =* 2.0 Hz, 2H), 5.91 (t, *J =* 5.6 Hz, 2H), 5.77 (t, *J =* 5.8 Hz, 2H), 4.50 (s, 4H), 4.39 (t, *J* = 5.6 Hz, 2H), 3.73 - 3.58 (m, 10H), 3.57 - 3.43 (m, 10H), 3.34 (s, 2H), 3.31 (s, 2H), 3.08 (q, *J =* 5.7 Hz, 4H), 2.94 (q, *J =* 5.6, 5.2 Hz, 4H), 2.86 (dd, *J =* 11.6, 5.2 Hz, 2H), 2.67 (dd, *J =* 11.5, 6.1 Hz, 2H), 2.36 (s, 6H). 1.31 (t, *J =* 4.7 Hz, 4H). |
| | MS (ESI) m/z (M+H)⁺ = 1097.4 |
| | HPLC 100% purity; retention time was 7.228 min. |
| | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |
| **Embodiment B5** | 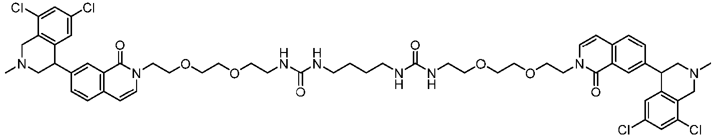 |
| | **¹H NMR** (400 MHz, Methanol-*d*₄) δ 8.04 (d, *J =* 1.8 Hz, 2H), 7.53 (d, *J* = 8.2 Hz, 2H), 7.47 - 7.42 (m, 2H), 7.31 - 7.24 (m, 4H), 6.70 (d, *J* = 2.1, 1.0 Hz, 2H), 6.56 (d, *J =* 7.3 Hz, 2H), 4.44 (t, *J* = 9.1, 5.8 Hz, 2H), 4.11 (t, *J =* 5.2 Hz, 4H), 4.01 (d, *J =* 16.1 Hz, 2H), 3.78 (d, *J =* 16.1 Hz, 2H), 3.72 (t, *J* = 5.2 Hz, 4H), 3.51 - 3.44 (m, 4H), 3.44 - 3.37 (m, 4H), 3.29 (t, *J =* 5.3 Hz, 4H), 3.07 (t, *J =* 5.3 Hz, 4H), 3.01 - 2.94 (m, 4H), 2.85 (t, *J =* 11.9, 9.3 Hz, 2H), 2.57 (s, 6H), 1.37 - 1.30 (m, 4H). |
| | MS (ESI) *m*/*z* (M+H)⁺ = 1121.2. |
| | HPLC 100% purity; retention time was 7.978 min. |
| | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |
| **Embodiment B6** | 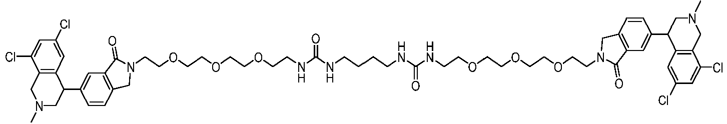 |
| | ¹H NMR (400 MHz, DMSO-*d*₆) δ 7.52 - 7.45 (m, 8H), 6.85 (d, *J =* 2.1 Hz, 2H), 5.91 (t, *J =* 5.7 Hz, 2H), 5.79 (t, *J =* 5.8 Hz, 2H), 4.51 (s, 4H), 4.42 - 4.38 (m, 2H), 3.72 (d, *J =* 16.1 Hz, 2H), 3.67-3.59 (m, 8H), 3.54 - 3.47 (m, 10H), 3.47-3.45 (m, 4H), 3.43-3.41 (m, 4H), 3.31 (d, *J =* 5.7 Hz, 4H), 3.10 (q, *J =* 5.7 Hz, 4H), 2.94 (d, *J =* 5.6 Hz, 4H), 2.84 (dd, *J =* 11.5, 5.2 Hz, 2H), 2.68 - 2.63 (m, 2H), 2.35 (s, 6H), 1.31-1.28 (m, 4H). |
| | MS (ESI) m/z (M+2H)⁺/2 = 593.2. |
| | HPLC 100% purity; retention time was 7.099 min |
| | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |

### Synthesis of embodiment C1

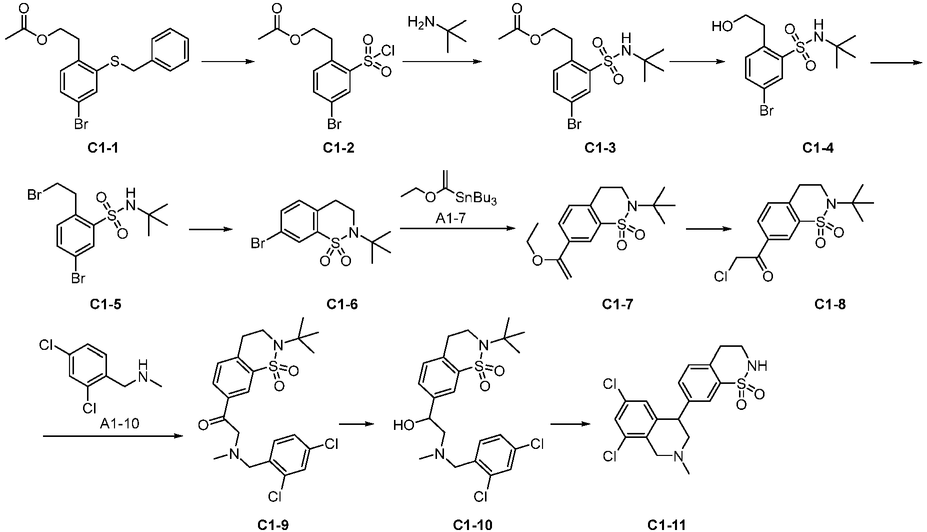

### Step 1: preparation of compound C1-2

Compound C1-1 (200.0 mg, 0.548 mmol) was dissolved in a mixed solvent of acetic acid (2.50 mL) and water (0.25 mL), which was placed in a water bath at 10°C to cool down, to which *N*-chlorosuccinimide (220.0 mg, 1.64 mmol, 3.0 equiv) was added. The reaction was continued to stir for 1.0 hour in a water bath at 10°C. The reaction mixture was quenched by adding saturated brine (5.0 mL), then the reaction was extracted with ethyl acetate (10.0 mL × 3 times), and the organic phases were combined, dried over anhydrous sodium sulfate and concentrated, and the crude product was purified by silica gel column chromatography (ethyl acetate/petroleum ether (v/v) = 0-10%) to obtain compound C1-2.

**¹H NMR** (400 MHz, Chloroform-d) δ 8.24 (d, *J =* 2.1 Hz, 1H), 7.79 (dd, *J* = 8.3, 2.1 Hz, 1H), 7.36 (s, 1H), 4.40 (t, *J =* 6.7 Hz, 2H), 3.44 (t, *J =* 6.7 Hz, 2H), 2.05 (s, 3H).

### Step 2: preparation of compound C1-3

*Tert*-butylamine (44.0 mg, 0.60 mmol, 1.1 equiv) and triethylamine (83.0 mg, 0.82 mmol, 1.5 equiv) were dissolved in dichloromethane (2.50 mL), which was placed in a water bath at 10°C to cool down, then compound C1-2 (185.0 mg, 0.54 mmol) was dissolved in dichloromethane (1.0 mL) which was added dropwise to the above reaction mixture. The reaction was heated to room temperature (25 °C) and continued to stir for 2.0 hours. The reaction mixture was directly concentrated to obtain the crude product, which was purified by silica gel column chromatography (ethyl acetate/petroleum ether (v/v) = 0-15%) to obtain compound C1-3.

**¹H NMR** (400 MHz, Chloroform-d) δ 8.20 (d, *J* = 2.2 Hz, 1H), 7.60 (dd, *J* = 8.2, 2.2 Hz, 1H), 7.22 (d, *J =* 8.2 Hz, 1H), 4.33 (t, *J =* 7.6 Hz, 2H), 3.33 (t, *J =* 7.6 Hz, 2H), 2.07 (s, 3H), 1.27 (s, 9H).

### Step 3: preparation of compound C1-4

Compound C1-3 (150.0 mg, 0.398 mmol) was dissolved in a mixed solvent of tetrahydrofuran (0.70 mL), methanol (0.70 mL) and lithium hydroxide (34.0 mg, 1.4 mmol, 3.5 equiv) in water (0.25 mL), and the system was stirred at room temperature (25 °C) for 1.0 hour. The reaction mixture was quenched by adding saturated ammonium chloride aqueous solution (5.0 mL), then extracted by ethyl acetate (10.0 mL × 3 times), and the organic phases were combined, washed with saturated brine (10.0 mL × 2 times), the organic phase was dried over anhydrous sodium sulfate after and concentrated to obtain compound C1-4, which was used directly in the next step without further purification.

MS (ESI) *m*/*z* (M+H)⁺ = 335.8.

### Step 4: preparation of compound C1-5

Compound C1-4 (1.0 g, 3.0 mmol) and triphenylphosphine (787.0 mg, 3.0 mmol, 1.0 equiv) were dissolved in dichloromethane (10.0 mL) solution, to which a solution of carbon tetrabromide (1.1 g, 3.3 mmol, 1.1 equiv) in dichloromethane (5.0 mL) was added at 0°C, after the addition, the system was heated to room temperature (25°C) and stirred for 1.0 hour. The reaction mixture was directly concentrated to obtain the crude product, which was purified by silica gel column chromatography (ethyl acetate/petroleum ether (v/v) = 0-10%) to obtain compound C1-5.

**¹H NMR** (400 MHz, Chloroform-d) δ 8.11 (d, *J =* 2.1 Hz, 1H), 7.56 (dd, *J* = 8.2, 2.1 Hz, 1H), 7.20 (s, 1H), 3.65 - 3.55 (m, 2H), 3.44 (t, *J =* 7.4 Hz , 2H), 1.20 (d, *J =* 2.6 Hz, 9H).

### Step 5: preparation of compound C1-6

Compound C1-5 (8.20 g, 20.65 mmol) and potassium carbonate (5.70 g, 41.3 mmol, 2.0 equiv) were added to N,N-dimethylformamide (100.0 mL) solution, and the system was stirred overnight at room temperature (25°C). The reaction mixture was directly concentrated to obtain the crude product, which was purified by silica gel column chromatography (ethyl acetate/petroleum ether (v/v) = 0-5%) to obtain compound C1-6.

**¹H NMR** (400 MHz, Chloroform-d) δ 7.97 (d, *J =* 2.3 Hz, 1H), 7.53 (dt, *J =* 8.3, 1.8 Hz, 1H), 7.08 (dd, *J* = 8.4, 1.0 Hz, 1H), 3.98 (ddd, *J* = 6.8, 5.6, 1.3 Hz, 2H), 2.90 (t, *J =* 6.2 Hz, 2H), 1.46 (d, *J =* 1.6 Hz, 9H).

### Step 6: preparation of compound C1-7

Compound C1-6 (3.17 g, 10.0 mmol), tetrakis(triphenylphosphine)palladium (3.17 g, 10.0 mmol, 0.1 equiv) and tributyl (1-ethoxyethylene) tin (5.43 g, 15.0 mmol, 1.5 equiv) were added to *N*,*N*-dimethylformamide (30.0 mL) solution, and the system was heated to 100°C and reacted for 16.0 hours under argon atmosphere. The reaction mixture was filtered to remove the residue and washed with ethyl acetate, and the organic phase was concentrated to obtain compound C1-7, which was directly used in the next step without further purification.

MS (ESI) *m*/*z* (M+H-56)⁺ = 253.8.

### Step 7: preparation of compound C1-8

Compound C1-7 (309.0 mg, 1.0 mmol) and N-chlorosuccinimide (134.0 mg, 1.0 mmol, 1.0 equiv) were added to a mixed solution of tetrahydrofuran (3.0 mL) and water (1.0 mL), and the system was stirred for 1.0 hour in ice bath. The reaction mixture was added with saturated aqueous ammonium chloride solution (10.0 mL), extracted with ethyl acetate (10.0 mL × 3 times), and the organic phases were combined, washed with saturated brine (10.0 mL × 2 times), and the organic phases were combined, dried over anhydrous sodium sulfate and concentrated, and the crude product was purified by silica gel column chromatography (ethyl acetate/petroleum ether (v/v) = 0-50%) to obtain compound C1-8.

MS (ESI) *m*/*z* (M+H-56)⁺ = 259.8.

### Step 8: preparation of compound C1-9

Compound C1-8 (3.15 g, 10.0 mmol), compound A1-10 (2.30 g, 12.0 mmol, 1.2 equiv) and potassium carbonate (4.14 g, 30.0 mmol, 3.0 equiv) were added to acetonitrile (30.0 mL) solution, and the system was heated to 80°C and stirred for 2.0 hours. The reaction mixture was directly concentrated, and the crude product was purified by silica gel column chromatography (ethyl acetate/petroleum ether (v/v) = 0-50%) to obtain compound C1-9.

MS (ESI) *m*/*z* (M+H)⁺ = 469.0.

### Step 9: preparation of compound C1-10

Compound C1-9 (2.33 g, 5.0 mmol) was dissolved in methanol (25.0 mL) solution, after being cooled to 0°C, sodium borohydride (380.0 mg, 10.0 mmol, 2.0 equiv) was gradually added to the reaction mixture in small amounts, and the system was continued to react for 30 minutes. The reaction mixture was quenched by adding acetone (2.0 mL) and concentrated, the crude product was purified by silica gel column chromatography (methanol/dichloromethane (v/v) = 0-1%) to obtain compound C1-10.

MS (ESI) *m*/*z* (M+H)⁺ = 470.8.

### Step 10: preparation of compound C1-11

Compound C1-10 (2.0 g, 4.25 mmol) was dissolved in dichloromethane (20.0 mL) solution, after being cooled to 0°C, concentrated sulfuric acid (10.0 mL) was gradually added dropwise to the reaction mixture in small amounts. After the addition, the system was heated to room temperature (25°C) and stirred overnight. The reaction mixture was added to ice water to be quenched, then the pH of which was adjusted to 8-9 by adding saturated aqueous sodium carbonate solution, finally the reaction mixture was extracted with dichloromethane (100.0 mL × 3 times), and the organic phase was dried over anhydrous sodium sulfate and concentrated, and the crude product was separated and purified to obtain compound C1-11.

**¹H NMR** (400 MHz, Chloroform-d) δ 7.72 (d, *J* = 1.8 Hz, 1H), 7.26 - 7.20 (m, 2H), 7.17 (d, *J =* 8.0 Hz, 1H), 6.75 (d, *J =* 2.0 Hz, 1H), 4.62 (t, *J =* 7.9 Hz, 1H), 4.25 (t, *J* = 6.1 Hz, 1H), 3.80 (q, *J =* 6.5 Hz, 2H), 3.66 (t, *J =* 14.3 Hz, 2H), 2.97 (t, *J =* 6.1 Hz, 3H), 2.63 (dd, *J* = 11.7, 7.2 Hz, 1H), 2.48 (s, 3H).

MS (ESI) *m*/*z* (M+H)⁺ = 397.0.

Similar to the synthesis of embodiment A1, embodiment C1 was synthesized from C1-11, as shown in Table 5 below:

**Table 5: Structural formula and analysis data of embodiment C1**

| | |
|---|---|
| Structural formula | 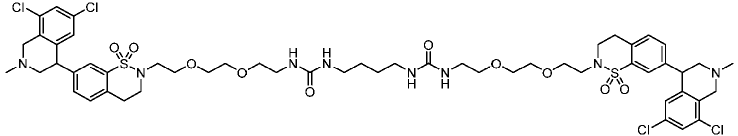 |
| Analysis data | **¹H NMR** (400 MHz, Chloroform-d) δ 7.64 (d, *J =* 2.0 Hz, 2H), 7.39 - 7.34 (m, 2H), 7.34 - 7.31 (m, 2H), 7.28 (d, *J =* 7.9 Hz, 2H), 6.74 (t, *J =* 1.4 Hz, 2H), 5.32 - 5.19 (m, 4H), 4.86 (s, 2H), 4.37 (s, 2H), 4.15 (d, *J =* 16.0 Hz, 2H), 3.98 (m, 4H), 3.70 (t, *J =* 5.2 Hz, 4H), 3.60 (dd, *J* = 6.1, 2.9 Hz, 4H), 3.55 (dd, *J =* 5.6, 2.3 Hz, 4H), 3.47 (t, *J =* 5.4 Hz, 4H), 3.43 (t, *J =* 5.2 Hz, 4H), 3.29 (q, *J =* 5.2 Hz, 4H), 3.24 (s, 2H), 3.05 (t, *J =* 6.3 Hz, 8H), 2.93 (s, 6H), 1.38 (q, *J =* 3.3 Hz, 4H). |
| | MS (ESI) *m*/*z* (M+H)⁺ = 1197.2. |

### Synthesis of embodiment D1

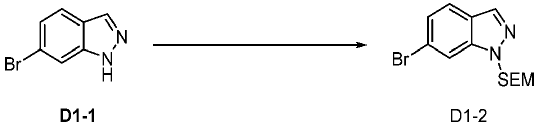

Compound D1-1 (1.96 g, 10 mmol) was dissolved in dichloromethane (20 mL), to which a solution of potassium hydroxide (1.4 g, 25 mmol) in water (3 mL) was added at 0 °C, followed by tetrabutylammonium bromide (64 mg, 0.2 mmol) and chloromethyltrimethylsilyl ethyl ether (2.1 mL, 12 mmol) were added sequentially, and the reaction was stirred at 0°C for 1 hour. The reaction mixture was diluted with ethyl acetate (100 mL), washed with water, and the organic phases were combined, dried over anhydrous sodium sulfate, filtered, and the filtrate was concentrated to obtain the crude product, which was purified by silica gel column chromatography (ethyl acetate/petroleum ether (v/v) = 10%) to obtain compound D1-2.

MS (ESI) *m*/*z* (M+H)⁺ = 326.8.

Similar to the synthesis of intermediate B1-6, intermediate D1-7 was synthesized, as shown in Table 6 below:

**Table 6: Structural formula and analysis data of intermediate D1-7**

| Intermediates | Structural formula | Analysis data |
|---|---|---|
| D1-7 | 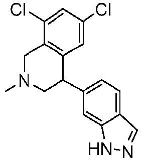 | ¹H NMR (400 MHz, Chloroform-*d*) δ 10.09 (s, 1H), 8.05 (d, *J =* 1.0 Hz, 1H), 7.70 (dd, *J* = 8.3, 0.8 Hz, 1H), 7.31 (d, *J = 1.2* Hz, 1H), 7.26 (s, 1H), 6.97 (dd, *J* = 8.4, 1.4 Hz, 1H), 6.81 (dd, *J* = 2.1, 1.0 Hz, 1H), 4.44 (s, 1H), 3.88 (d, *J =* 16.3 Hz, 1H), 3.65 (d, *J =* 16.1 Hz, 1H), 3.09 (d, *J* = 10.1 Hz, 1H), 2.80 - 2.68 (m, 1H), 2.55 (s, 3H). |
| | | MS (ESI) m/z (M+H)⁺ = 331.8 |
| | | HPLC retention time was 6.632 min. |
| | | Separation conditions: column: Waters XBridge 4.6*100mm, 3.5µm; column temperature: 40°C; mobile phase: water (10mM ammonium bicarbonate)-acetonitrile; acetonitrile: 5%-95% 7 min, 95% 8min; flow rate: 1.2 mL/min. |
| D1-7-S | 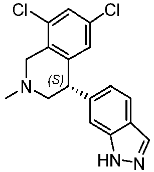 | D1-7 was split to obtain D1-7-S, and the specific splitting method was as follows: |
| | | Instrument: Waters UPC2 analytical SFC (SFC-H); column: ChiralPak AD, 150×4.6mm I.D., 3µm; mobile phase: A represented CO2, B represented ethanol (0.05%DEA); gradient: from 5 % to 40% B within 5 minutes and hold 40% for 2.5 minutes, then hold 5% B for 2.5 minutes; flow rate: 2.5 mL/min; back pressure: 100 bar; column temperature: 35°C; wavelength: 220nm. |
| | | Retention time: 6.095 min. |
| | | MS (ESI) m/z M+H⁺ = 332.1. |

Similar to the synthesis of embodiment B3, embodiments D1-P1 and D1-P2 were synthesized from D1-7, as shown in Table 7 below:

**Table 7**

| | |
|---|---|
| | 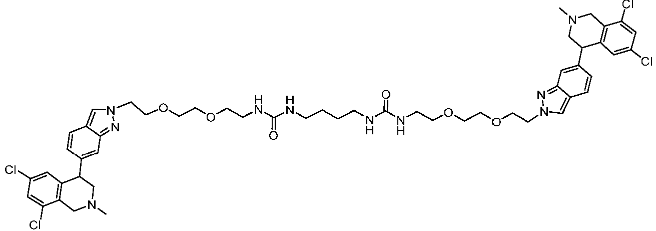 |
| Embodiment D1-P1 | ¹H NMR (400 MHz, Methanol-d4) δ 8.21 (d, J = 0.9 Hz, 2H), 7.62 (dd, J = 8.7, 0.9 Hz, 2H), 7.43 (d, J = 1.3 Hz, 2H), 7.29 (dd, J = 2.1, 0.8 Hz, 2H), 6.79 (dd, J = 8.7, 1.5 Hz, 4H), 4.57 (t, J = 5.1 Hz, 4H), 4.36 (dd, J = 9.2, 5.9 Hz, 2H), 3.98 - 3.86 (m, 6H), 3.57 - 3.51 (m, 5H), 3.51 - 3.46 (m, 5H), 3.36 (t, J = 5.4 Hz, 4H), 3.16 (t, J = 5.3 Hz, 4H), 3.11 - 3.01 (m, 6H), 2.65 (dd, J = 11.7, 9.3 Hz, 2H), 2.47 (s, 6H), 1.45 - 1.38 (m, 4H). |
| | MS (ESI) m/z M+H⁺ = 1067.40 |
| | HPLC 100% purity; retention time was 7.562 min. |
| | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |
| Embodiment D1-P2 | 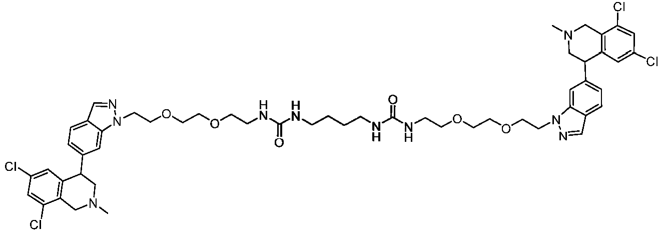 |
| | ¹H NMR (400 MHz, DMSO-d6) δ 8.02 (d, J = 0.8 Hz, 2H), 7.64 (d, J = 8.3 Hz, 2H), 7.58 (d, J = 1.2 Hz, 2H), 7.47 (d, J = 2.1 Hz, 2H), 6.95 (dd, J = 8.4, 1.3 Hz, 2H), 6.79 (dd, J = 2.1, 0.9 Hz, 2H), 5.90 (t, J = 5.7 Hz, 2H), 5.75 (t, J = 5.7 Hz, 2H), 4.54 - 4.47 (m, 4H), 4.39 (t, J = 6.5 Hz, 2H), 3.82 (t, J = 5.4 Hz, 4H), 3.62 (q, J = 16.1 Hz, 4H), 3.44 (dd, J = 5.8, 3.8 Hz, 4H), 3.38 - 3.35 (m, 4H), 3.24 (t, J = 5.7 Hz, 4H), 3.05 (q, J = 5.6 Hz, 4H), 2.97 - 2.88 (m, 6H), 2.72 - 2.65 (m, 2H), 2.39 (s, 6H), 1.33 - 1.25 (m, 4H). |
| | MS (ESI) m/z M+H⁺ = 1067.40 |
| | HPLC 100% purity; retention time was 8.046 min. |
| | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |

Similar to the synthesis of embodiment B3, embodiment D1-P1-S was synthesized from D1-7-S, as shown in Table 8 below:

**Table 8**

| | |
|---|---|
| Embodiment D1-P1-S | 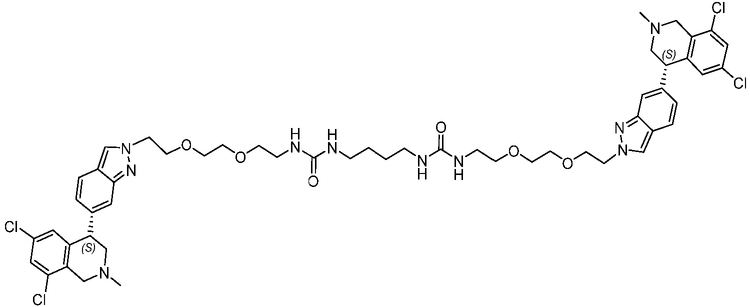 |
| | ¹H NMR (400 MHz, Chloroform-d) δ 8.00 (d, *J =* 2.0 Hz, 2H), 7.58 (dd, *J =* 8.7, 0.8 Hz, 2H), 7.50 (d, *J =* 1.3 Hz, 2H), 7.22 (dd, *J =* 2.1, 0.7 Hz, 2H), 6.82 (ddd, *J =* 5.7, 3.5, 1.4 Hz, 4H), 5.37 (d, *J =* 5.7 Hz, 2H), 5.23 (t, *J =* 5.7 Hz, 2H), 4.56 (t, *J* = 5.3 Hz, 4H), 4.33 (t, *J* = 7.0 Hz, 2H), 4.00 (t, *J* = 5.3 Hz, 4H), 3.85 (d, *J =* 16.1 Hz, 2H), 3.60 - 3.46 (m, 10H), 3.43 (t, *J =* 5.0 Hz, 4H), 3.29 (q, *J =* 5.3 Hz, 4H), 3.14 (d, *J =* 5.8 Hz, 4H), 3.04 (dd, *J =* 11.7, 5.5 Hz, 2H), 2.68 (dd, *J =* 11.6, 8.4 Hz, 2H), 2.49 (s, 6H), 1.48 - 1.39 (m, 4H). |

### Synthesis of embodiment D2-P1 hydrochloride

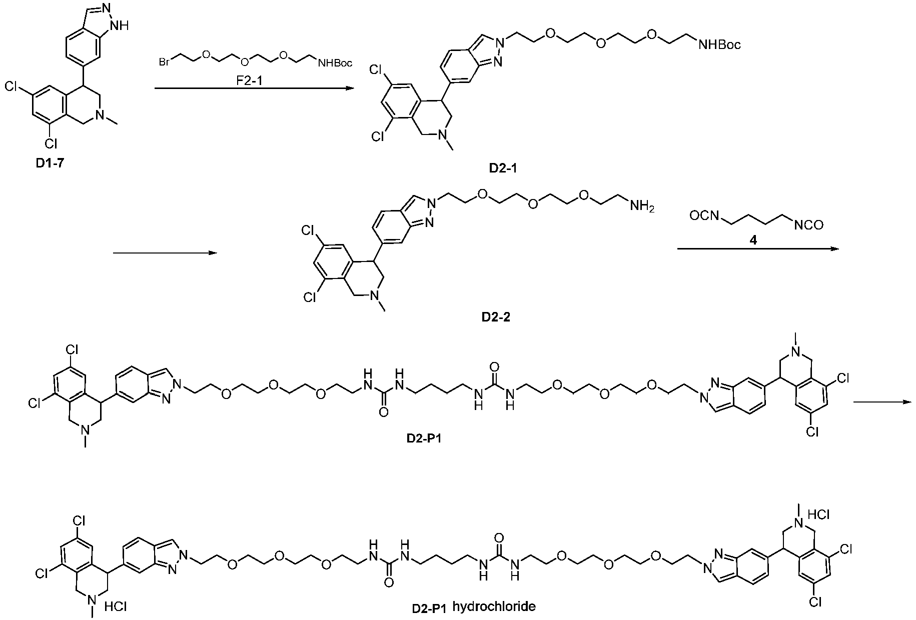

### Step 1: preparation of compound D2-P1

Similar to the synthesis of embodiment B3, embodiment D2-P1 was synthesized from D1-7.

### Step 2: preparation of compound D2-P1 hydrochloride

D2-P1 (113 mg, 0.1 mmol) was dissolved in a mixed solvent of methanol (2 mL) and water (3 mL), to which concentrated hydrochloric acid (1 mL) was added at room temperature (25°C), and the system was ultrasonicated at room temperature for 30 minutes. The system was concentrated and freeze-dried to obtain compound D2-P1 hydrochloride.

¹H NMR (400 MHz, DMSO-*d*₆, T=60 °C) δ 12.13 (s, 2H), 8.38 (d, *J =* 0.8 Hz, 2H), 7.72 (d, *J =* 8.6 Hz, 2H), 7.58 (d, *J =* 2.1 Hz, 4H), 6.78 (s, 4H), 4.93 - 4.76 (m, 2H), 4.59 (t, *J* = 5.4 Hz, 6H), 4.50 - 4.36 (m, 2H), 3.93 (t, *J =* 5.4 Hz, 4H), 3.84 - 3.71 (m, 2H), 3.64 (t, *J =* 11.9 Hz, 2H), 3.54 (dd, *J =* 5.7, 3.3 Hz, 4H), 3.51 - 3.47 (m, 4H), 3.47 - 3.42 (m, 8H), 3.35 (t, *J =* 5.9 Hz, 4H), 3.12 (t, *J =* 5.8 Hz, 4H), 3.03 - 2.90 (m, 10H), 1.37 - 1.30 (m, 4H).

MS (ESI) m/z (M+H)⁺ = 1155.60.

HPLC retention time was 7.417 min.

Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min.

### Synthesis of embodiment D2-P2 hydrochloride

Similar to the synthesis of D2-P1 hydrochloride, embodiment D2-P2 hydrochloride was synthesized from D1-7.

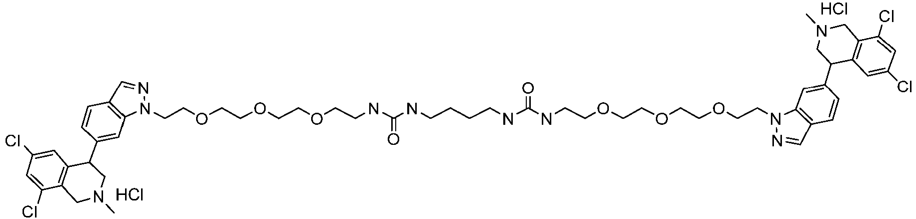

¹H NMR (400 MHz, DMSO-*d*₆) δ 12.22 (d, *J* = 57.8 Hz, 2H), 8.10 (s, 2H), 7.81 - 7.72 (m, 4H), 7.69 - 7.62 (m, 2H), 6.91 (dd, *J =* 8.3, 1.3 Hz, 2H), 6.75 - 6.61 (m, 2H), 4.97 - 4.76 (m, 2H), 4.75 - 4.51 (m, 6H), 4.51 - 4.33 (m, 2H), 3.97 - 3.60 (m, 8H), 3.51 - 3.42 (m, 4H), 3.42 - 3.34 (m, 12H), 3.31 (t, *J =* 5.8 Hz, 4H), 3.11 (t, *J =* 5.8 Hz, 4H), 3.05 - 2.84 (m, 10H), 1.39 - 1.21 (m, 4H).

MS (ESI) m/z (M+H)⁺ = 1155.6.

HPLC retention time was 7.976 min.

Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min.

### Synthesis of embodiment E1

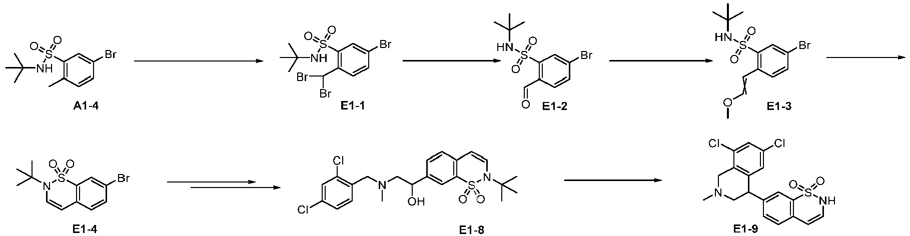

### Step 1: preparation of compound E1-1

Compound A1-4 (10 g, 32.8 mmol), N-bromosuccinimide (12.85 g, 72.16 mmol), azobisisobutyronitrile (1.08 g, 6.56 mmol) were dissolved in carbon tetrachloride (80 mL). The reaction was heated to 80°C and stirred for 18 hours. After the reaction, the mixture was filtered by celite, washed with water and concentrated to obtain the crude product. The crude product was purified by silica gel column chromatography (ethyl acetate/petroleum ether (v/v) = 0-10% to obtain compound E1-1.

MS (ESI) *m*/*z* (M+Na)⁺ = 482.6.

¹H NMR (400 MHz, Chloroform-d) δ 8.08 (d, *J =* 2.1 Hz, 1H), 8.05 (d, *J =* 8.5 Hz, 1H), 7.77 (dd, *J =* 8.5, 2.1 Hz, 1H), 7.58 (s, 1H), 4.67 (s, 1H), 1.27 (s, 9H).

### Step 2: preparation of compound E1-2

Compound E1-1 (10 g, 21.7 mmol) was dissolved in a mixed solvent of tetrahydrofuran (75 mL) and water (25 mL), to which silver nitrate (11.1 g, 65.1 mmol) was added at room temperature (25°C), and the reaction was heated to 80°C and stirred for 2 hours. The reaction mixture was cooled to room temperature (25°C), filtered by celite, concentrated to 1/3 volume, diluted with ethyl acetate (200 mL), washed with water, and the organic phase was dried over anhydrous sodium sulfate, filtered, and the filtrate was concentrated to obtain the crude product. The crude product was purified by silica gel column chromatography (ethyl acetate/petroleum ether (v/v) = 0-10% to obtain compound E1-2.

MS (ESI) *m*/*z* (M-55) = 264.

### Step 3: preparation of compound E1-3

(Methoxymethyl) triphenylphosphonium chloride (8.17 g, 23.9 mmol) was dissolved in tetrahydrofuran (50 mL), to which sodium bis(trimethylsilyl)amide (12 mL, 23.9 mmol, 2 M) was added under argon atmosphere at 0°C. After the addition, the system was stirred at 0°C for 1 hour. The reaction mixture was cooled to -78°C, to which a tetrahydrofuran solution (10 mL) of the compound E1-2 in step 2 was added dropwise, and the system was slowly heated to room temperature (25°C) and stirred for 16 hours. The reaction mixture was concentrated to obtain the crude product, which was purified by silica gel column chromatography (ethyl acetate/petroleum ether (v/v) = 0-20%) to obtain compound E1-3.

¹H NMR (400 MHz, Chloroform-d) δ 8.16 (d, *J* = 2.2 Hz, 1H), 7.55 (ddd, *J* = 8.3, 2.1, 0.6 Hz, 1H), 7.24 (d, *J* = 0.6 Hz, 1H), 6.96 (d, *J* = 12.8 Hz, 1H), 6.43 (d, *J* = 12.8 Hz, 1H), 4.46 (s, 1H), 3.74 (s, 3H), 1.17 (s, 9H).

### Step 4: preparation of compound E1-4

Compound E1-3 (2.2 g, 6.3 mmol) was dissolved in tetrahydrofuran (30 mL), to which 2 *N* HCl (31 mL, 63 mmol) was added at room temperature (25°C). After the addition, the reaction was heated to 50°C and stirred for 1 hour. The reaction mixture was concentrated to remove tetrahydrofuran, extracted with ethyl acetate (100 mL), and the organic phases were combined, dried over anhydrous sodium sulfate, filtered, and the filtrate was concentrated to obtain the crude product, which was purified by silica gel column chromatography (ethyl acetate/petroleum ether (v/v) = 0-20%) to obtain compound E1-4.

¹H NMR (400 MHz, Chloroform-d) δ 8.02 (dt, *J* = 2.0, 0.5 Hz, 1H), 7.66 (dd, *J* = 8.4, 2.0 Hz, 1H), 7.23 (d, *J =* 8.3 Hz, 1H), 6.85 (d, *J =* 8.2 Hz, 1H), 6.29 - 6.24 (m, 1H), 1.62 (s, 9H).

### Step 5: preparation of compound E1-8

Similar to the synthesis of compound B1-5, compound E1-8 was synthesized.

MS (ESI) *m*/*z* (M+H)⁺ = 469.2.

### Step 6: preparation of compound E1-9

Compound E1-8 (1.3 g, 2.8 mmol) was dissolved in 1,2-dichloroethane (10 mL), to which aluminum trichloride (1.85 g, 14 mmol) was added under nitrogen atmosphere, and the reaction was heated to 80 °C and continued to stir for 0.5 hour. The system was cooled to room temperature (25°C), diluted with ethyl acetate (200 mL), the pH of which was adjusted to alkaline with saturated aqueous sodium bicarbonate solution, filtered with celite, washed with water, and the organic phases were combined, dried over anhydrous sodium sulfate, filtered, and the filtrate was concentrated to obtain the crude product, which was purified by silica gel column chromatography (ethyl acetate/petroleum ether (v/ v) = 0-60%) to obtain compound E1-9.

MS (ESI) *m*/*z* (M+H)⁺ = 395.2.

¹H NMR (400 MHz, DMSO-*d*₆) δ 11.12 (s, 1H), 7.65 (d, *J =* 1.7 Hz, 1H), 7.51 (d, *J*= 2.1 Hz, 1H), 7.46 (dd, *J =* 8.2, 1.8 Hz, 1H), 7.41 (d, *J =* 8.1 Hz, 1H), 6.92 (d, *J =* 2.1 Hz, 1H), 6.80 (d, *J = 7.6* Hz, 1H), 6.19 (d, *J =* 7.6 Hz, 1H), 4.42 (t, *J =* 5.3 Hz, 1H), 3.75 (d, *J =* 16.2 Hz, 1H), 3.45 (d, *J =* 16.2 Hz, 1H), 2.84 (dd, *J =* 11.6, 5.0 Hz, 1H), 2.74 - 2.68 (m, 1H), 2.37 (s, 3H).

Similar to the synthesis of embodiment C1, embodiment E1 was synthesized from E1-9, as shown in Table 9 below:

**Table 9**

| Structural formula | 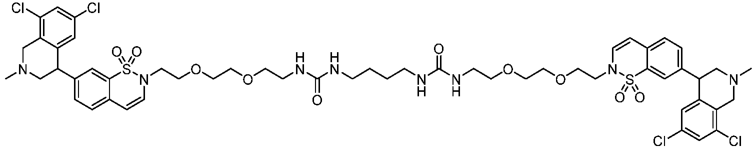 |
|---|---|
| Analysis data | ¹H NMR (400 MHz, DMSO-*d*₆) δ 7.73 (d, *J =* 1.6 Hz, 2H), 7.57 - 7.41 (m, 6H), 6.97 - 6.90 (m, 4H), 6.35 (d, *J* = 7.9 Hz, 2H), 5.92 (t, *J =* 5.7 Hz, 2H), 5.79 (t, *J* = 5.8 Hz, 2H), 4.45 (t, *J=* 5.1 Hz, 2H), 3.84 (t, *J =* 5.5 Hz, 4H), 3.76 (d, *J =* 16.2 Hz, 2H), 3.61 (t, *J =* 5.4 Hz, 4H), 3.51 - 3.46 (m, 4H), 3.45 - 3.38 (m, 6H), 3.30 (t, *J =* 5.7 Hz, 4H), 3.09 (q, *J =* 5.7 Hz, 4H), 2.94 (q, *J =* 5.8 Hz, 4H), 2.81 (dd, *J =* 11.7, 5.0 Hz, 2H), 2.71 (dd, *J =* 11.7, 5.2 Hz, 2H), 2.36 (s, 6H), 1.35 - 1.25 (m, 4H). |
| | MS (ESI) m/z M+H⁺ = 1193.4. |
| | HPLC 100% purity; retention time was 8.051 min. |
| | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |

### Synthesis of embodiment F1

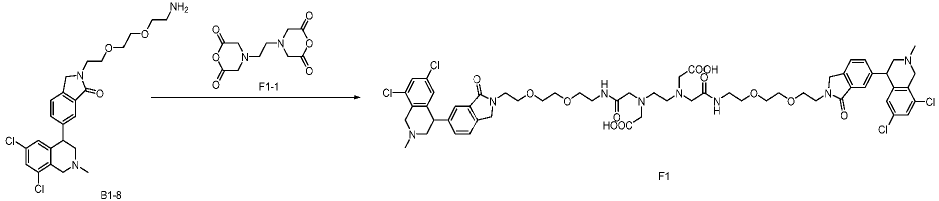

### Step 1: preparation of compound F1

Compound B1-8 (91 mg, 0.19 mmol) was dissolved in N,N-dimethylformamide (2 mL), to which ethylenediaminetetraacetic dianhydride (19 mg, 0.07 mmol) and triethylamine were added (57 mg, 0.57 mmol). After the addition, the system was reacted at room temperature (25°C) for 2 hours. The reaction mixture was purified by high performance preparative liquid chromatography (separation conditions: column: Agilent 10 Prep-C18 250×21.2mm; column temperature: 25°C; mobile phase: water (10mM/L ammonium bicarbonate)-acetonitrile; acetonitrile: 60%-80% 12min, flow rate: 30 mL/min) to obtain compound F1.

¹H NMR (400 MHz, Methanol-*d*₄) δ 7.50 (d, *J* = 1.5 Hz, 2H), 7.46 (d, *J* = 7.9 Hz, 2H), 7.36 (dd, *J =* 7.9, 1.6 Hz, 2H), 7.26 (d, *J =* 2.3 Hz, 2H), 6.69 (dd, *J =* 2.2, 1.0 Hz, 2H), 4.51 (s, 4H), 4.45 - 4.39 (m, 2H), 3.92 (d, *J =* 16.1 Hz, 2H), 3.76 - 3.63 (m, 10H), 3.57 (d, *J =* 1.5 Hz, 4H), 3.52 - 3.33 (m, 18H), 3.15 (q, *J =* 5.7 Hz, 6H), 3.03 (s, 4H), 2.77 (dd, *J =* 11.9, 8.9 Hz, 2H), 2.52 (s, 6H).

MS (ESI) m/z M+H⁺ = 1211.20.

HPLC 100% purity; retention time was 6.180 min.

Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min.

### Synthesis of embodiment F2

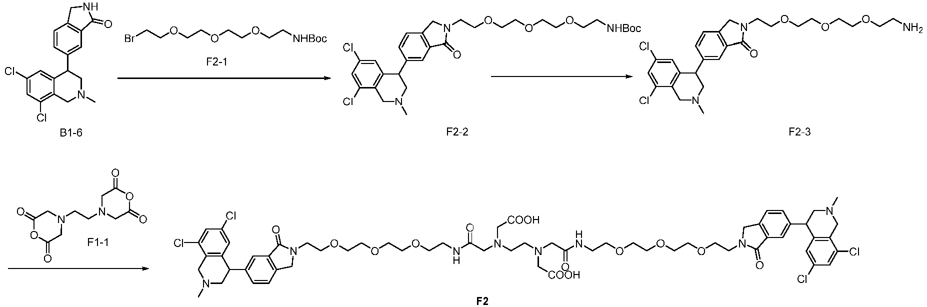

### Step 1: preparation of compound F2-2

Compound B1-6 (200 mg, 0.58 mmol), F2-1 (410 mg, 1.15 mmol), cesium carbonate (563 mg, 1.73 mmol), 18-crown-6 (15.2 mg, 0.06 mmol) were dissolved in acetonitrile (1.5 mL), and the system was replaced with a nitrogen stream, and heated to 90°C and stirred for 16 hours under nitrogen protection. The reaction mixture was cooled to room temperature, filtered, and the filtrate was purified by C18 reversed-phase silica gel column chromatography (acetonitrile/0.1% aqueous ammonium bicarbonate (v/v) = 5-75%) to obtain compound F2-2.

MS (ESI) m/z (M+H)⁺ = 622.0.

### Step 2: preparation of compound F2-3

Compound F2-2 (260 mg, 0.42 mmol) was dissolved in anhydrous dichloromethane (4 mL), to which trifluoroacetic acid (1 mL) was added under ice bath, and the system was heated to room temperature (25°C) and stirred for 3 hours. The reaction mixture was concentrated under vacuum to obtain the crude product, dissolved with dichloromethane (20 mL), to which water (20 mL) was added, and the pH of which was adjusted to 9 with saturated aqueous sodium bicarbonate solution, and separated, and the aqueous phase was extracted with dichloromethane (20 mL × 2), and the organic phases were combined, dried over anhydrous sodium sulfate, filtered, and the filtrate was concentrated to obtain compound F2-3.

MS (ESI) m/z (M+H)⁺ = 522.0.

Similar to the synthesis of embodiment F1, embodiment F2 was synthesized from F2-3, as shown in Table 10 below:

**Table 10: Structural formula and analysis data of embodiments F2**

| Structural formula | 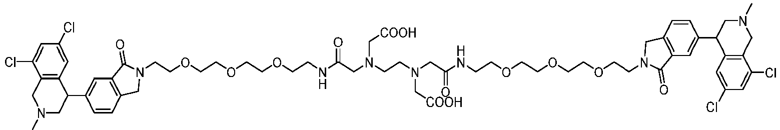 |
|---|---|
| Analysis data | ¹H NMR (400 MHz, DMSO-d6) δ 7.95 (t, *J =* 5.8 Hz, 2H), 7.53 - 7.45 (m, 8H), 6.85 (d, *J =* 2.1 Hz, 2H), 4.50 (s, 4H), 4.40 (t, *J =* 5.2 Hz, 2H), 3.72 (d, *J =* 16.1 Hz, 2H), 3.63 (dq, *J* = 8.8, 4.2 Hz, 8H), 3.53 - 3.45 (m, 12H), 3.39-3.36 (m, 12H), 3.24-3.19 (m, 8H), 2.84 (dd, *J* = 11.5, 5.1 Hz, 2H), 2.68-2.64 (m, 6H), 2.36 (s, 6H). |
| | MS (ESI) m/z (M+H)⁺/2 = 651.3. |
| | HPLC 91.13% purity; retention time was 3.392 min. |
| | Separation conditions: column: Agilent 10 Prep-C18 250*21.2mm, 3.5um; column temperature: 25°C; mobile phase: water (0.1% formic acid)-acetonitrile; acetonitrile: 12%-32% 9min, flow rate: 30 mL/min) |

### Synthesis of embodiment G1

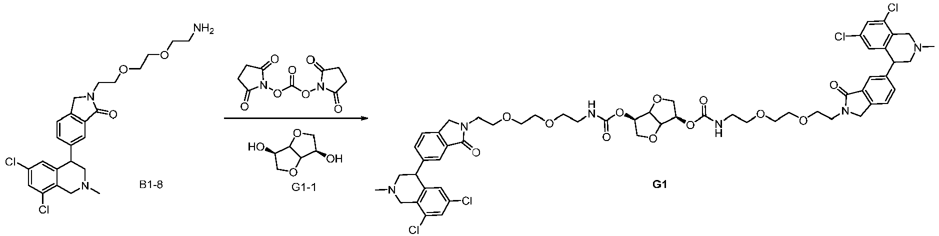

1,4:3,6-bis-anhydromannitol (15 mg, 0.102 mmol) was dissolved in *N,N-*dimethylformamide (2 mL), to which N,N'-disuccinimidyl carbonate (52.2 mg, 0.204 mmol) was added, and the system was stirred at room temperature (25°C) for 1 hour. Compound B1-8 (108 mg, 0.226 mmol) and triethylamine (30.9 mg, 0.306 mmol) were added to the above reaction mixture, and after the addition was complete, the system was reacted at 60°C for 16 hours, the reaction mixture was purified by high performance preparative liquid chromatography (separation conditions: column: Agilent 10 Prep-C18 250×21.2mm; column temperature: 25°C; mobile phase: water (10mM/L NH₄HCO₃)-acetonitrile; acetonitrile: 60%-90% 12min, flow rate: 30 mL/min) to obtain compound G1.

¹H NMR (400 MHz, Methanol-*d*₄) δ 7.48 - 7.41 (m, 4H), 7.36 (dd, *J =* 7.9, 1.6 Hz, 2H), 7.22 (d, *J =* 2.0 Hz, 2H), 6.68 (d, *J =* 1.9 Hz, 2H), 4.50 (s, 6H), 4.34 - 4.27 (m, 2H), 3.85 - 3.60 (m, 14H), 3.59 - 3.43 (m, 12H), 3.35 (t, *J =* 5.5 Hz, 4H), 3.08 (t, *J =* 5.4 Hz, 4H), 2.95 (dd, *J =* 11.7, 5.6 Hz, 2H), 2.56 (dd, *J =* 11.7, 8.2 Hz, 2H), 2.37 (s, 6H).

MS (ESI) m/z M+H⁺ = 1155.40.

HPLC 100% purity; retention time was 7.593 min.

Separation conditions: column: Waters XBridge 4.6*100mm, 3.5um; column temperature: 40°C; mobile phase: water (10mM NH₄HCO₃)-acetonitrile; acetonitrile: 5%-95% 7 min, 95% 8min; flow rate: 1.2 mL/min.

### Synthesis of embodiment H1

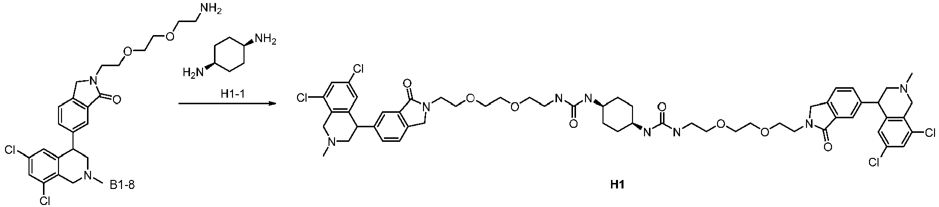

Compound B1-8 (110 mg, 0.23 mmol) was dissolved in N,N-dimethylformamide (2 mL), to which *N*,*N*'-carbonyldiimidazole (44.8 mg, 0.27 mmol) was added, after the addition, the system was stirred at room temperature (25°C) for 1 hour. After the complete conversion into active intermediate was monitored through sampling, cis-1,4-cyclohexanediamine (10.5 mg, 0.092 mmol) and triethylamine (81.8 mg, 0.81 mmol) were added to the reaction mixture, and the system was reacted at 80°C for 16 hours, the reaction mixture was purified by high performance preparative liquid chromatography (separation conditions: column: Agilent 10 Prep-C18 250×21.2mm; column temperature: 25°C; mobile phase: water (10mM/L NH₄HCO₃)-acetonitrile; acetonitrile: 60%-80% 12min, flow rate: 30 mL/min) to obtain compound H1.

¹H NMR (400 MHz, Methanol-*d*₄) δ 7.45 - 7.37 (m, 6H), 7.22 (d, *J =* 2.1 Hz, 2H), 6.69 - 6.65 (d, 2H), 4.48 (s, 4H), 4.32 - 4.27 (t, 2H), 3.71 - 3.62 (m, 10H), 3.54 - 3.42 (m, 12H), 3.30 (t, *J =* 5.2 Hz, 4H), 3.08 (dd, *J =* 6.5, 4.8 Hz, 4H), 2.96 - 2.89 (m, 2H), 2.53 (dd, *J* = 11.7, 8.1 Hz, 2H), 2.36 (s, 6H), 1.56 - 1.36 (m, 8H).

MS (ESI) m/z (M+H)⁺ = 1123.40.

HPLC 100% purity; retention time was 7.249 min.

Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min.

Similar to the synthesis of embodiment H1, embodiment H2 was synthesized, as shown in Table 11 below:

**Table 11: Structural formula and analysis data of embodiments H2**

| | |
|---|---|
| **Embodiment H2** | 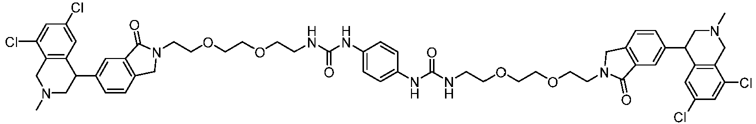 |
| | ¹H NMR (400 MHz, DMSO-d6) δ 8.32 (s, 2H), 7.51 - 7.43 (m, 8H), 7.20 (s, 4H), 6.84 (d, J = 2.0 Hz, 2H), 6.03 (t, J = 5.6 Hz, 2H), 4.51 (s, 4H), 4.39 (t, J = 5.4 Hz, 2H), 3.75 - 3.61 (m, 10H), 3.53 (ddt, J = 10.2, 7.5, 3.5 Hz, 10H), 3.40 (t, J = 5.5 Hz, 4H), 3.17 (q, J = 5.5 Hz, 4H), 2.84 (dd, J = 11.6, 5.1 Hz, 2H), 2.66 (dd, J = 11.5, 6.6 Hz, 2H), 2.36 (s, 6H). |

### Synthesis of embodiment I1

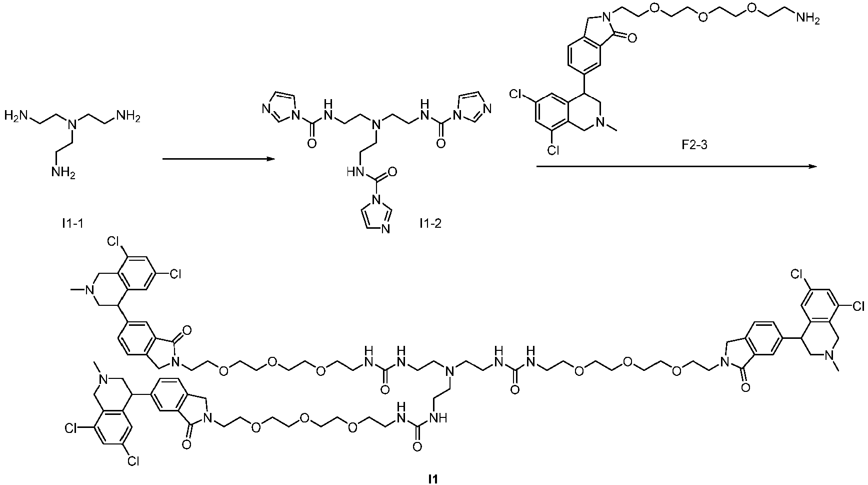

### Step 1: preparation of compound I1-2

N,N'-carbonyl diimidazole (487 mg, 3.00 mmol) was dissolved in *N,N-*dimethylformamide (1.5 ml), to which compound I1-1 (146 mg, 1.00 mmol) was added in batches at 0 °C, after the addition, the system was slowly heated to room temperature (25°C) and stirred for 2 h. The system was directly used in the next step without further purification.

MS (ESI) m/z (M+H)⁺ = 429.00

### Step 2: preparation of compound I1

Compound F2-3 (80 mg, 0.15 mmol) was dissolved in N,N-dimethylformamide (2 mL), after the addition, the system was heated to 75°C, to which the reaction solution of compound I1-2 (0.05 mL, 0.05 mmol) was added dropwise, after the addition, the system was stirred at 75°C for 17 hours. The reaction mixture was purified by high performance preparative liquid chromatography (separation conditions: column: Welch Xtimate^{®} C18 21.2x250mm; column temperature: 25°C; mobile phase: water (10mM/L ammonium bicarbonate)-acetonitrile; acetonitrile: 65%-95% 9min; flow rate 30 mL/min) to obtain compound I1.

¹H NMR (400 MHz, DMSO-*d*₆) δ 7.51-7.44 (m, 12H), 6.84 (d, *J* = 2.2 Hz, 3H), 5.95 (s, 6H), 4.50 (s, 6H), 4.39 (t, *J =* 5.3 Hz, 3H), 3.73-3.69 (m, 3H), 3.64-3.59 (m, 12H), 3.52 - 3.46 (m, 15H), 3.46 - 3.44 (m, 6H), 3.43 - 3.40 (m, 6H),3.32- 3.31 (m, 6H), 3.14 - 3.09 (m, 6H), 3.02-3.98 (m, 6H), 2.85-2.82 (m, 3H), 2.68-2.64 (m, 3H), 2.36 (s, 9H).

MS (ESI) m/z (M+2H)⁺/2 = 897.0.

HPLC 100% purity; retention time was 3.350 min.

Separation conditions: column: Waters Xselect CSH C18 4.6*100mm, 3.5um; column temperature: 60°C; mobile phase: water (0.01% trifluoroacetic acid)-acetonitrile (0.01% trifluoroacetic acid); acetonitrile: 5%- 95% 7 min; flow rate: 1.2 mL/min.

### Synthesis of embodiment I2

### Step 1: preparation of compound I2-3

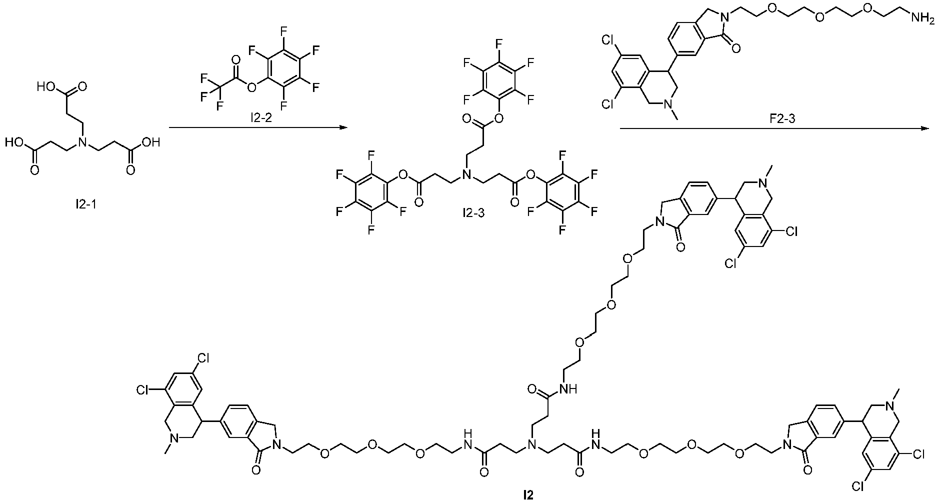

Compound I2-1 (300 mg, 1.29 mmol) was dissolved in dichloromethane (3 mL), to which compound I2-2 (1.26 g, 4.5 mmol) and triethylamine (911 mg, 9.0 mmol) were added sequentially at 0°C, after the addition, the system was slowly heated to room temperature (25°C) and stirred for 1 hour. The system was the reaction mixture of compound I2-3, which was directly used in the next step without further purification.

MS (ESI) m/z (M+H)⁺ = 732.00

### Step 2: preparation of compound I2

Compound F2-3 (80 mg, 0.15 mmol) and triethylamine (31 mg, 0.31 mmol) were dissolved in *N*,*N*-dimethylformamide (2 mL), to which the reaction mixture of compound I2-3 (0.12 mL, 0.05 mmol) was added dropwise at room temperature (25°C), after the addition, the system was stirred at room temperature (25°C) for 3 hours. The reaction mixture was purified by high performance preparative liquid chromatography (separation conditions: column: Welch Xtimate^{®} C18 21.2x250mm; column temperature: 25°C; mobile phase: water (10mM/L ammonium bicarbonate)-acetonitrile; acetonitrile: 65%-95% 9min; flow rate 30 mL/min) to obtain compound I2.

¹H NMR (400 MHz, DMSO-*d*₆) δ 7.91 (s, 3H), 7.50 - 7.45 (m, 12H), 6.84 (d, *J* = 2.0 Hz, 3H), 4.49 (s, 6H), 4.39 (t, *J =* 5.5 Hz, 3H), 3.71 (d, *J =* 16.2 Hz, 3H), 3.66-3.59 (m, 12H), 3.52-3.48 (m , 15H), 3.45 - 3.40 (m, 12H), 3.35-3.43 (m, 6H), 3.17-3.13 (m, 6H), 2.85-2.81 (m, 3H), 2.67-2.58 (m, 9H), 2.35 (s, 9H), 2.18 (s, 6H).

MS (ESI) m/z (M+2H)⁺/2 = 873.4.

HPLC 100% purity; retention time was 9.618 min.

Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min.

### Synthesis of embodiment I3

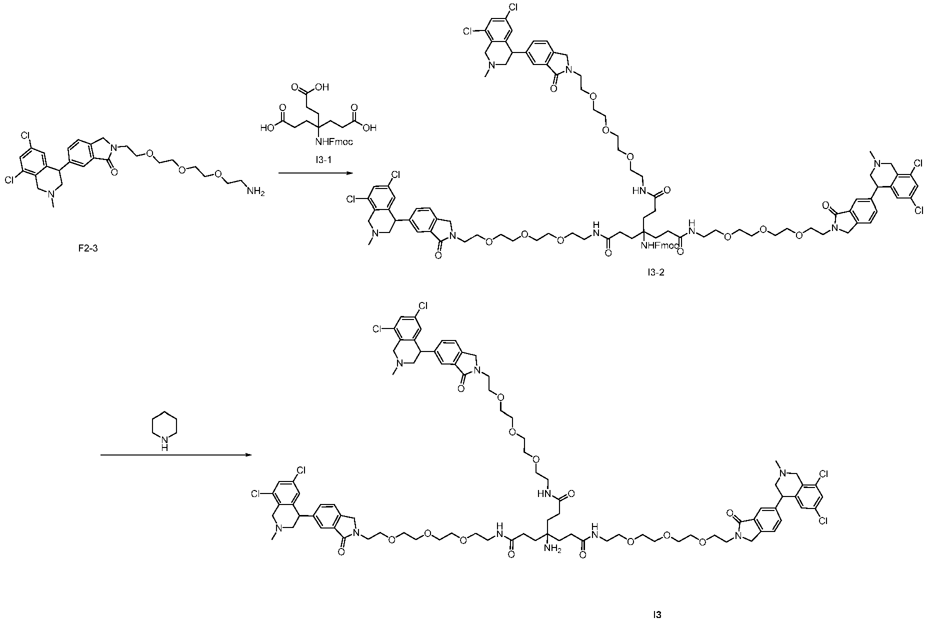

### Step 1: preparation of compound I3-2

4-(2-Carboxyethyl)-4-[[(9H-fluoren-9-methoxy)carbonyl]amino]heptanedioic acid (94 mg, 0.2 mmol) was dissolved in *N*,*N*-dimethylformamide (5 mL), to which *N,N-*diisopropylethylamine (170 mg, 1.32 mmol), 2-(7-azobenzotriazole)-*N*,*N*,*N*',*N*'-tetramethyluronium hexafluorophosphate (384 mg, 1.0 mmol) were added sequentially. After the addition, the system was stirred at room temperature (25°C) for 10 minutes, to which compound F2-3 (410 mg, 0.785 mmol) was added, after the addition, the system was stirred at room temperature (25°C) for 18 hours. The system was filtered, and the filtrate was purified by C18 reversed-phase silica gel column chromatography (acetonitrile/0.1% aqueous ammonium bicarbonate (v/v) = 5~95%) to obtain compound I3-2.

(ESI) m/z (M/2+H)⁺ = 992.0.

### Step 2: preparation of compound I3

Compound I3-2 (280 mg, 0.141 mmol) was dissolved in *N*,*N*-dimethylformamide (3 mL), to which piperidine (120 mg, 1.41 mmol) was added, after the addition, the system was stirred at room temperature (25°C) for 1 hour. The reaction mixture was purified by high performance preparative liquid chromatography (separation conditions: column: Agilent 10 Prep-C18 250×21.2mm; column temperature: 25°C; mobile phase: water (10 mM/L trifluoroacetic acid)-acetonitrile; acetonitrile: 10%-30% 12min, flow rate: 30 mL/min) to obtain compound I3.

¹H NMR (400 MHz, DMSO-*d*₆) δ 7.97 (t, *J* = 5.7 Hz, 3H), 7.55 - 7.42 (m, 12H), 6.84 (d, *J* = 2.0 Hz, 3H), 4.50 (s, 6H), 4.39 (t, *J =* 5.5 Hz, 3H), 3.71 (d, *J =* 16.1 Hz, 3H), 3.65 - 3.56 (m, 12H), 3.54 - 3.39 (m, 27H), 3.38 - 3.27 (m, 6H), 3.15 (q, *J =* 5.8 Hz, 6H), 2.83 (dd, *J =* 11.6, 5.1 Hz, 3H), 2.73 - 2.61 (m, 3H), 2.35 (s, 9H), 2.12 (t, *J =* 8.1 Hz, 6H), 1.67 - 1.51 (m, 6H).

MS (ESI) m/z (M/2+H)⁺ = 881.3.

HPLC 98.9% purity; retention time was 5.747 min.

Separation conditions: column: ZORBAX Extend-C18 4.6*150mm, 3.5um; column temperature: 30°C; mobile phase: water (10mM/L TFA)-acetonitrile; acetonitrile: 5%-5% 0-8 min, 5-95% 8-15min; flow rate: 1.0 mL/min.

### Synthesis of embodiment I4

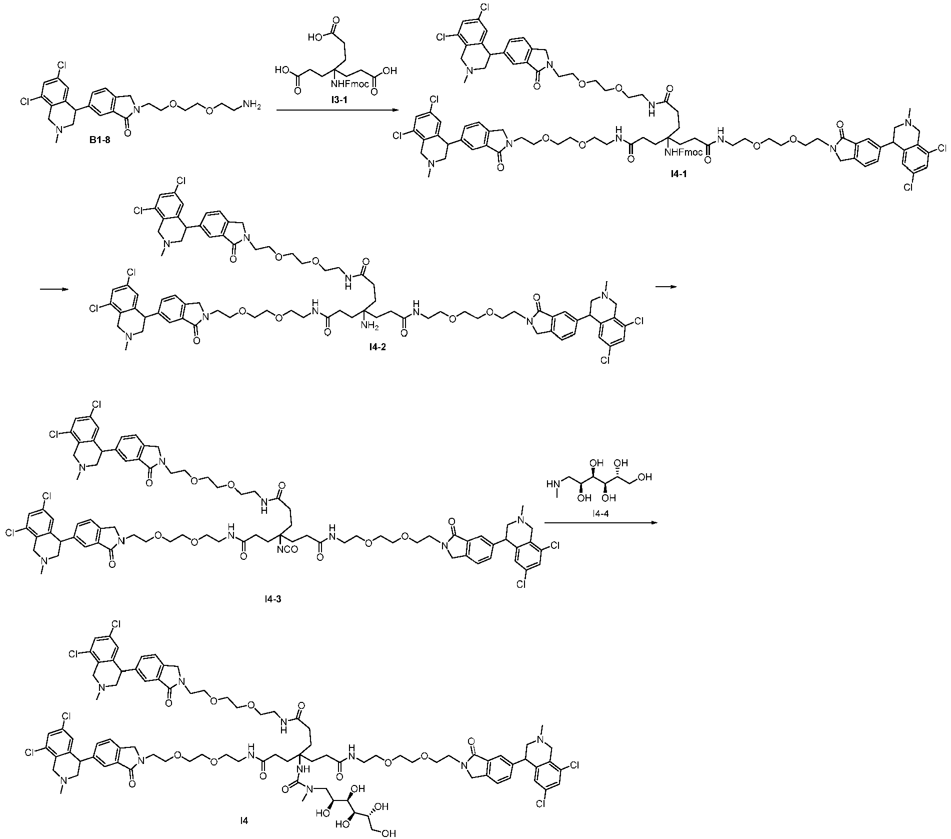

### Step 1: preparation of compound I4-2

Similar to the synthesis of compound I3, intermediate I4-3 was synthesized.

(ESI) m/z (M/2+H)⁺ = 814.5.

### Step 2: preparation of compound I4-3

Compound I4-2 (90 mg, 0.055 mmol) and triethylamine (33 mg, 0.33 mmol) were dissolved in a mixed solvent of tetrahydrofuran (2 mL) and dichloromethane (2 mL), and the system was cooled to 0°C under nitrogen atmosphere, to which triphosgene (49 mg, 0.165 mmol) was added. The system was diluted by adding ethyl acetate (100 mL), washed with saturated aqueous sodium bicarbonate solution and saturated aqueous sodium chloride solution sequentially, and the organic phases were combined, dried over anhydrous sodium sulfate, filtered, and the filtrate was concentrated to obtain compound I4-3, which was directly used in the next reaction step without further purification.

(ESI) m/z (M/2+H)⁺ = 827.5.

### Step 3: preparation of compound I4

Compound I4-3 (91 mg, 0.055 mmol) was dissolved in N,N-dimethylformamide (2 mL), to which compound I4-4 (32 mg, 0.165 mmol) was added at room temperature (25°C), after the addition, the system was stirred at room temperature (25°C) for 1 hour. The reaction mixture was purified by high performance preparative liquid chromatography (separation conditions: column: Agilent 10 Prep-C18 250×21.2mm; column temperature: 25°C; mobile phase: water (10mM/L ammonium bicarbonate)-acetonitrile; acetonitrile: 65%-95% 12min, flow rate: 30 mL/min) to obtain compound I4.

¹H NMR (400 MHz, DMSO-*d*₆) δ 7.79 (t, *J* = 5.6 Hz, 3H), 7.54 - 7.37 (m, 13H), 6.85 (d, *J =* 2.1 Hz, 3H), 5.75 (s, 1H), 5.13 (d, *J =* 4.4 Hz, 1H), 4.55 (dd, *J =* 14.2, 5.9 Hz, 2H), 4.51 - 4.46 (m, 7H), 4.41 - 4.35 (m, 4H), 3.76 - 3.55 (m, 19H), 3.54 - 3.43 (m, 18H), 3.37 - 3.34 (m, 5H), 3.17 - 3.08 (m, 7H), 2.83 (dd, *J =* 11.6, 5.1 Hz, 3H), 2.76 (s, 3H), 2.67 - 2.62 (m, 3H), 2.35 (s, 9H), 2.05 - 1.95 (m, 6H), 1.83 - 1.71 (m, 6H).

(ESI) m/z (M/2+H)⁺ = 924.8.

HPLC 100% purity; retention time was 8.367 min.

Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min.

### Synthesis of embodiment J1

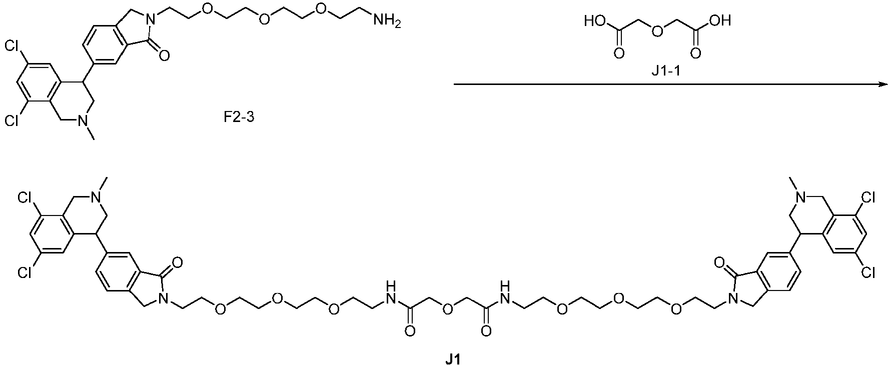

### Step 1: preparation of compound J1

Compound F2-3 (90 mg, 0.17 mmol) and compound J1-1 (7.7 mg, 0.06 mmol) were dissolved in *N*,*N*-dimethylformamide (2 mL), to which 2-(7-azobenzotriazole)-*N*,*N*,*N*',*N*'-tetramethyluronium hexafluorophosphate (66 mg, 0.17 mmol) and *N*,*N*-diisopropylethylamine (45 mg, 0.34 mmol) were added at room temperature (25°C), after the addition, the system was stirred at room temperature (25°C) for 2 hours. The reaction mixture was purified by high performance preparative liquid chromatography (separation conditions: column: Welch Xtimate^{®} C18 21.2x250mm; column temperature: 25°C; mobile phase: water (10mM/L ammonium bicarbonate)-acetonitrile; acetonitrile: 65%-95% 9min; flow rate 30 mL/min) to obtain compound J1.

¹H NMR (400 MHz, DMSO-*d*₆) δ 8.04 (t, *J =* 5.8 Hz, 2H), 7.51 - 7.45 (m, 8H), 6.85 (d, *J =* 2.0 Hz, 2H), 4.50 (s, 4H), 4.40 (t, *J =* 5.4 Hz, 2H), 3.92 (s, 4H), 3.72 (d, *J =* 16.2 Hz, 2H), 3.66-3.59 (m, 8H), 3.53-3.48 (m, 8H), 3.47-3.42 (m, 10H), 3.41-3.38 (m, 4H), 3.27-2.22 (m, 4H), 2.87-2.82 (m, 2H), 2.69 - 2.64 (m, 2H), 2.36 (s, 6H).

MS (ESI) m/z (M+H)⁺ = 1143.4.

HPLC 100% purity; retention time was 7.582 min.

Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min.

Similar to the synthesis of embodiment J1, embodiments J2- J3 were synthesized, as shown in Table 12 below:

**Table 12: Structural formula and analysis data of embodiments J2 - J3**

| | |
|---|---|
| **Embodiment J2** | 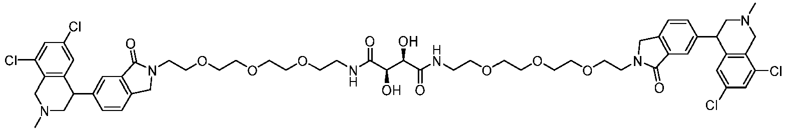 |
| | ¹H NMR (400 MHz, DMSO-*d*₆) δ 7.61 (t, *J =* 5.9 Hz, 2H), 7.51 - 7.46 (m, 8H), 6.85 (d, *J =* 2.1 Hz, 2H), 5.60 (d, *J =* 7.2 Hz, 2H), 4.51 (s, 4H), 4.40 (t, *J =* 5.4 Hz, 2H), 4.23 (d, *J =* 7.0 Hz, 2H), 3.72 (d, *J* = 16.2 Hz, 2H), 3.67 - 3.61 (m, 8H), 3.54 - 3.50 (m, 8H), 3.47-3.43 (m, 10H), 3.40 (t, *J =* 6.0 Hz, 4H), 3.28 - 3.18 (m, 4H), 2.86 - 2.82 (m, 2H), 2.68 - 2.64 (m, 2H), 2.36 (s, 6H). |
| | MS (ESI) m/z (M+H)⁺ = 1159.4. |
| | HPLC 100% purity; retention time was 7.243 min. |
| | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |
| **Embodiment J3** | 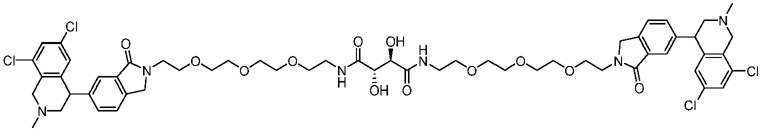 |
| | ¹H NMR (400 MHz, DMSO-*d*₆) δ 7.57 - 7.41 (m, 10H), 6.84 (d, *J =* 2.0 Hz, 2H), 5.86 (d, *J =* 5.3 Hz, 2H), 4.51 (s, 4H), 4.44 (s, 2H), 4.13 (d, *J* = 5.0 Hz, 2H), 3.79 (s, 2H), 3.69 - 3.61 (m, 8H), 3.54-3.42 (m, 18H), 3.39-3.37 (m, 4H), 3.28-3.11 (m, 4H), 2.94-2.76 m, 4H), 2.43 (s, 3H). |
| | MS (ESI) m/z (M+H)⁺ = 1159.4. |
| | HPLC 100% purity; retention time was 7.328 min. |
| | Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min. |

### Synthesis of embodiment J4

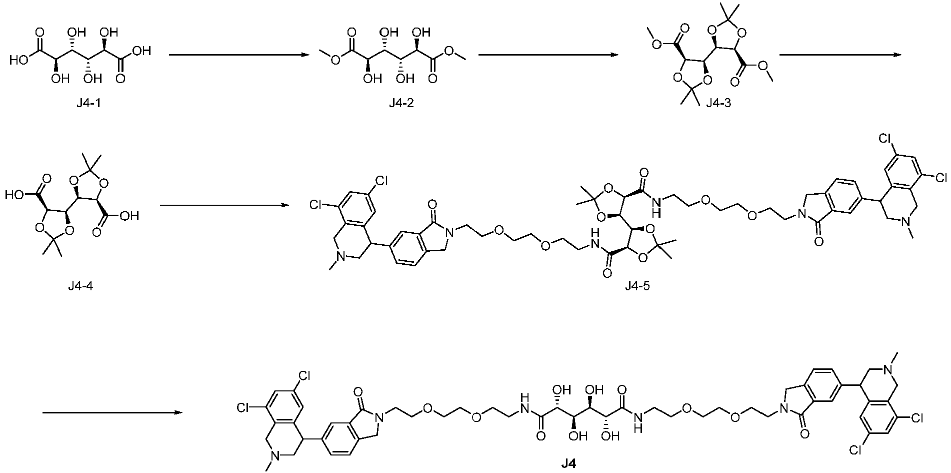

### Step 1: preparation of compound J4-2

Compound J4-1 (10 g, 47 mmol) was dissolved in methanol (200 mL), to which concentrated sulfuric acid (2.5 mL, 47 mmol) was added, after the addition, the system was stirred at 80°C for 3 days. The system was cooled to room temperature, continued to stir for 2 days, and a large amount of solids were precipitated. The reaction mixture was filtered, and the resulting solid was washed with methanol for three times, dried to obtain compound J4-2, which was directly used in the next step without further purification.

¹H NMR (400 MHz, DMSO-d6) δ 4.92 (d, J = 8.0 Hz, 2H), 4.85 - 4.78 (m, 2H), 4.31 (dd, J = 8.0, 1.1 Hz, 2H), 3.81 - 3.74 (m, 2H), 3.64 (s, 6H).

### Step 2: preparation of compound J4-3

Compound J4-2 (500 mg, 2.1 mmol) was dissolved in acetone (5 mL), to which 2,2-dimethoxypropane (2.6 mL, 47 mmol) and p-toluenesulfonic acid (79 mg, 0.415 mmol) were added, after the addition, the system was stirred at 65°C for 3 hours. The reaction mixture was cooled to room temperature and spin-dried to obtain solids, to which methanol was added, then the reaction mixture was filtered, and the resulting solid was washed with methanol for three times, dried to obtain compound J4-3, which was directly used in the next step without further purification.

¹H NMR (400 MHz, ) δ 4.57 - 4.53 (m, 2H), 4.44 - 4.39 (m, 2H), 3.71 (s, 6H), 1.38 (s, 6H), 1.33 (s, 6H).

### Step 3: preparation of compound J4-4

Compound J4-3 (500 mg, 1.57 mmol) was dissolved in a mixed solvent of tetrahydrofuran (4 mL) and water (1 mL), to which sodium hydroxide (100 mg, 2.5 mmol) was added, after the addition, the system was stirred at room temperature (25°C) for 16 hours. The pH of the reaction mixture was adjusted to 5 with 3M hydrochloric acid solution, then the reaction mixture was extracted with ethyl acetate, and the organic phase was dried, filtered and concentrated to obtain solids, to which methanol was added for slurry, then the reaction mixture was filtered, and the resulting solid was washed with methanol for three times, dried to obtain compound J4-4, which was directly used in the next step without further purification.

¹H NMR (400 MHz, DMSO-d6) δ 13.18 (s, 2H), 4.46 - 4.43 (m, 2H), 4.41 - 4.37 (m, 2H), 1.38 (s, 6H), 1.33 (s, 6H).

### Step 4: preparation of compound J4-5

Similar to the synthesis of embodiment J1, compound J4-5 was synthesized.

MS (ESI) m/z (M+H)⁺ = 1211.60

### Step 5: preparation of compound J4

Compound J4-5 (52 mg, 0.042 mmol) was dissolved in methanol (2 mL), to which concentrated hydrochloric acid (0.5 mL) was added, after the addition, the system was stirred at room temperature for 5 hours. The reaction mixture was concentrated, dissolved by adding methanol, and the pH of which was adjusted to 8 by adding sodium bicarbonate solution, then the reaction mixture was filtered, and the filtrate was purified by high performance preparative liquid chromatography (separation conditions: column: Agilent 10 Prep-C18 250×21.2mm; column temperature: 25°C; mobile phase: water (10mM/L ammonium bicarbonate)-acetonitrile; acetonitrile: 65%-95% 9min, flow rate: 30 mL/min) to obtain compound J4.

¹H NMR (400 MHz, DMSO-*d*₆) δ 7.47 (dd, *J* = 14.8, 7.0 Hz, 4H), 7.40 (q, *J* = 3.5, 2.9 Hz, 6H), 6.78 (d, J= 2.0 Hz, 2H), 5.20 (d, J= 7.0 Hz, 2H), 4.45 (s, 4H), 4.35 - 4.28 (m, 4H), 4.07 (d, *J* = 7.0 Hz, 2H), 3.73 (dd, *J* = 5.8, 2.5 Hz, 2H), 3.68 - 3.54 (m, 10H), 3.47 - 3.32 (m, 14H), 3.22 - 3.18 (m, 2H), 3.12 (dd, *J =* 13.3, 6.2 Hz, 2H), 2.77 (dd, *J* = 11.6, 5.1 Hz, 2H), 2.59 (dd, *J* = 11.6, 5.7 Hz, 2H), 2.29 (s, 6H).

MS (ESI) m/z (M+H)⁺ = 1131.40

HPLC 100% purity; retention time was 6.968 min

Separation conditions: column: Waters XBridge 4.6*100mm, 3.5um; column temperature: 40°C; mobile phase: water (10mM ammonium bicarbonate)-acetonitrile; acetonitrile: 5%-95% 7 min, 95% 8min; flow rate: 1.2 mL/min.

### Experimental Example 1 Cell-based NHE3 activity analysis under transient and persistent conditions

Opossum kidney (OK) cells were obtained from ATCC and cultured and expanded according to its instructions. OK monoclonal stable cell lines expressing the Rat NHE3 (Gene Bank registration number: M85300) protein and OK monoclonal stable cell lines expressing the Human NHE3 (Gene Bank registration number: NM_004174.1) protein were constructed by lentivirus infection.

The general procedure of the cell-based NHE3 activity experiments under transient conditions (transient inhibition) was based on an adaptation method of the pH-sensitive dye method originally reported by Tisen (Proc. Natl. Acad. Sci. USA) (1984) 81(23):7436-7440) to measure rat NHE3-mediated Na+-dependent reverse H+ transport, and was optimized according to the instructions "Measuring Intracellular pH With the FLIPR and FLIPR 384 Fluorometric Imaging Plate Reader Systems"published by Molecular Devices. The specific method was: Rat NHE3 OK monoclonal cells or Human NHE3 OK monoclonal cells were inoculated into 384-well plates and grew overnight. The medium was aspirated from the wells and the cells were washed twice with FLIPR buffer (Hank's BSS 1X, 20 mM HEPES, 1% BSA) followed by incubation with FLIPR buffer (Hank's BSS 1X, 20 mM HEPES, 1% BSA) containing 5 mM BCECF-AM (Invitrogen) for 45 min at room temperature. Subsequently, 5 mL of 200 mM NH4Cl-HEPES buffer (Hank's BSS 1X, 20 mM HEPES, 200 mM NH₄Cl) was added and the cells were incubated for 15-20 minutes at room temperature. Cells were washed twice with 20mM NH₄Cl-HEPES buffer (Hank's BSS 1X, 20mM HEPES, 20mM NH₄Cl). NHE3-mediated pH sensitization in cells was normalized by addition of containing 0.4 mM amiloride (EIPA) and 0-30 mM test compound or Na-HEPES buffer (100 mM NaCl, 50 mM HEPES, 10 mM glucose, 5 mM KCl, 2 mM CaCl₂, 1 mM MgCl₂, pH 7.4) and monitoring of BCECF-AM fluorescence at λem 535 nm excitated by λex 440 nm and λex 490 nm respectively. Ratio = fluorescence value (λex 490nm)/fluorescence value (λex 440nm) was set, the average value of the Ratio difference between the end time and the start time between the duplicate wells was calculated, the average value and the concentration value of the compound were drawn, and Graphpad Prism software was used to estimate IC50 value. The results were shown in Table 13:

The general procedure of the cell-based NHE3 activity assay under persistent conditions (persistent inhibition) was: Rat NHE3 OK monoclonal cells or Human NHE3 OK monoclonal cells were inoculated into 384-well plates and grew overnight. The medium was aspirated from the wells and the cells were washed twice with FLIPR buffer (Hank's BSS 1X, 20 mM HEPES, 1% BSA) followed by the addition of Na-HEPES buffer containing 0-30 mM test compound (100 mM NaCl, 50 mM HEPES, 10 mM glucose, 5 mM KCl, 2 mM CaC₁₂, 1 mM MgCl₂, pH 7.4) and incubated for 60 minutes at room temperature. The compounds were aspirated from the wells and incubated with FLIPR buffer (Hank's BSS 1X, 20 mM HEPES, 1% BSA) containing 5 mM BCECF-AM (Invitrogen) for 45 minutes at room temperature. Subsequently, 5 mL of 200 mM NH₄Cl-HEPES buffer (Hank's BSS 1X, 20 mM HEPES, 200 mM NH₄Cl) was added and incubated for 15-20 minutes at room temperature. The cells were washed twice with 20mM NH₄Cl-HEPES buffer (Hank's BSS 1X, 20 mM HEPES, 20 mM NH₄Cl). NHE3-mediated pH sensitization in cells was normalized by addition of Na-HEPES buffer (100 mM NaCl, 50 mM HEPES, 10 mM glucose, 5 mM KCl, 2 mM CaCl₂, 1 mM MgCl₂, pH 7.4) containing 0.4 mM amiloride (EIPA, a selective antagonist of NHE-1 activity that does not inhibit NHE3) and monitoring of BCECF fluorescence at λem 535 nm excitated by λex 440 nm and λex 490 nm respectively. Ratio = fluorescence value (λex 490nm)/fluorescence value (λex 440nm) was set, the average value of the Ratio difference between the end time and the start time between the duplicate wells was calculated, the average value and the concentration value of the compound were drawn, and the dose-response-inhibition (four parameters) equation was used to estimate EC50 value by GraphpadPrism. The results were shown in Tables 13 and Table 14:

**Table 13 NHE3 inhibitory activity of intermediates based on opossum kidney cells under transient conditions**

| Intermediates number | Cell-based NHE3 inhibition IC₅₀ (uM) under transient conditions |
|---|---|
| A1-13 | 0.60 |
| A2-13 | 1.63 |
| B1-6 | 0.53 |
| B2-6 | 0.17 |
| B3-6 | 0.62 |
| B4-6 | 0.79 |
| B5-6 | 1.69 |
| C1-11 | 1.24 |
| D1-7 | 1.44 |
| E1-9 | 3.17 |

**Table 14 NHE3 inhibitory activity of compounds based on opossum kidney cells under transient and persistent conditions**

| Embodiment compound No. | Cell-based NHE3 inhibition IC₅₀ (uM) under transient conditions | Cell-based NHE3 inhibition IC₅₀ (uM) under persistent conditions |
|---|---|---|
| Tenapanor | 0.74 | 0.56 |
| A1 | 0.43 | 0.50 |
| A2 | 3.14 | ND |
| B1 | 0.52 | 0.02 |
| B2 | 2.05 | ND |
| B3 | 0.77 | 0.41 |
| B4 | 0.93 | 0.45 |
| B5 | 0.23 | 0.57 |
| B6 | 1.05 | 0.04 |
| C1 | 0.22 | 0.59 |
| D1-P1 | 0.28 | 0.02 |
| D1-P2 | 2.33 | 3.61 |
| D2-P1 hydrochloride salt | 0.16 | 0.02 |
| D2-P2 hydrochloride salt | 0.27 | 0.12 |
| E1 | 0.06 | 0.05 |
| F1 | 0.91 | 0.64 |
| F2 | 0.62 | 0.08 |
| G1 | 0.93 | 0.10 |
| H1 | 0.91 | 0.04 |
| H2 | 0.48 | 0.06 |
| I1 | 0.35 | 0.09 |
| I2 | 0.17 | 0.07 |
| I3 | 0.19 | 0.13 |
| I4 | 0.20 | 0.31 |
| J1 | 0.43 | 0.03 |
| J2 | 0.32 | 0.05 |
| J3 | 0.26 | 0.08 |
| J4 | 0.25 | 0.05 |

### Experimental Example 2 Inhibition of phosphate and sodium absorption in the intestine of rats by a single dose

Compound D1-P1-S was evaluated by measuring urinary phosphorus, urinary sodium concentration and fecal form.

Six-week-old Sprague-Dawley rats were purchased from Beijing Vital River Laboratory Animal Technology Co., Ltd. Two animals per cage were placed in SPF class animal rooms for acclimatization for about a week. Throughout the study period, animals were fed feed and water freely and the light cycle was 12h light/12h dark. Animal groups: vehicle group, n=5; tenapanor 0.3mg/kg group, n=5; D1-P1-S 0.03mg/kg group, n=5; D1-P1-S 0.1mg/kg, n=5; D1-P1-S 0.3mg/kg, n=5; D1-P1-S1.0mg/kg, n=5.

On the day of the experiment, after 8 hours of fasting, the animals were administered the test compound or solvent (0.5% Tween 80 + 99.5% distilled water) by gavage. The animals were then transferred into metabolic cages for single-cage feeding, and the feed was restored. After 16 hours of administration, urine samples were collected and food consumption was recorded, and the fecal form in the collection funnel was evaluated by two independent observations. Standard for fecal form score: 1, normal pellets; 2, slightly soft feces (pellets adhered to the side wall of the collector due to moisture); 3, soft feces (loss the normal shape of the pellets); 4, loose and amorphous (completely loss shape, with blot pattern); 5, diarrhea (watery feces). Fecal form score (FFS) in rats was determined by averaging two independent observation scores for all rats within the same group (n=5); the vehicle group mean was 1.

The urine was centrifuged at 3220g for 5 minutes at 4°C, and the urinary phosphorus concentration (phosphomolybdic acid UV endpoint colorimetric method) and urinary sodium concentration (ion selective electrode method) were measured.

Results were indicated as mean ± standard error (Means ± SEM). Urinary phosphorus excretion amount (or urinary sodium excretion amount) in rats was standardized corrected against respective dietary phosphorus (or sodium) intake, and the formula was: standardized correction value of urinary phosphorus excretion amount (indicated as nP) = urinary phosphorus excretion amount ÷ dietary phosphorus intake amount; standardized correction value of urinary sodium excretion amount (indicated as nNa) = urinary sodium excretion amount ÷ dietary sodium intake amount; fecal form score was analyzed by monofactorial variance, and verified by non-parametric test. *, *p*<0.05; **, *p*<0.01; ***, *p*<0.001; ****, *p*<0.0001.

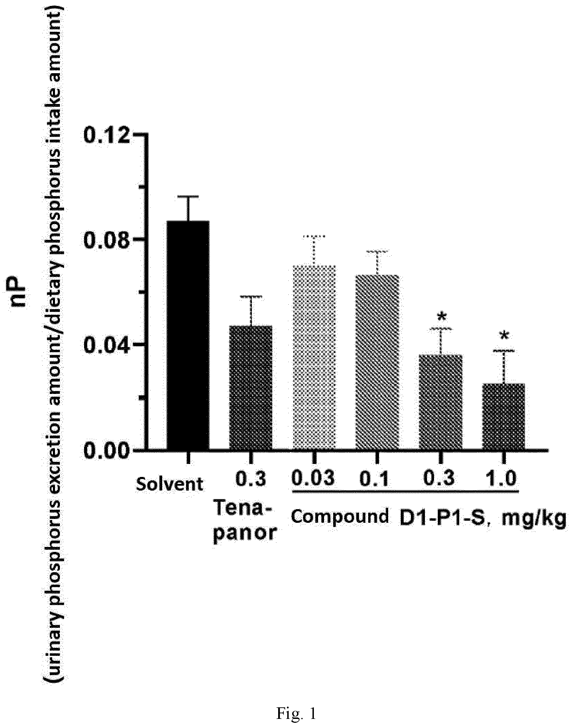

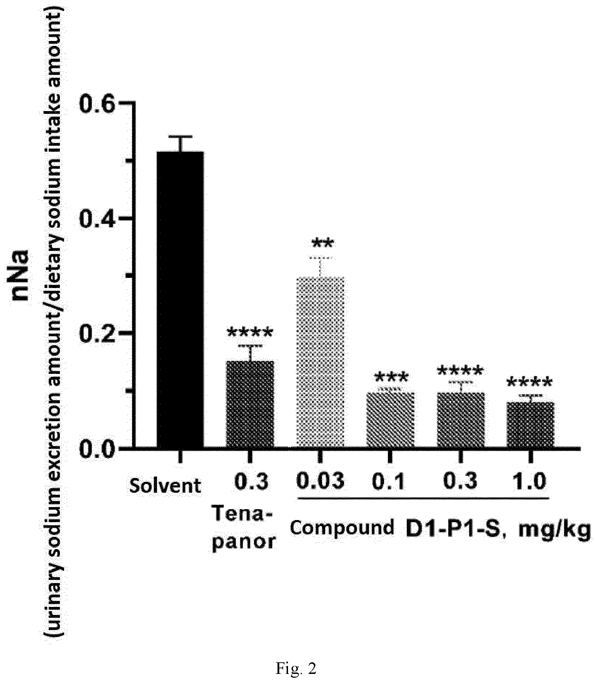

Figures 1 to 3 showed the effects of compound D1-P1-S on urinary phosphorus, urinary sodium excretion and fecal form in normal rats after a single administration. These results showed that compound D1-P1-S had dose-effect relationship in reducing urinary phosphorus excretion amount, and could significantly reduce urinary phosphorus excretion amount at doses of 0.3 mg/kg and 1.0 mg/kg. The effect of D1-P1-S was better than Tenapanor in reducing urinary phosphorus excretion amount at the same dose (0.3mg/kg). Compound D1-P1-S could significantly reduce urinary sodium excretion amount. The water content in rat feces has the tendency of increasing with the increase of the dose of compound D1-P1-S.

### Experimental Example 3 Effects of multiple administrations on serum phosphorus concentration in rats

Compound D1-P1-S was evaluated by measuring serum phosphorus concentration, urinary phosphorus, urinary sodium concentration and fecal form in rats.

Six-week-old Sprague-Dawley rats were purchased from Beijing Vital River Laboratory Animal Technology Co., Ltd. Two animals per cage were placed in SPF class animal rooms for acclimatization for about a week. Throughout the study period, animals were fed feed and water freely and the light cycle was 12h light/12h dark. Animal groups: vehicle group, n=5; Tenapanor 0.05mg/kg, n=5; D1-P1-S 0.01mg/kg, n=5; D1-P1-S 0.05mg/kg, n=5; D1-P1-S 0.5mg/kg, n=5; D1-P1-S 1.0mg/kg, n=5.

After the start of the experiment, the feeding rhythm of the rats was adjusted to diurnal feeding by fasting overnight for 16 hours and recovering feed for 8 hours during the day. After the administration started, the test compound or the solvent (0.5% Tween 80+99.5% distilled water) was gavaged twice a day during the feeding period, with an interval of 4 hours, for 14 consecutive days. Animal body weight and food consumption were measured every day, serum phosphorus concentration (including baseline value before administration), 24-hour urinary phosphorus excretion amount, and 24-hour urinary sodium excretion amount were measured 1-2 times a week, and feces form in the collection funnel was scored (through two independent observations to assess fecal form). Standard for fecal form score: 1, normal pellets; 2, slightly soft feces (pellets adhered to the side wall of the collector due to moisture); 3, soft feces (loss the normal shape of the pellets); 4, loose and amorphous (completely loss shape, with blot pattern); 5, diarrhea (watery feces). Fecal form score (FFS) in rats was determined by averaging two independent observation scores for all rats within the same group (n=5); the vehicle group mean was 1.

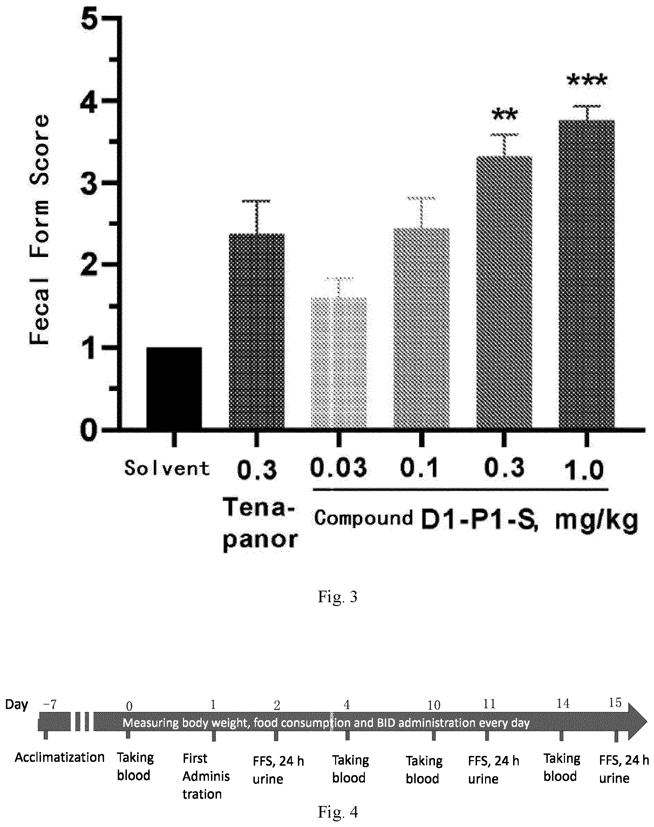

The experimental procedure was shown in Fig. 4 below.

After standing at room temperature for 2 hours, the blood was centrifuged at 4500g for 10 minutes at 4°C; and the urine was centrifuged at 3220g for 5 minutes at 4°C, and the serum phosphorus concentration and urinary phosphorus concentration (phosphomolybdic acid UV endpoint colorimetric method), urinary sodium concentration (indirect ion electrode method) were measured.

Results were indicated as mean±standard error (Means±SEM), n=5 animals/group. Urinary phosphorus excretion amount (or urinary sodium excretion amount) in rats was standardized corrected against respective dietary phosphorus (or sodium) intake, and the formula was: standardized correction value of urinary phosphorus excretion amount (indicated as nP) = urinary phosphorus excretion amount ÷ dietary phosphorus intake amount; standardized correction value of urinary sodium excretion amount (indicated as nNa) = urinary sodium excretion amount ÷ dietary sodium intake amount; fecal form score was analyzed by two-way variance, and verified by non-parametric test. *, p<0.05; **, p<0.01; ***, p<0.001; ****, p<0.0001.

### Experimental results

### Serum phosphorus concentration

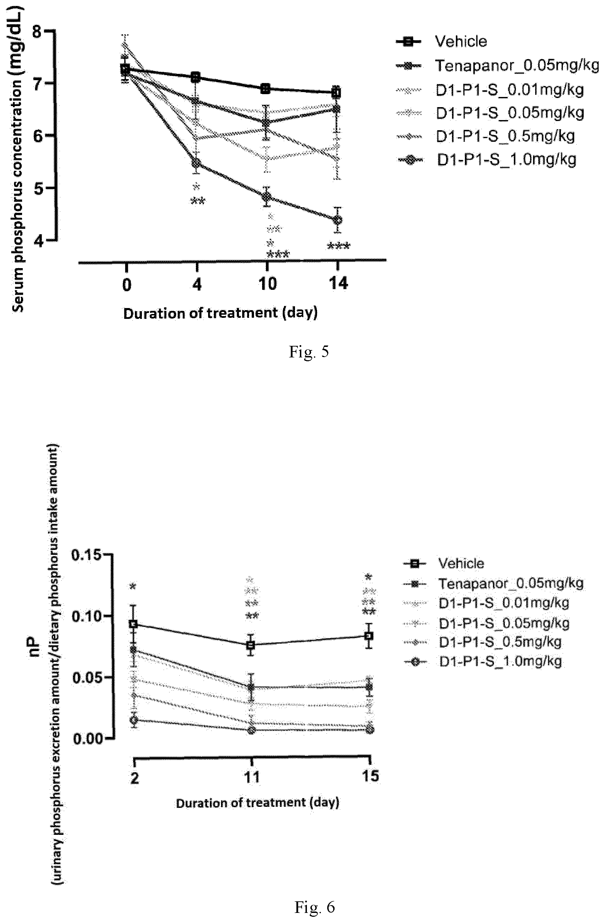

Compared with the serum phosphorus concentration of rats in the solvent control group, compound D1-P1-S significantly decreased the serum phosphorus concentration of rats after treatment at doses of 0.5mg/kg or 1.0mg/kg for 4 days; after 10 days of treatment at the dose of 0.01mg/kg, 0.05mg/kg, 0.5mg/kg, 1.0mg/kg doses, the serum phosphorus concentration of rats decreased significantly; except for the highest dose group of 1.0mg/kg, the serum phosphorus of the other groups tended to be stable. The effect of D1-P1-S at the dose of 0.01mg/kg to reduce serum phosphorus concentration was similar to that of Tenapanor at the dose of 0.05mg/kg. See Fig. 5 below for details.

### Urinary phosphorus excretion

The 24h nP of the rats in each group was compared with that of the rats in the solvent group; after 1 day of treatment with compound D1-P1-S at the doses of 0.01 mg/kg, 0.05 mg/kg, 0.5 mg/kg and 1.0 mg/kg, the nP of rats tended to decrease, the higher the dose, the lower the nP, and nP significantly decreased at the dose of 1.0 mg/kg. After 10 days of treatment, at the doses of 0.01mg/kg, 0.05mg/kg, 0.5mg/kg and 1.0mg/kg, the nP all decreased significantly; and the nP of rats tended to be stable after 15 days, but the nP of rats in low dose group (0.01mg /kg) tended to increase. The effect of D1-P1-S at the dose of 0.01mg/kg to reduce nP in rats was similar to that of Tenapanor at the dose of 0.05mg/kg. See Fig. 6 below for details.

### Urinary sodium excretion

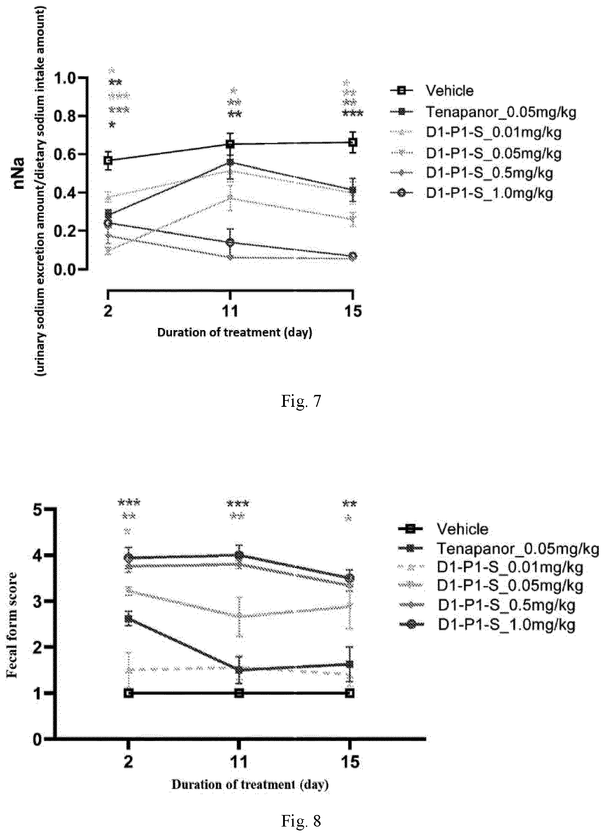

The 24h nNa of the rats in each group was compared with that of the rats in the solvent group; after 1 day of treatment with compound D1-P1-S at the doses of 0.01 mg/kg, 0.05 mg/kg, 0.5 mg/kg and 1.0 mg/kg, the nNa of rats decreased significantly. However, after 10 days of treatment, the nNa in rats showed an increasing trend at the doses of 0.01 mg/kg and 0.05 mg/kg. See Fig. 7 below for details.

### Fecal form score

Compared with the rats in the solvent control group, the fecal form score of rats was significantly increased after 1 day of treatment with compound D1-P1-S at the doses of 0.05mg/kg, 0.5mg/kg and 1.0mg/kg. Rats with high doses of 0.5mg/kg and 1.0mg/kg had more severe loose feces, but as the administration continued, the number of times of loose feces observed decreased. After treatment at a low dose of 0.01 mg/kg, no rats had loose feces. See Fig. 8 below details.

## Claims

1. A compound as shown in formula (I), a stereoisomer thereof and a pharmaceutically acceptable salt thereof, 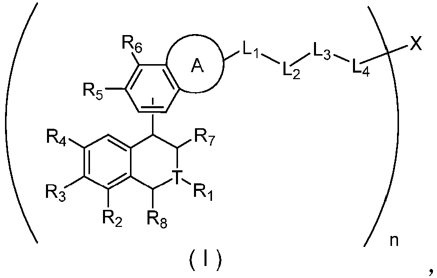 wherein,
R₁ is selected from CH₃, 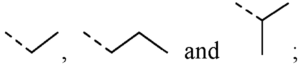
R₂, R₃, R₄ are each independently selected from H, F, Cl, Br, OH, NH₂, CN, CH₃, 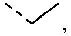 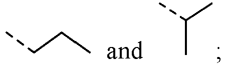
R₇, R₈ are each independently selected from H;
T is selected from N and CH;
the structural moiety 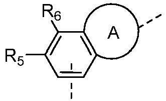 is selected from 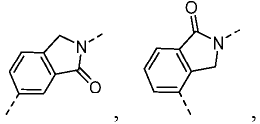 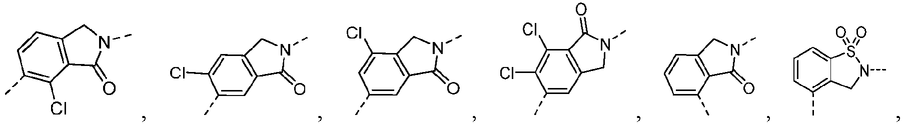 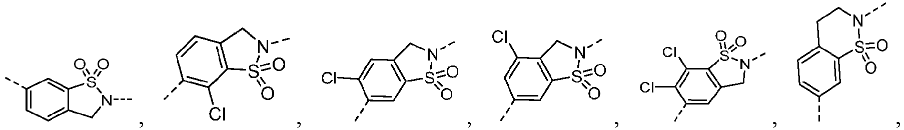 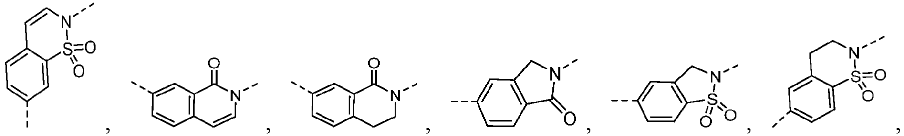 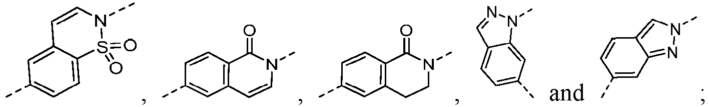
n is selected from 2 and 3;
the structural unit 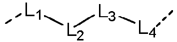 is selected from 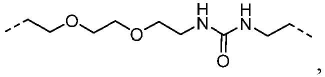 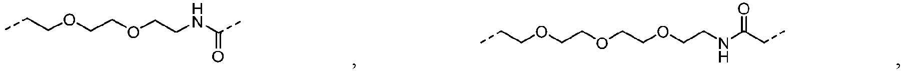 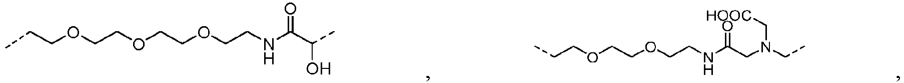 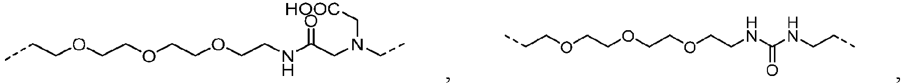 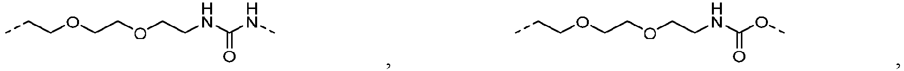 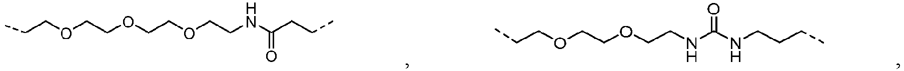 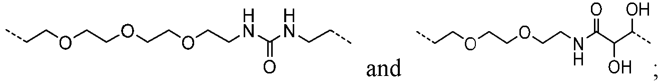
X is selected from a single bond, N, NH, O,

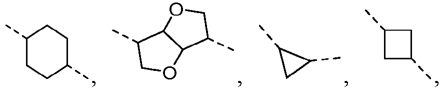

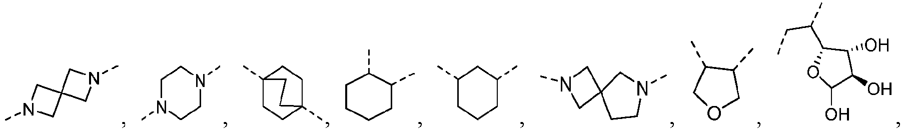

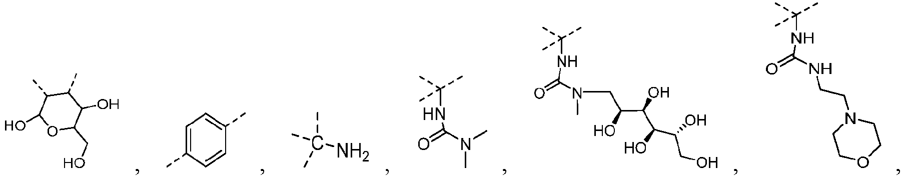

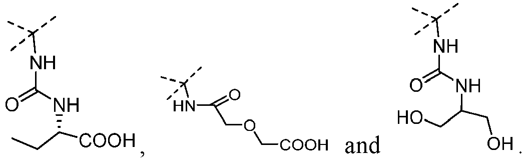

2. The compound, the stereoisomer thereof and the pharmaceutically acceptable salt thereof according to claim 1, wherein, the compound is selected from: 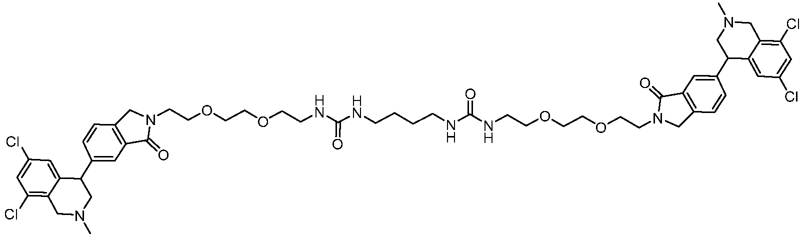 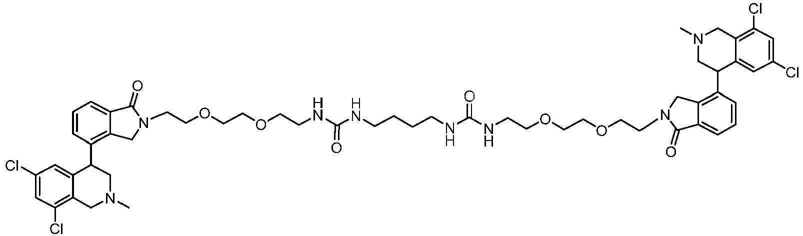 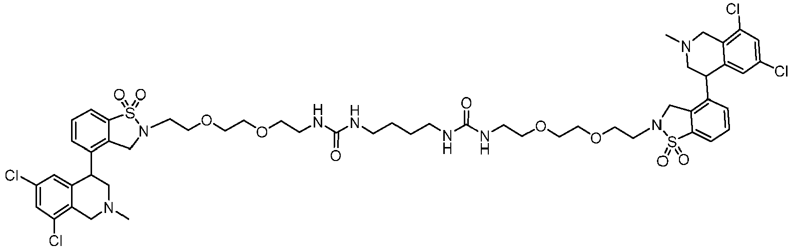 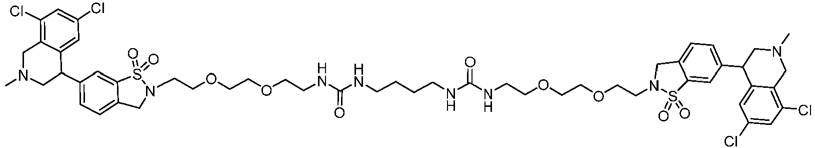 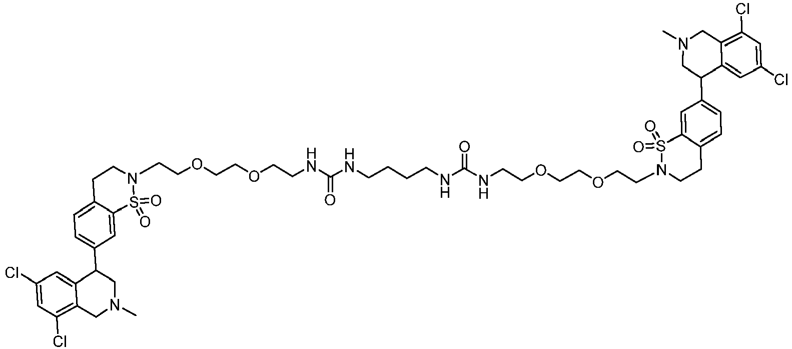 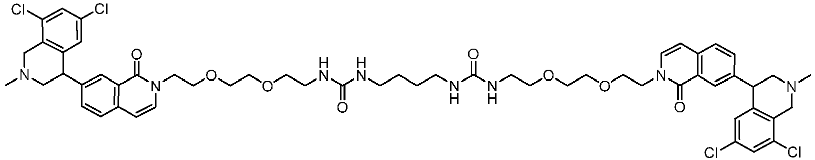 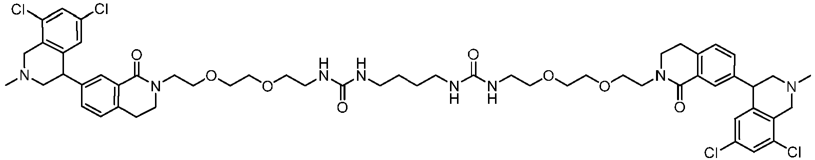 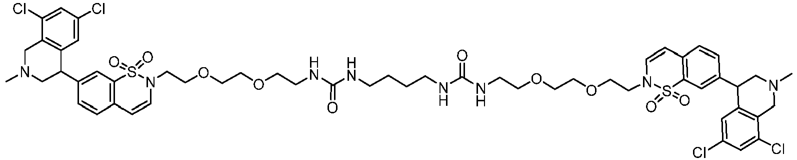 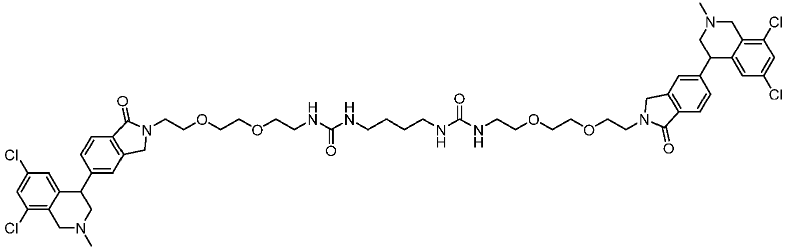 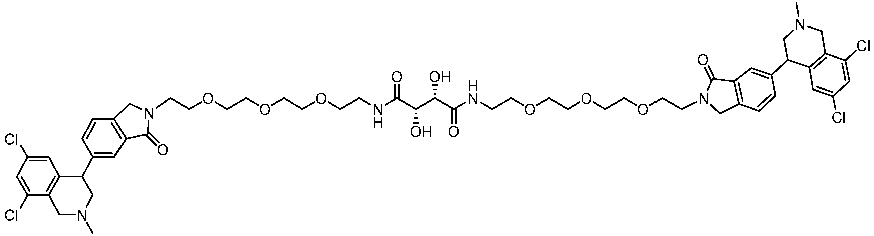 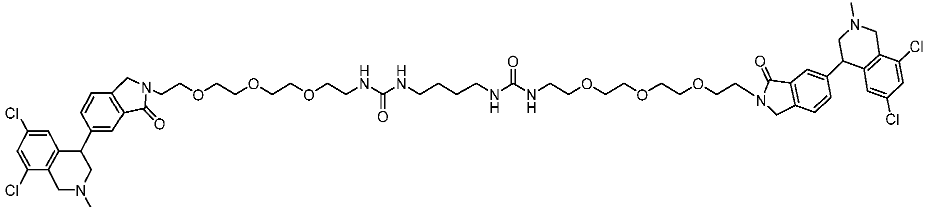 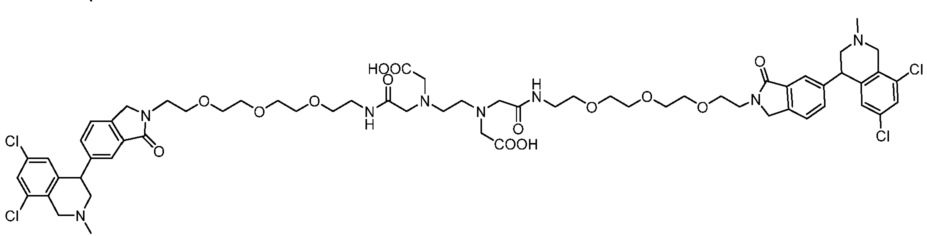 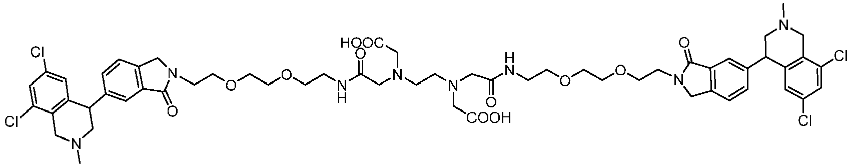 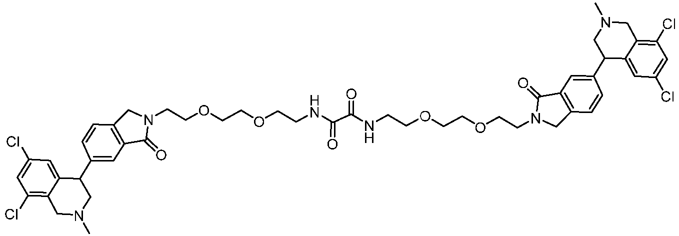 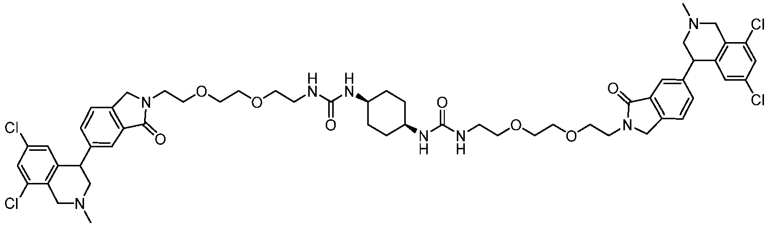 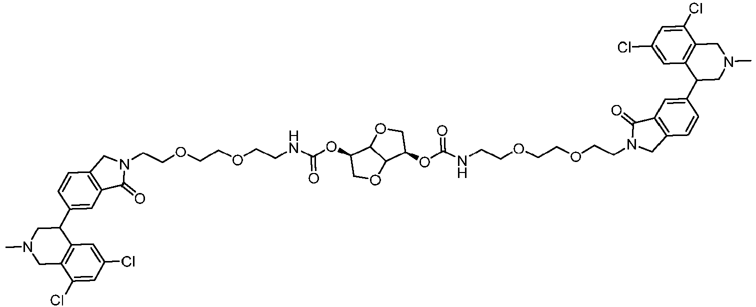 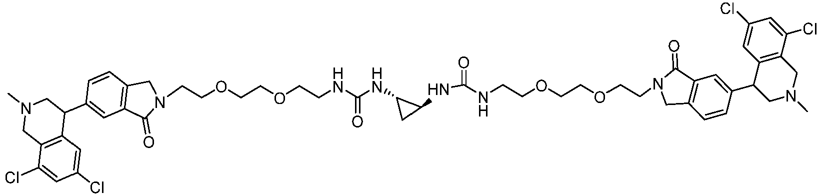 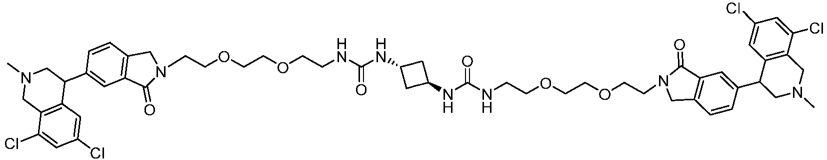 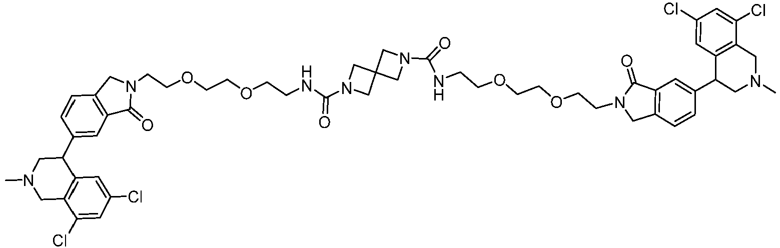 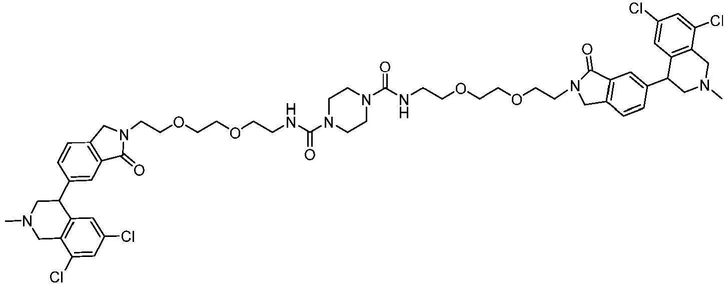 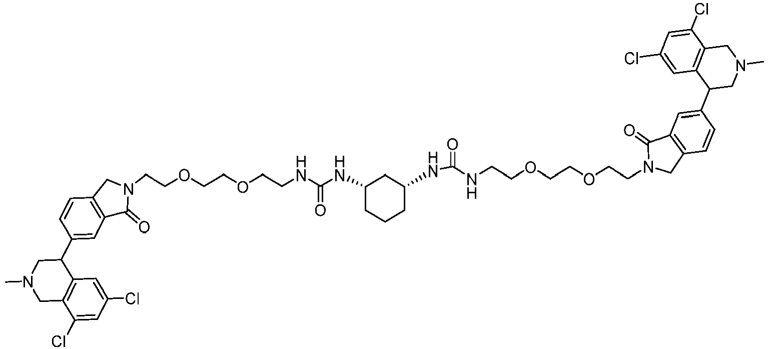 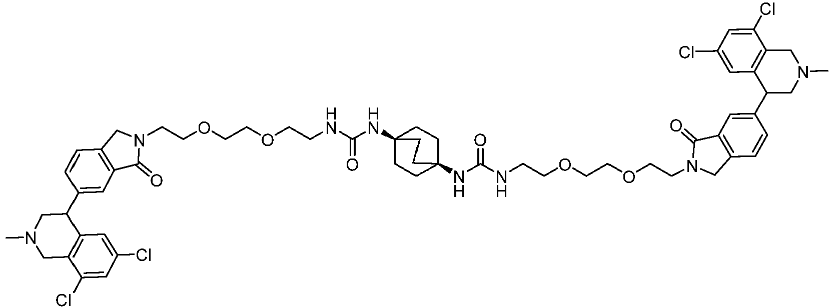 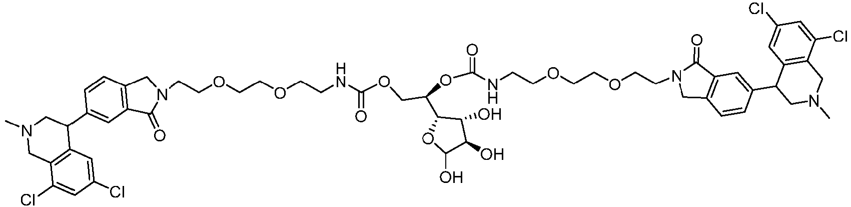 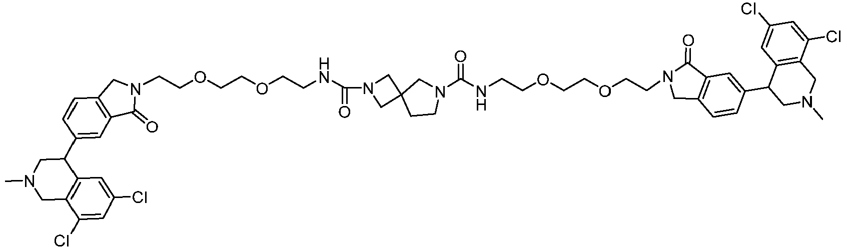 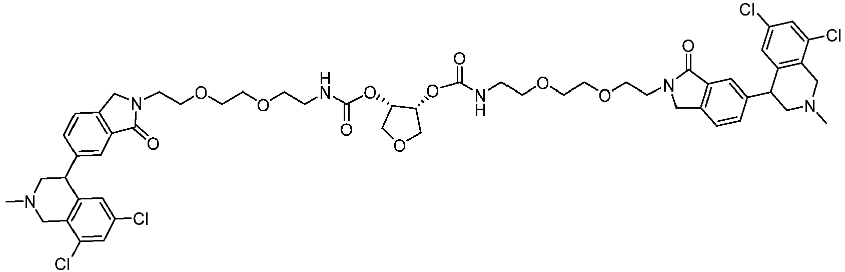 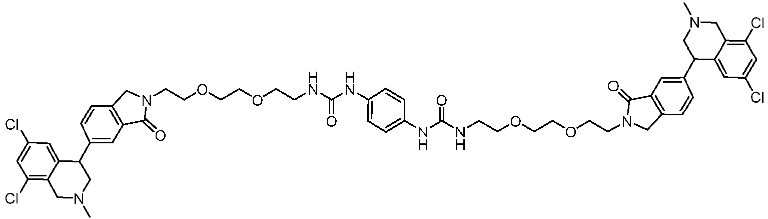 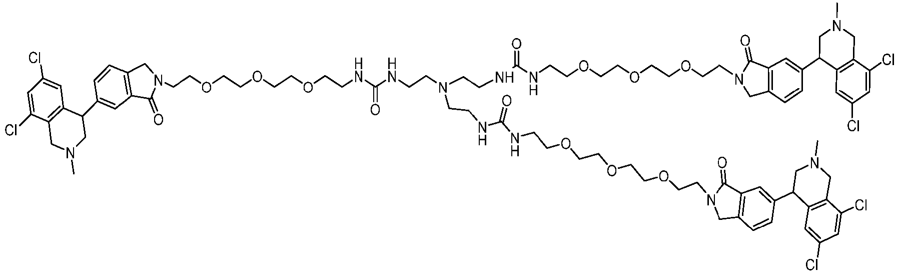 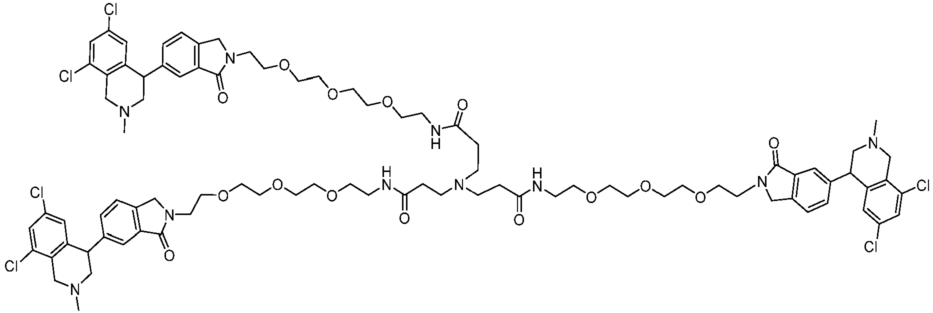 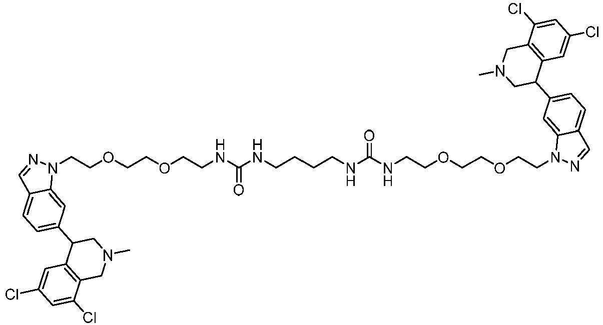 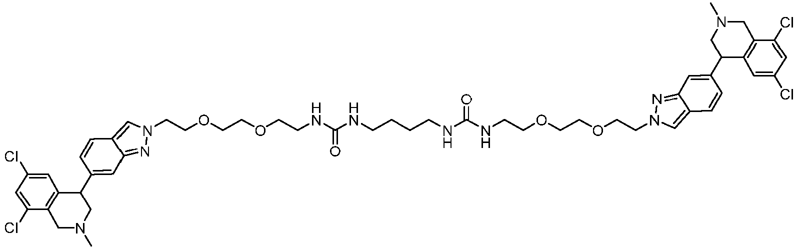 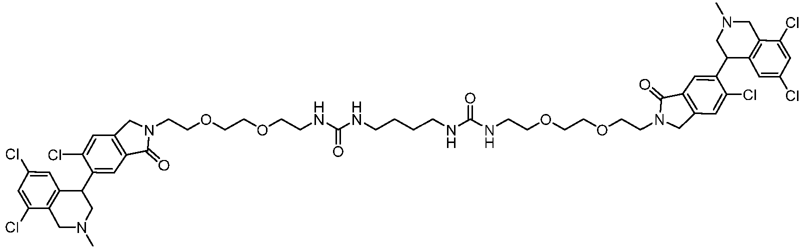 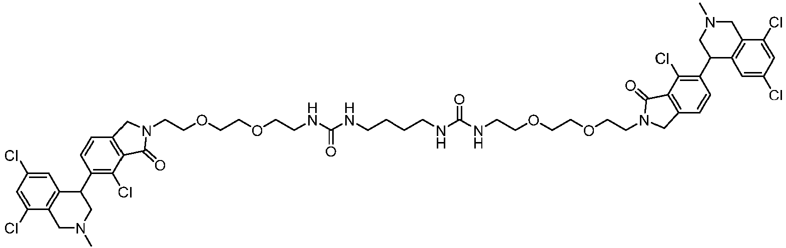 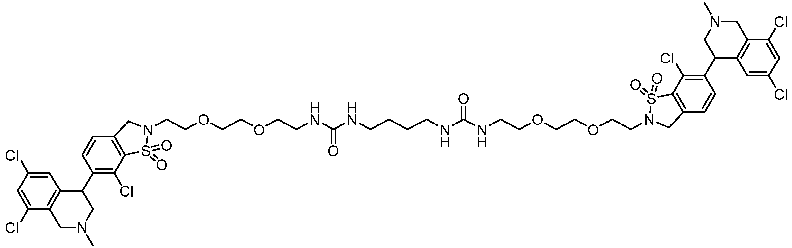 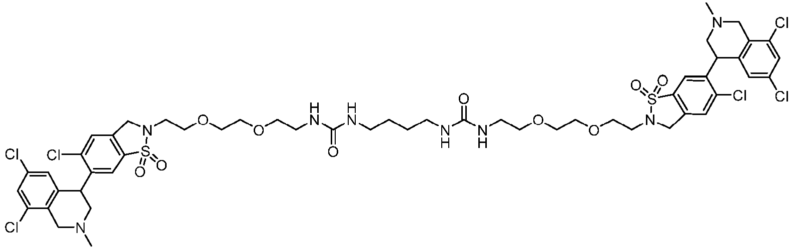 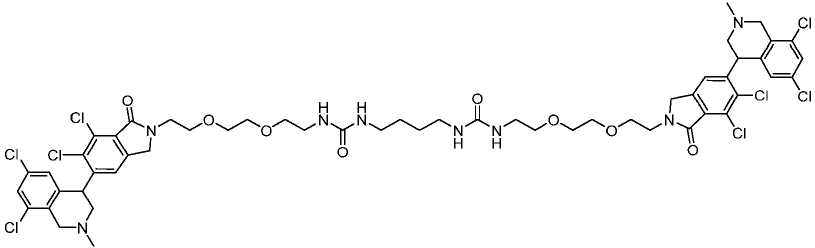 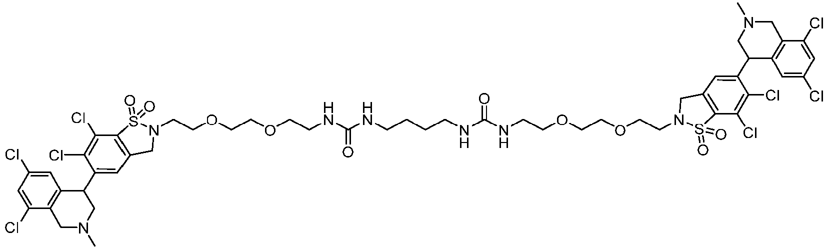 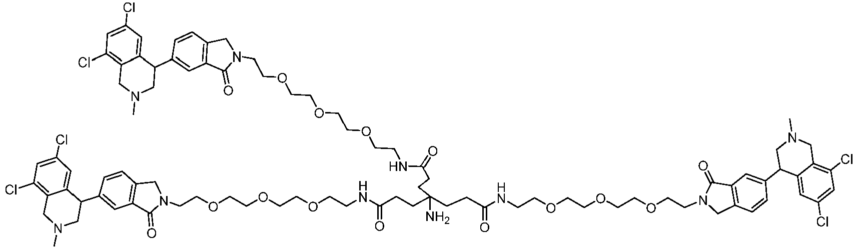 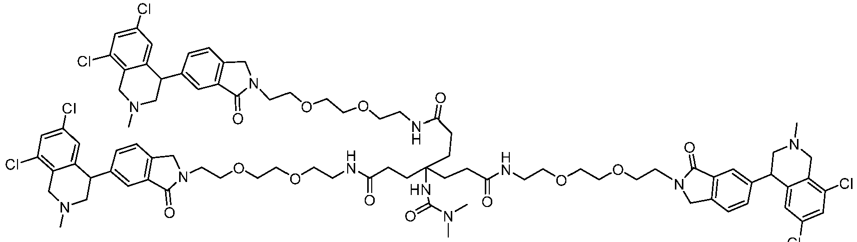 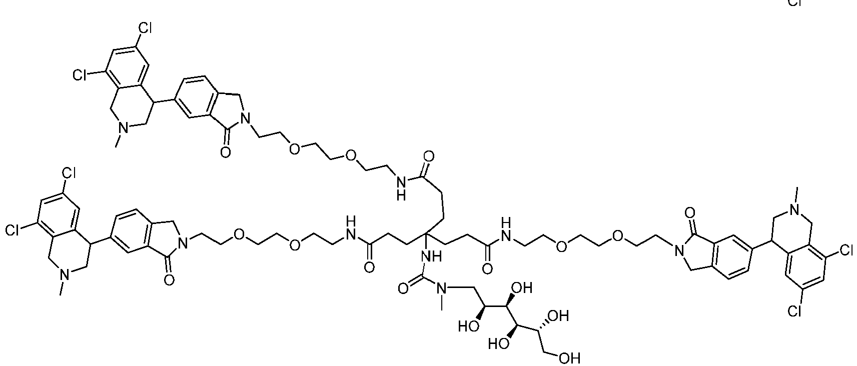 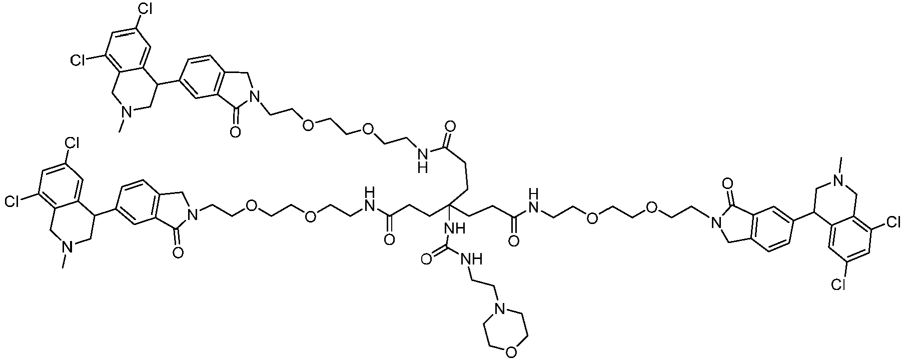 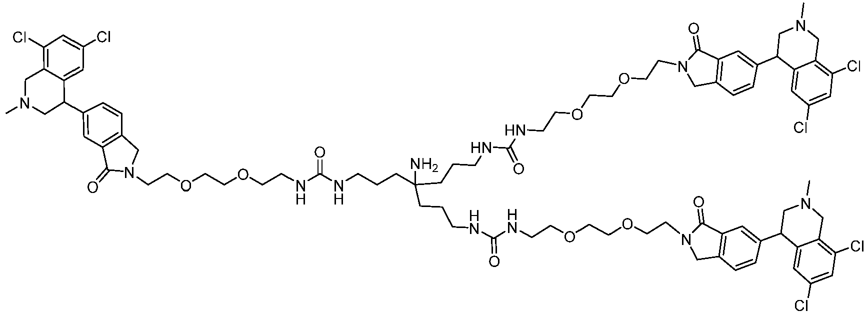 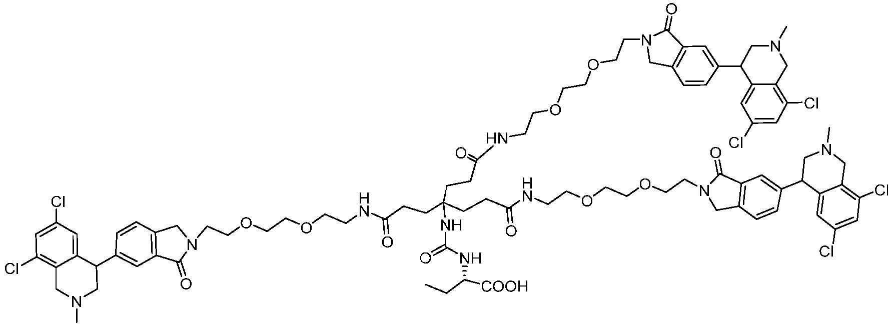 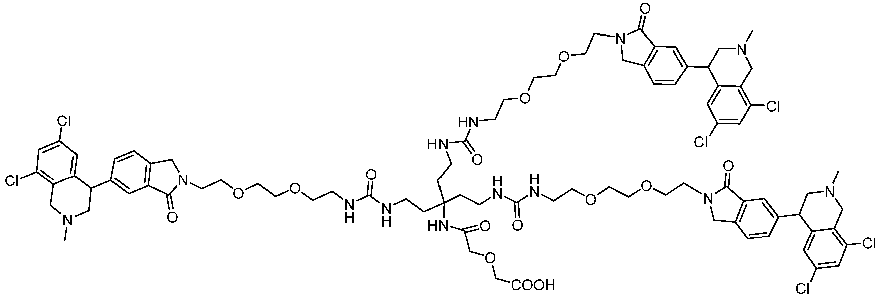 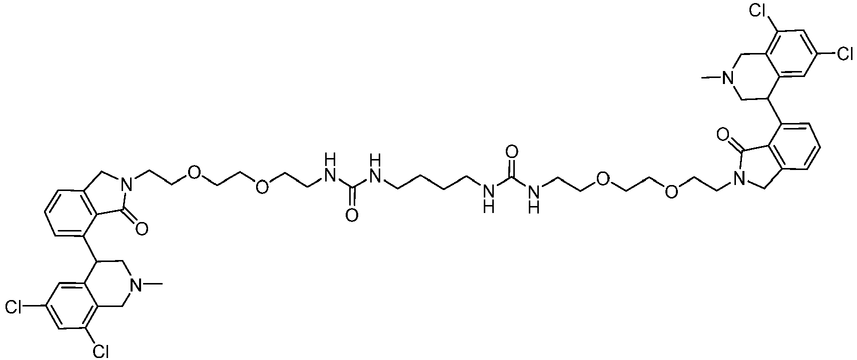 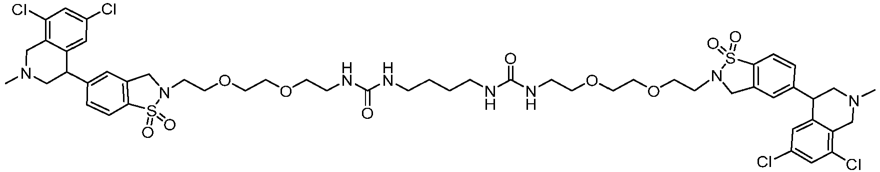 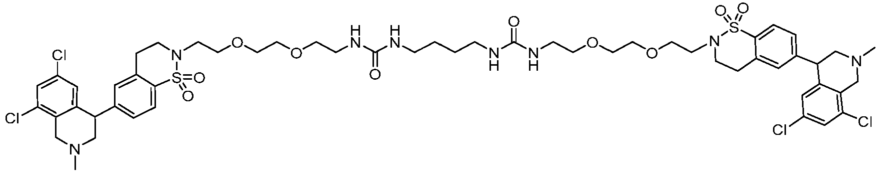 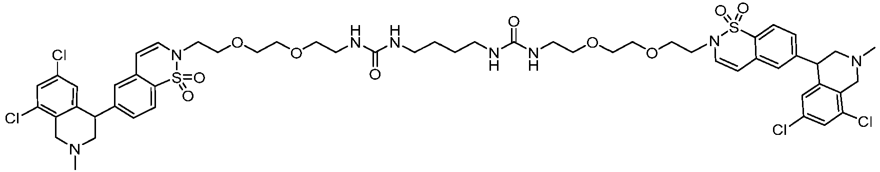 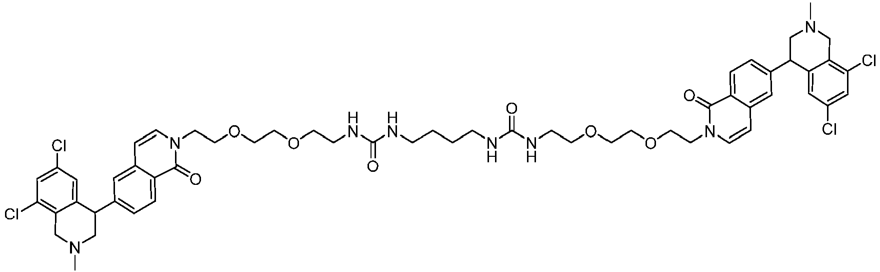 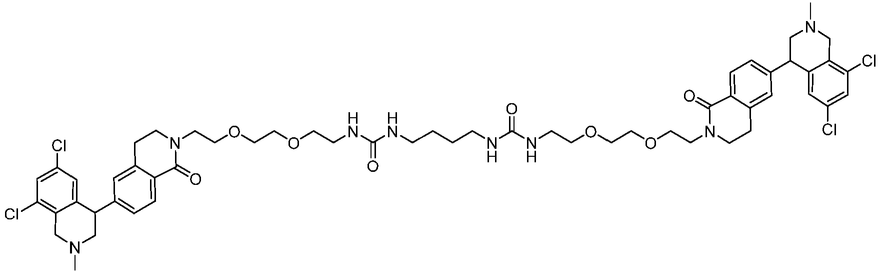 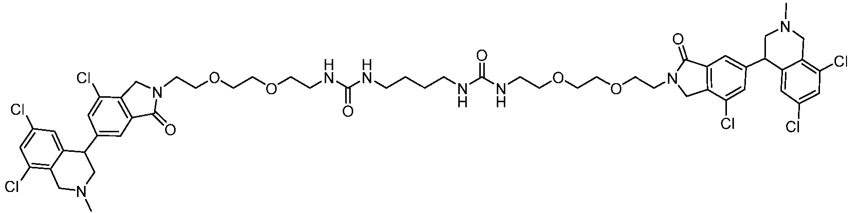 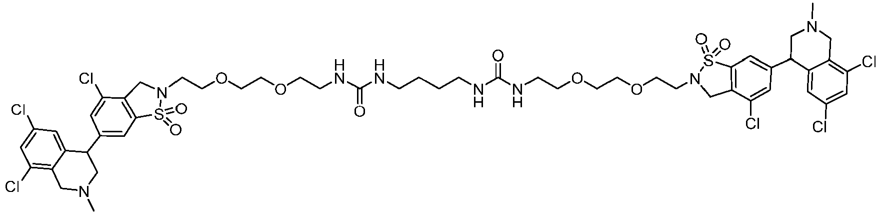 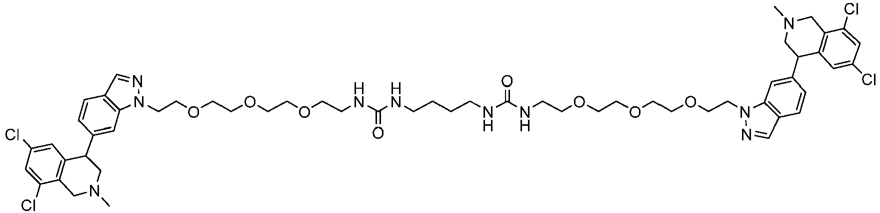 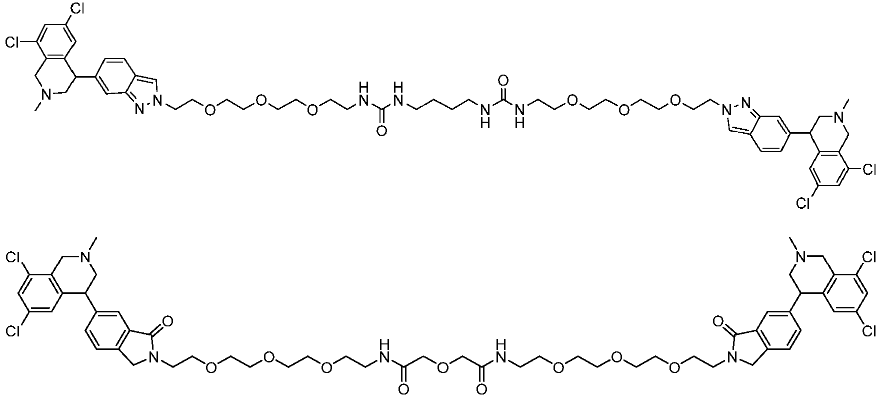 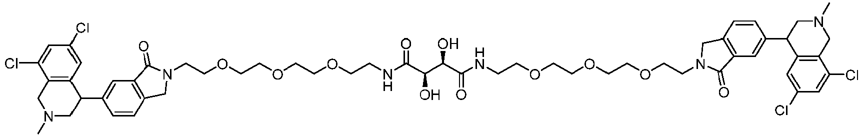 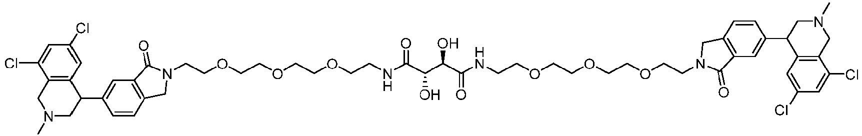 and

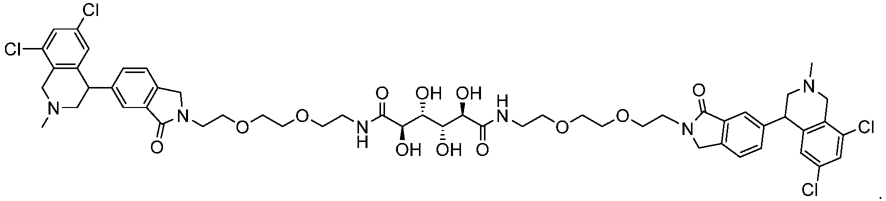

3. The compound, the stereoisomer thereof and the pharmaceutically acceptable salt thereof according to any one of claim 1-2 for use in treating irritable bowel syndrome, heart failure, chronic kidney disease, end-stage renal disease or liver disease.

## Patentansprüche

1. Verbindung, wie in Formel (I) gezeigt, ein Stereoisomer davon und ein pharmazeutisch unbedenkliches Salz davon, 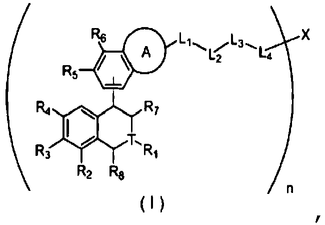 wobei,
R₁ ausgewählt ist aus CH₃, 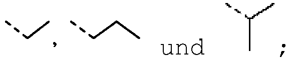
R₂, R₃, R₄ jeweils unabhängig voneinander ausgewählt sind aus H,
F, Cl, Br, OH, NH₂, CN, CH₃, 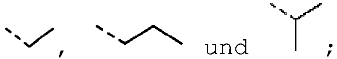
R₇, R₈ jeweils unabhängig voneinander ausgewählt sind aus H;
T ausgewählt ist aus N und CH;
die Strukturgruppierung 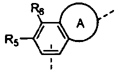 ausgewählt ist aus 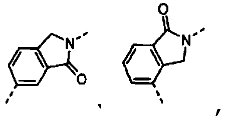 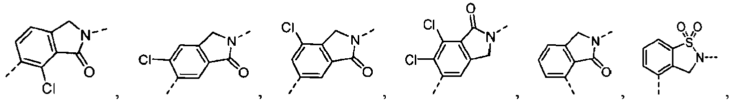 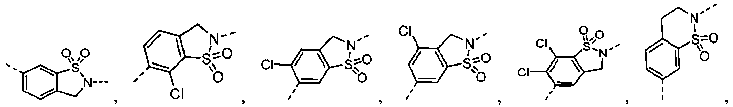 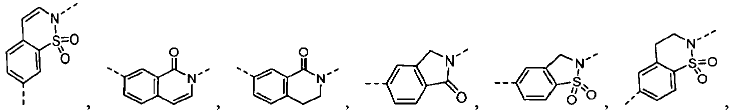 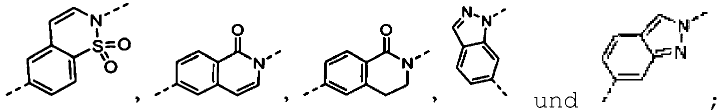
n ausgewählt ist aus 2 und 3;
die Struktureinheit 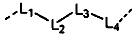 ausgewählt ist aus 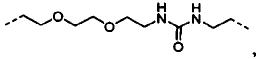 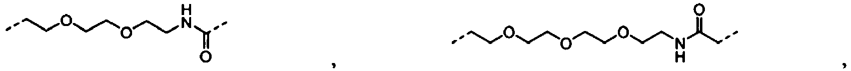 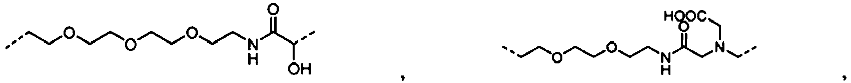 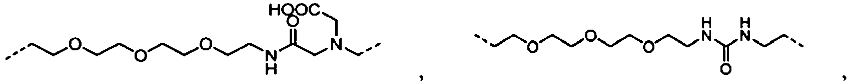 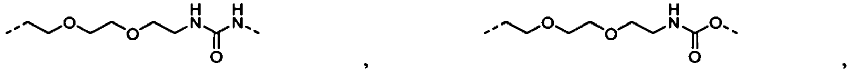 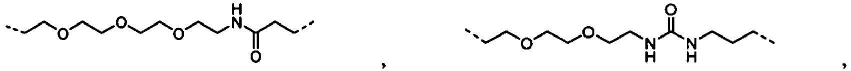 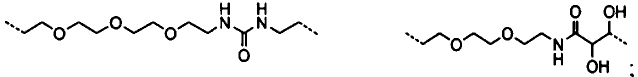 und
X ausgewählt ist aus einer Einfachbindung, N, NH, O,

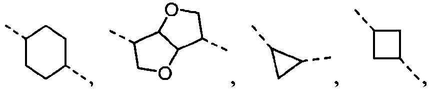

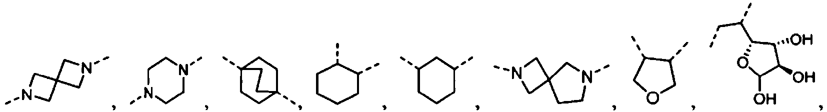

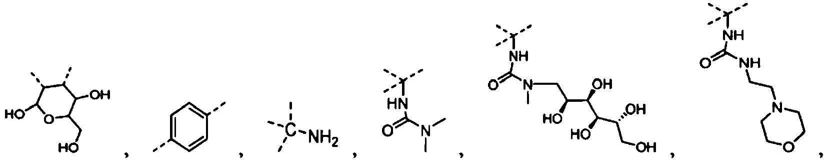

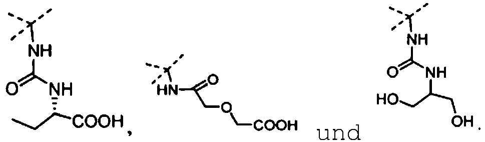

2. Verbindung, das Stereoisomer davon und das pharmazeutisch unbedenkliche Salz davon nach Anspruch 1, wobei die Verbindung ausgewählt ist aus: 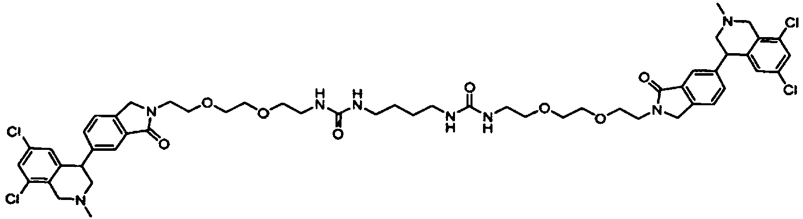 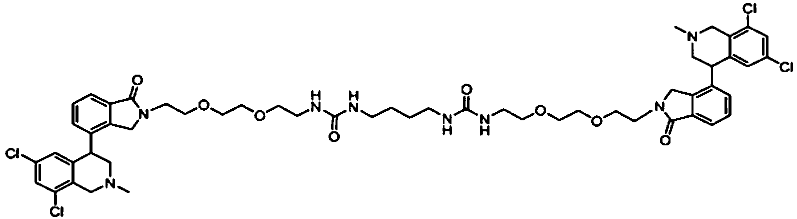 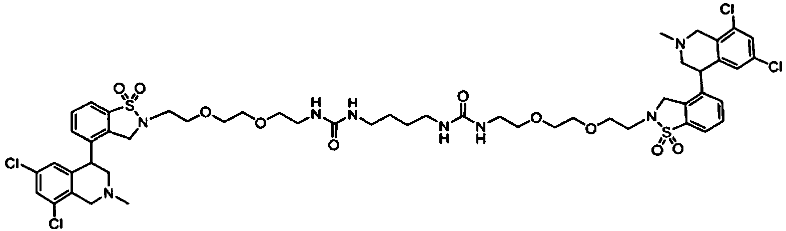 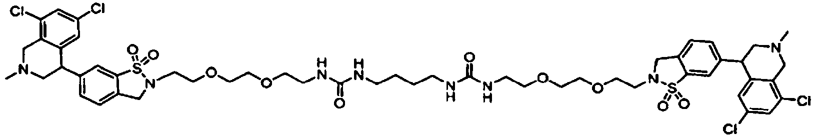 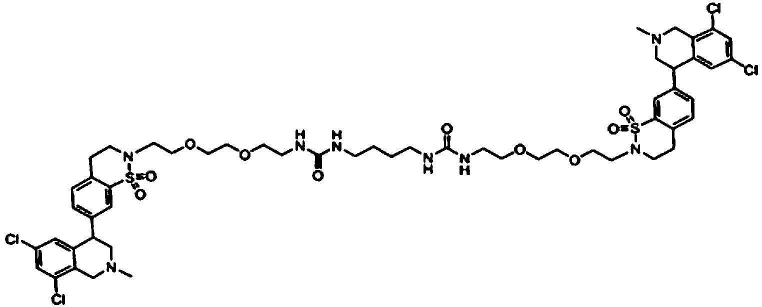 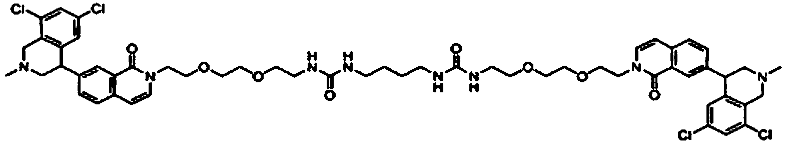 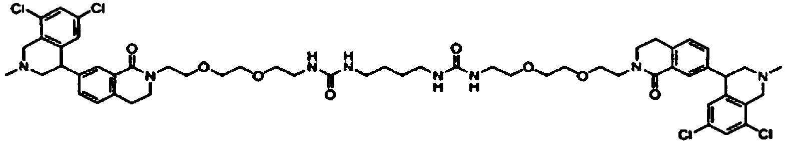 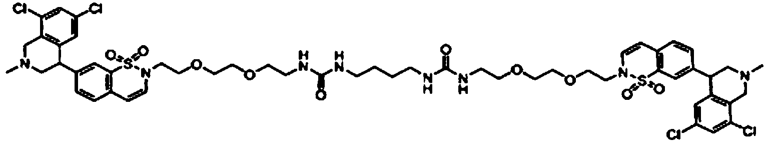 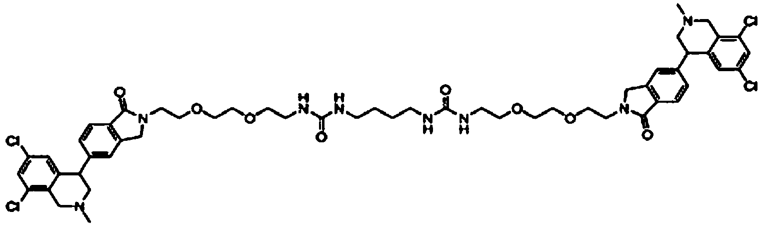 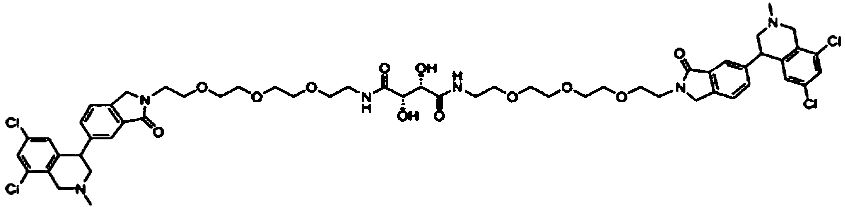 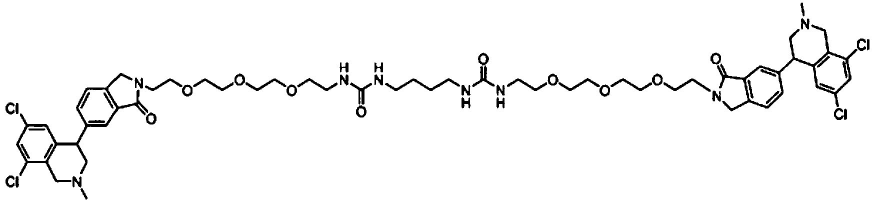 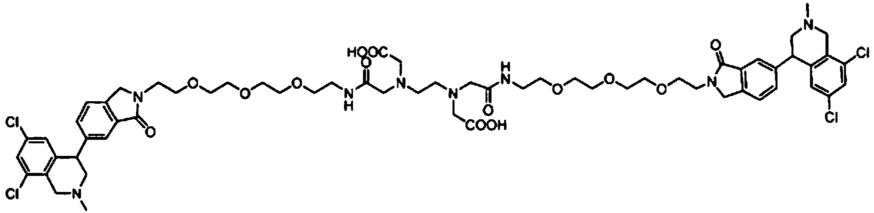 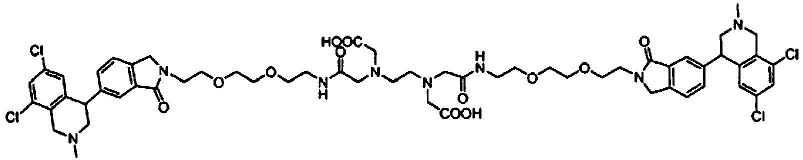 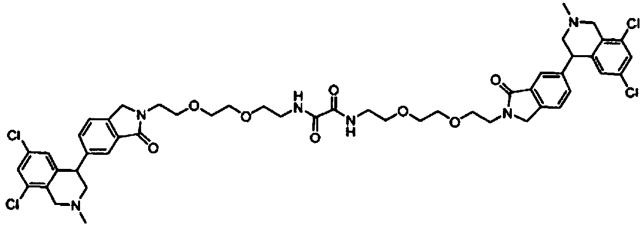 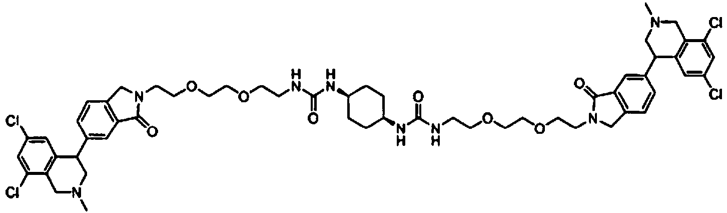 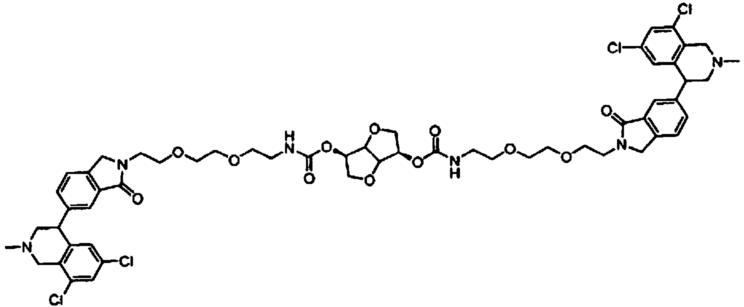 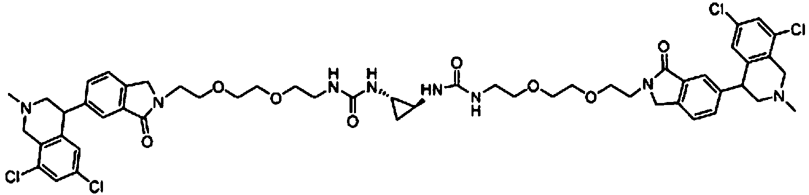 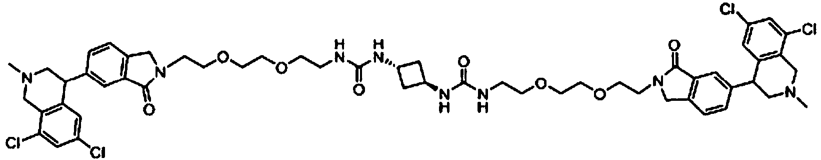 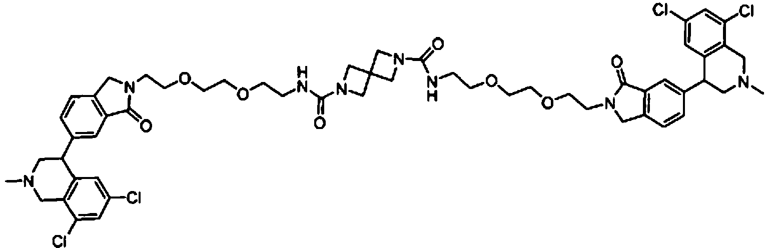 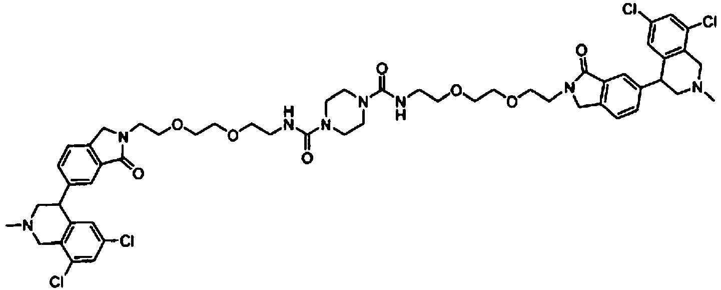 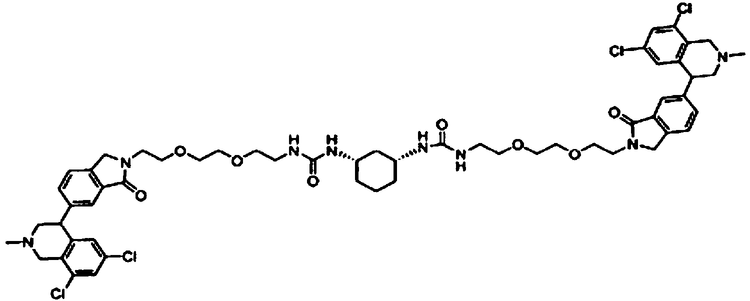 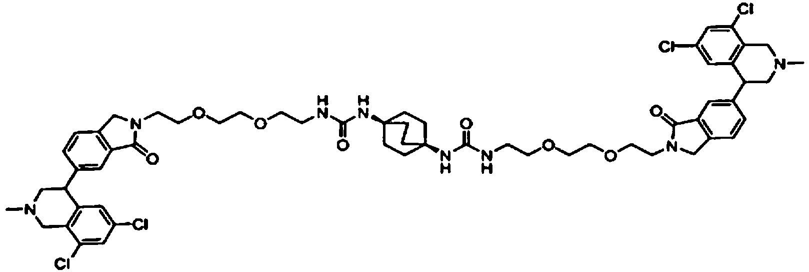 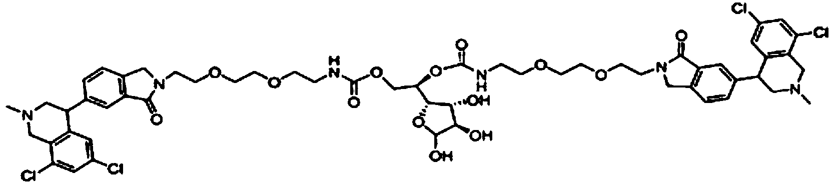 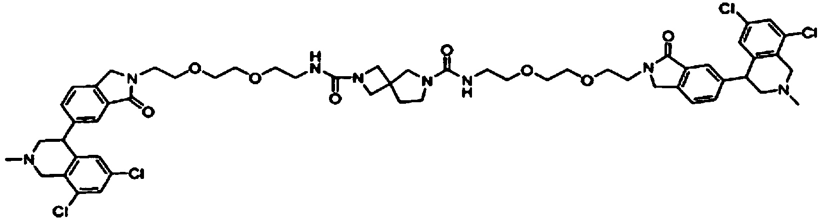 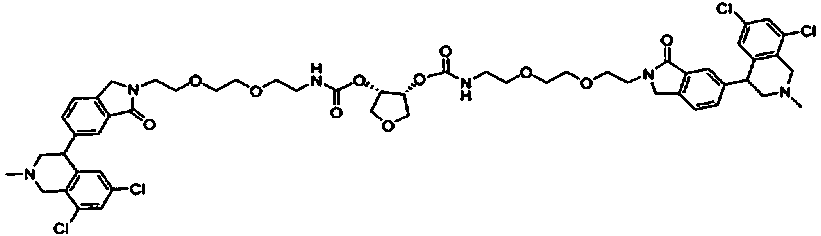 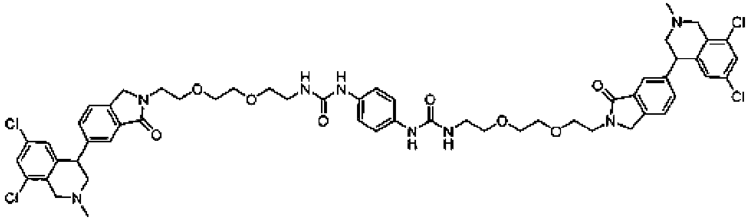 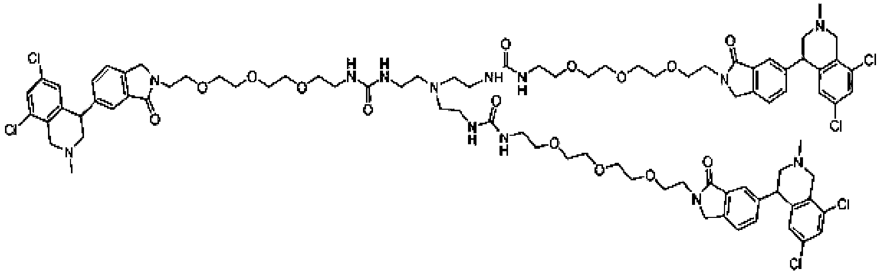 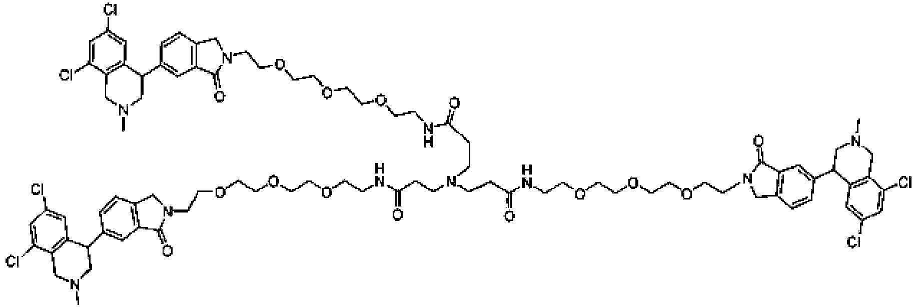 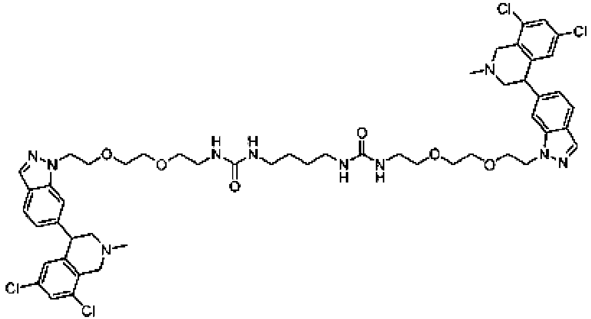 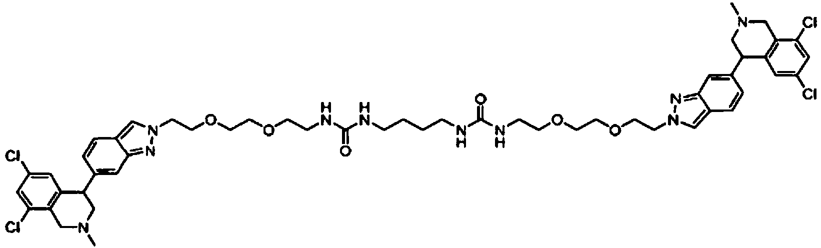 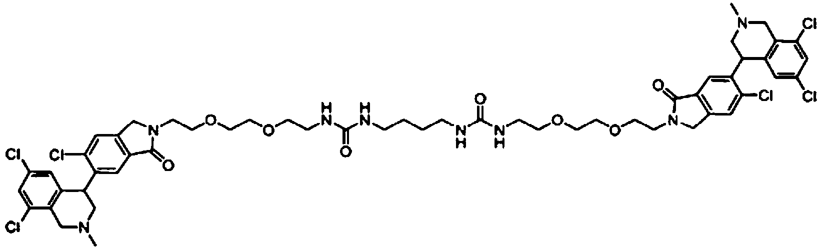 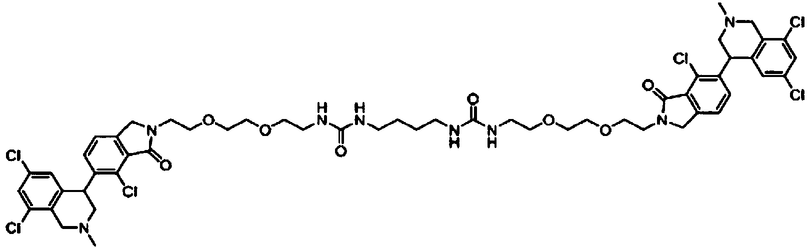 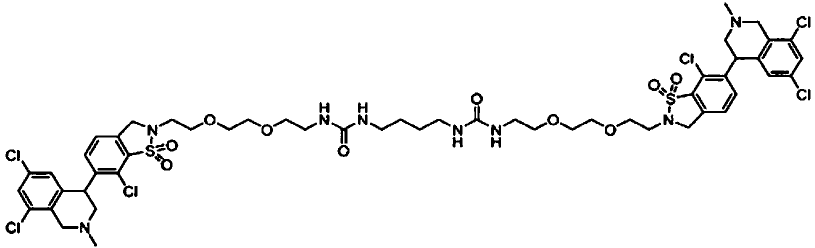 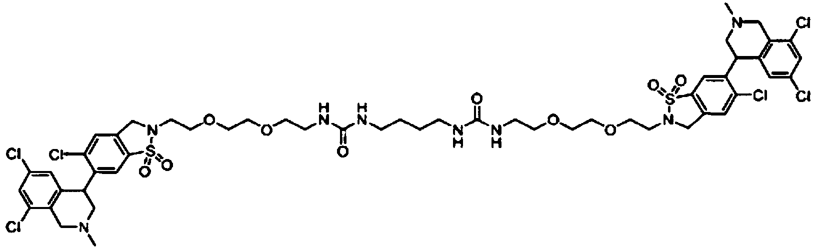 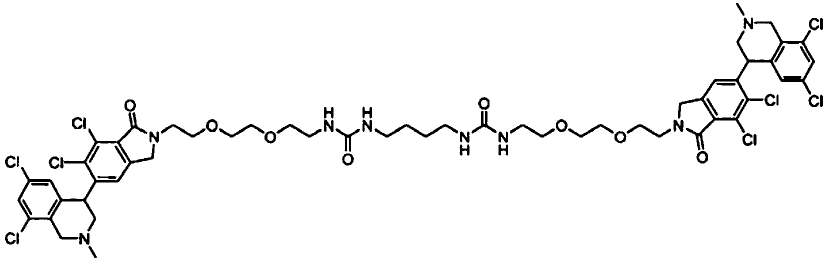 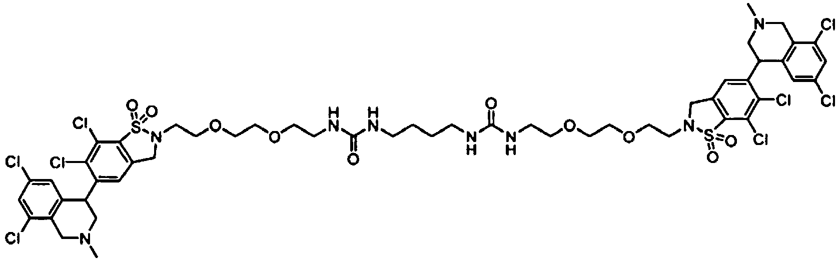 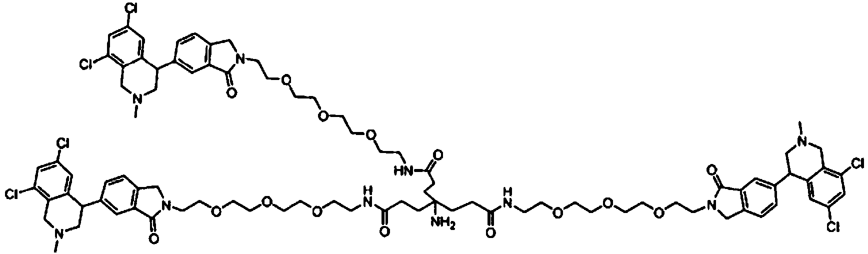 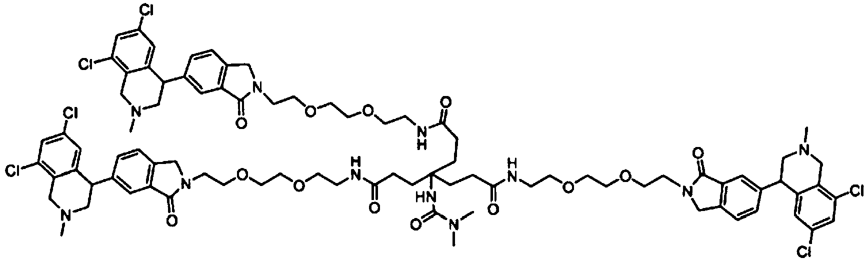 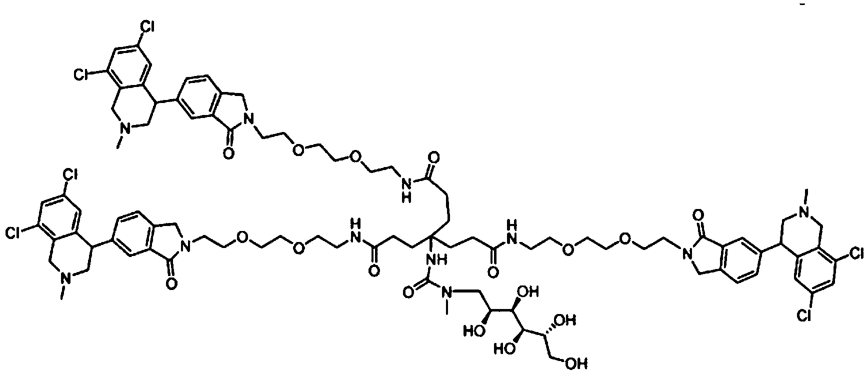 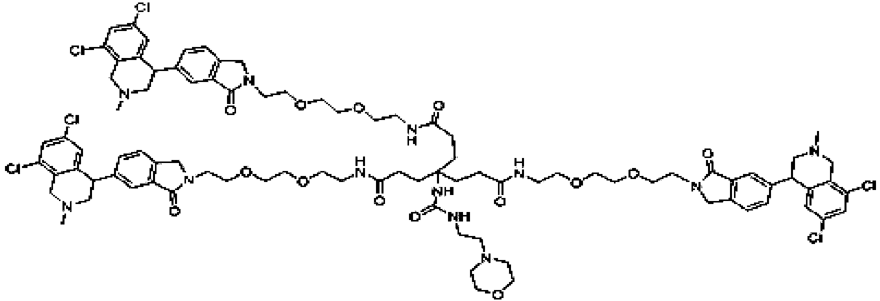 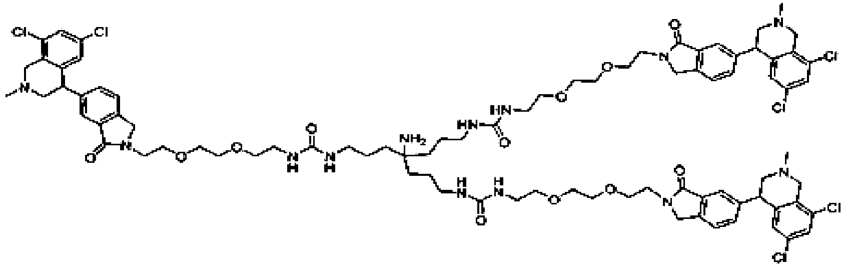 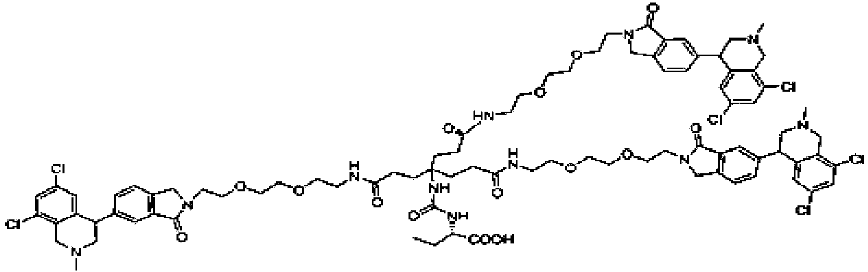 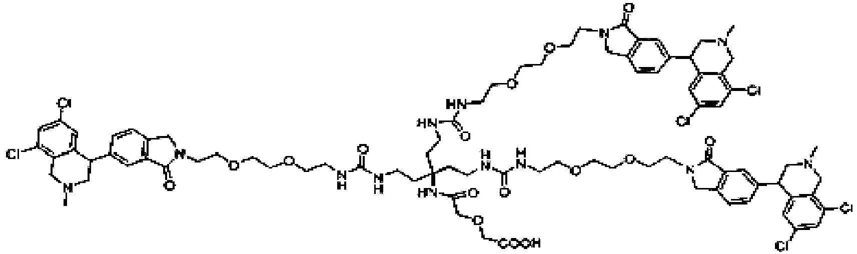 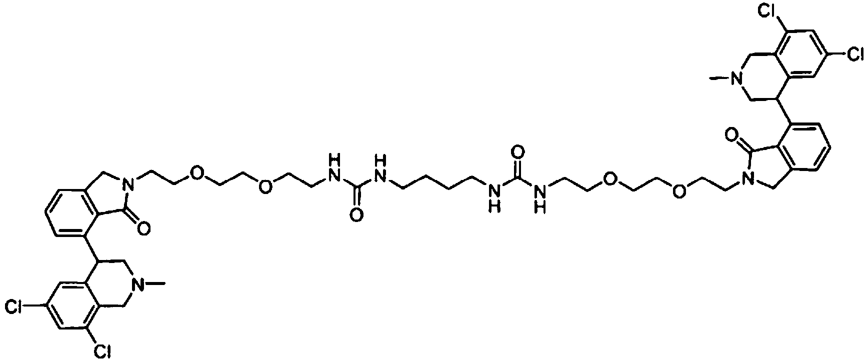 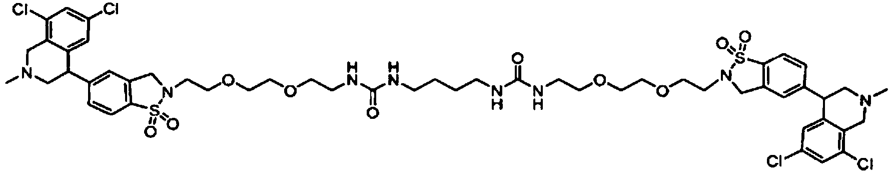 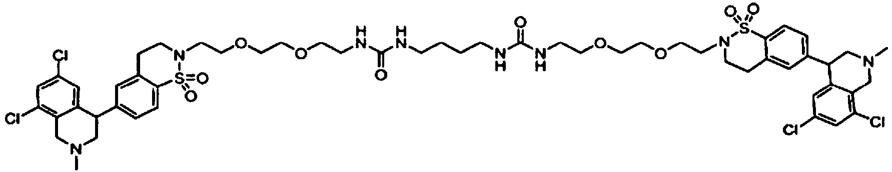 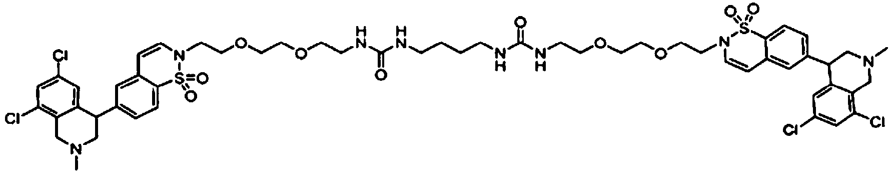 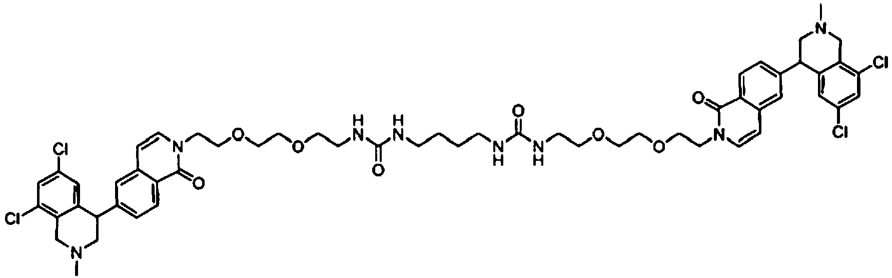 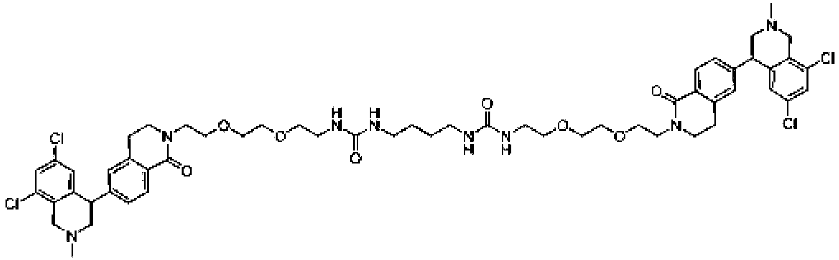 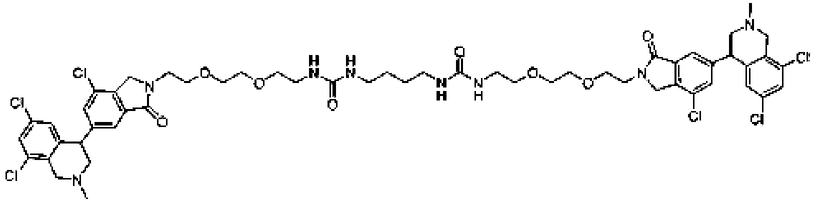 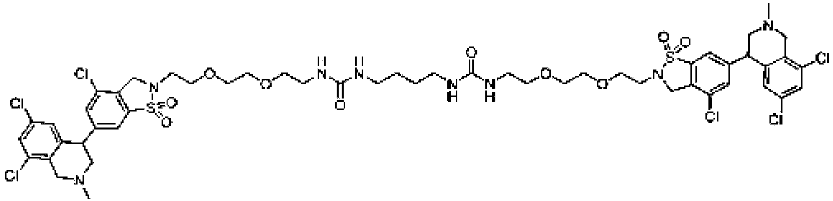 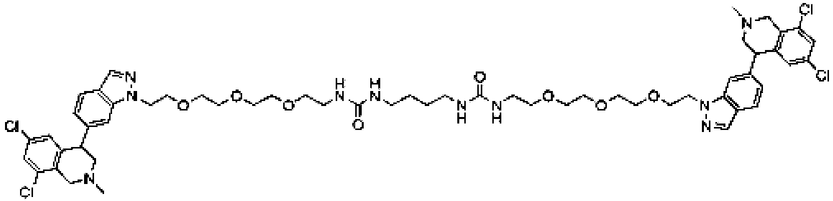 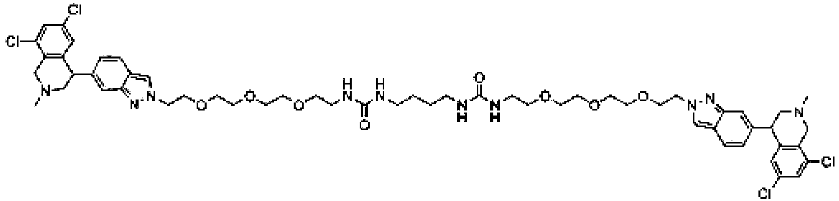 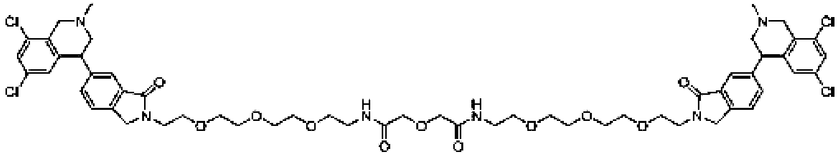 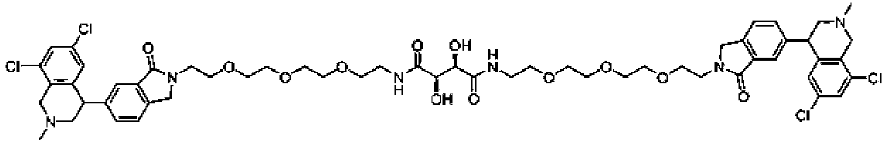 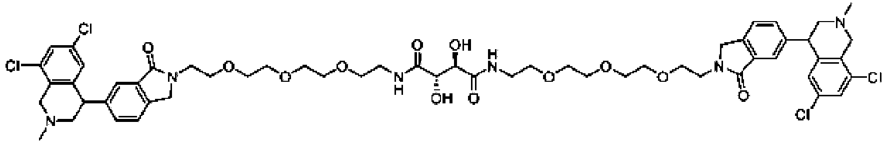 und

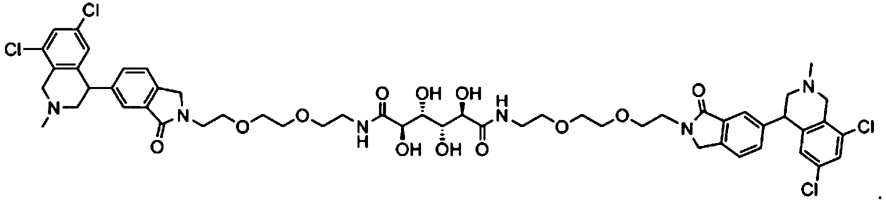

3. Verbindung, das Stereoisomer davon und das pharmazeutisch unbedenkliche Salz davon nach einem der Ansprüche 1 bis 2 zur Verwendung bei der Behandlung von Reizdarmsyndrom, Herzinsuffizienz, chronischer Nierenerkrankung, terminaler Nierenerkrankung oder Lebererkrankung.

## Revendications

1. Composé représenté par la formule (I), un stéréoisomère de celui-ci ou un sel pharmaceutiquement acceptable de celui-ci, 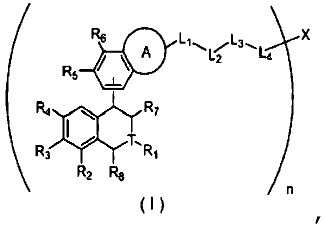 dans lequel,
R₁ est choisi parmi CH₃, 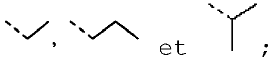
R₂, R₃, R₄ sont chacun indépendamment choisis parmi H, F, Cl, Br,
I, OH, NH₂, CN, CH₃, 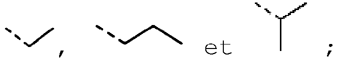
R₇, R₈ sont chacun indépendamment parmi H ;
T est choisi parmi N et CH ;
la partie structurale 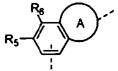 est sélectionnée parmi 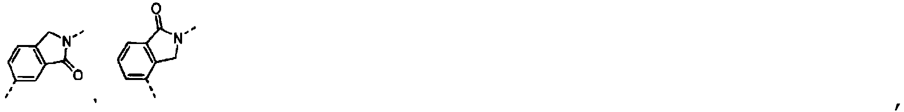 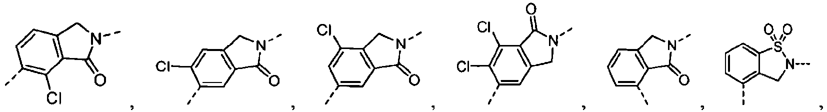 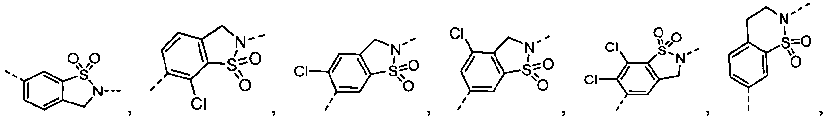 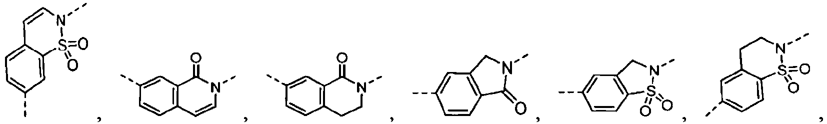 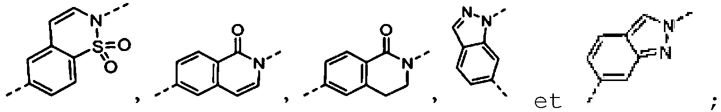
n est choisi parmi 2 et 3 ;
l'unité structurelle 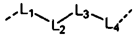 est sélectionnée parmi 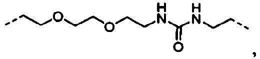 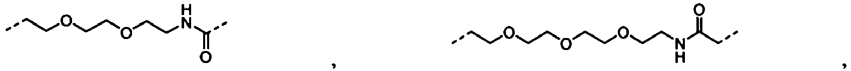 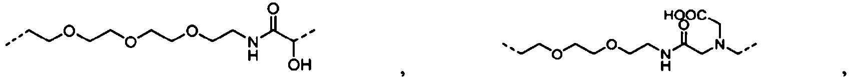 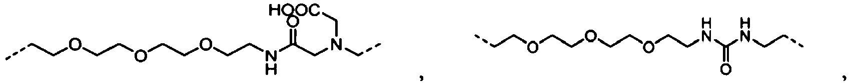 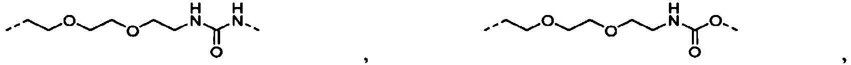 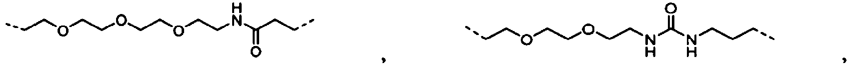 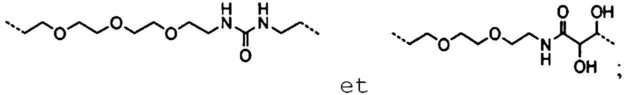
X est choisi parmi une liaison simple, N, NH, O,

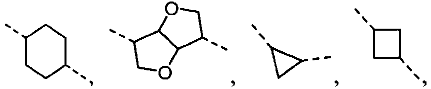

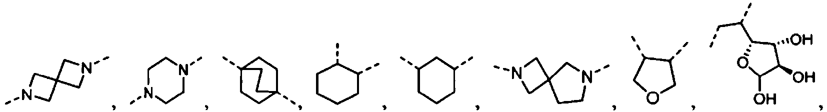

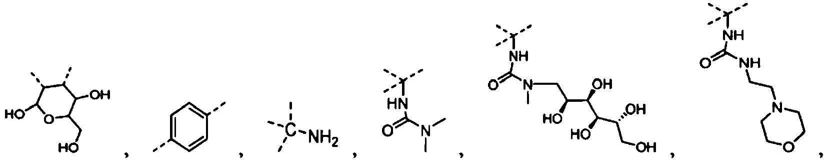

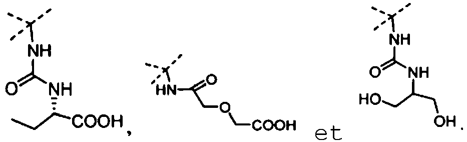

2. Composé, le stéréoisomère de celui-ci et le sel pharmaceutiquement acceptable de celui-ci selon la revendication 1, dans lequel, le composé est choisi parmi : 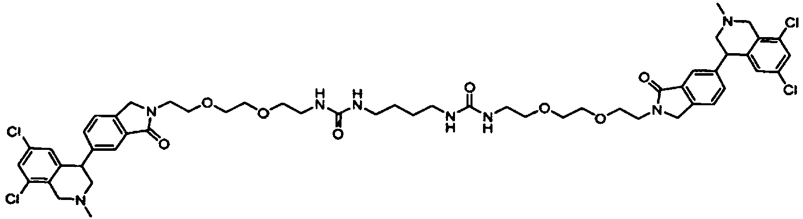 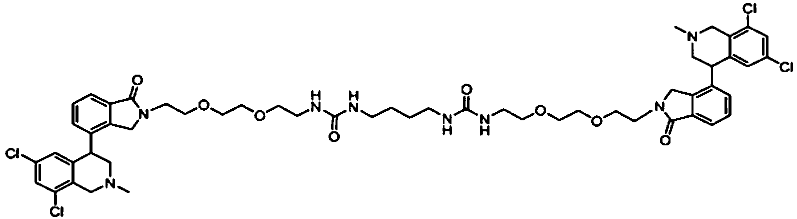 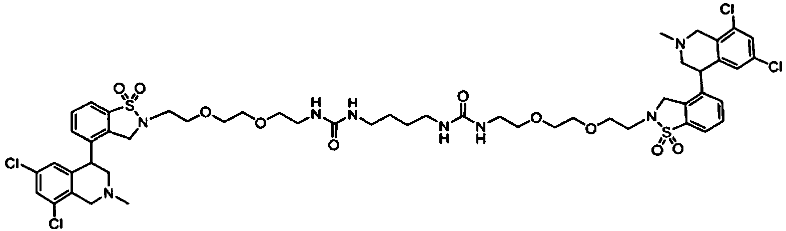 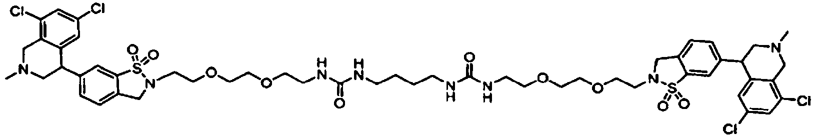 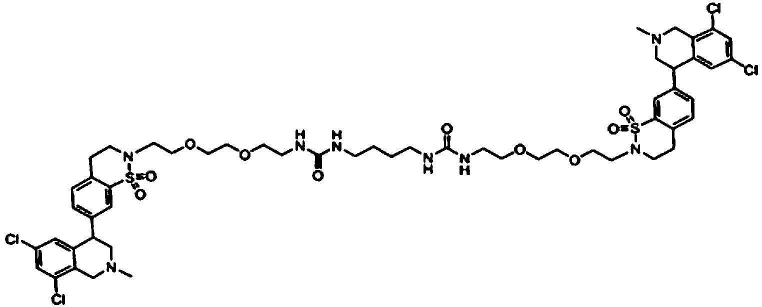 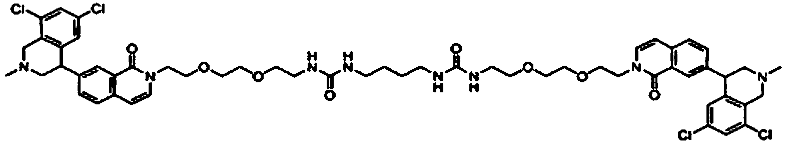 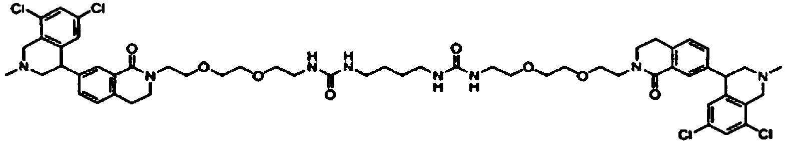 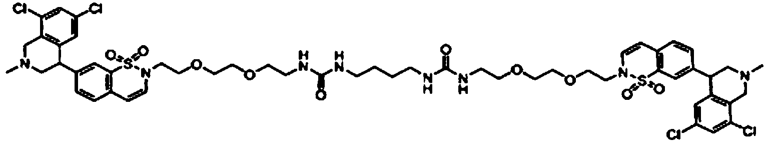 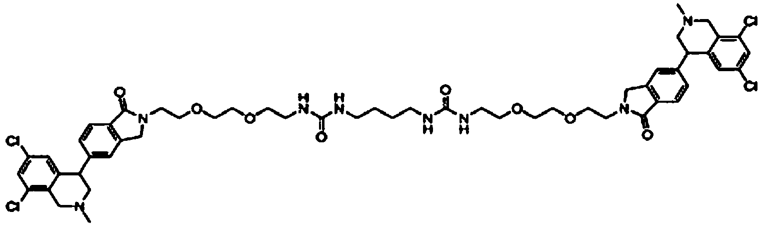 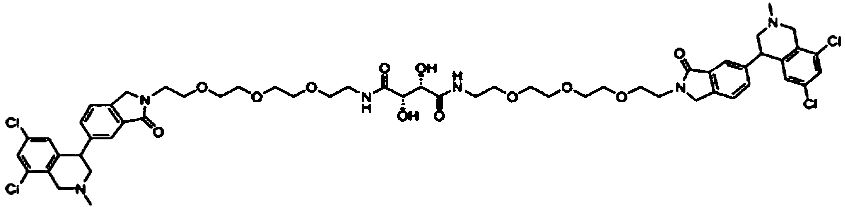 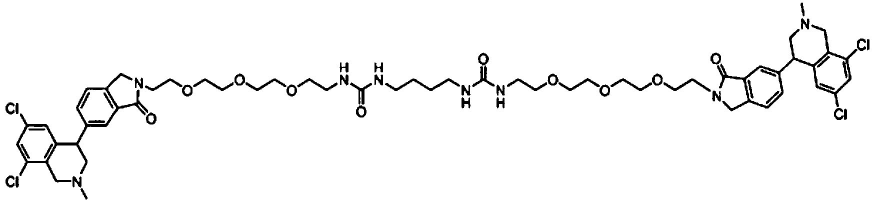 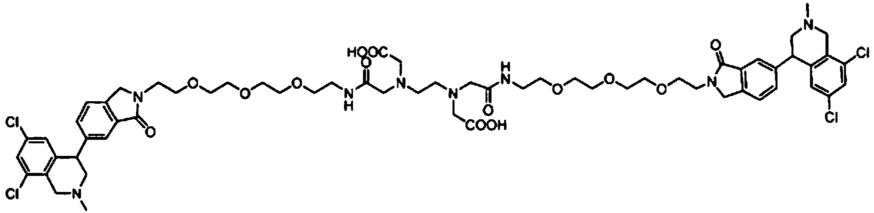 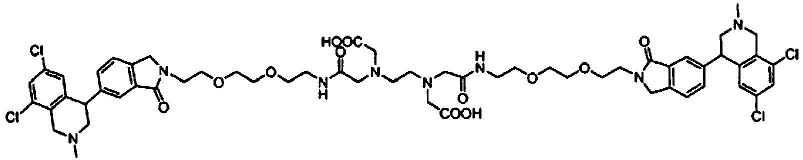 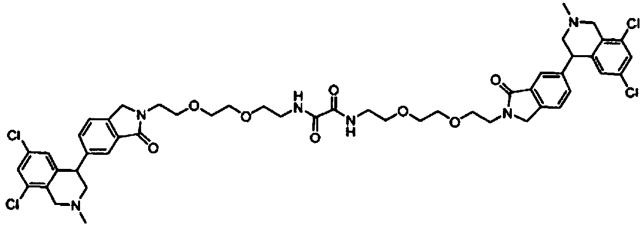 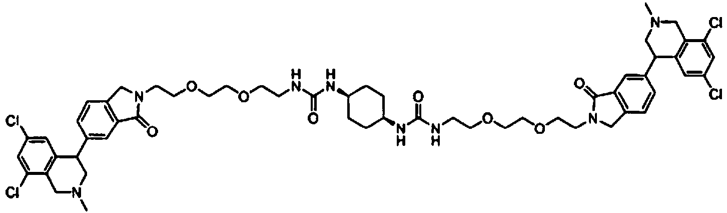 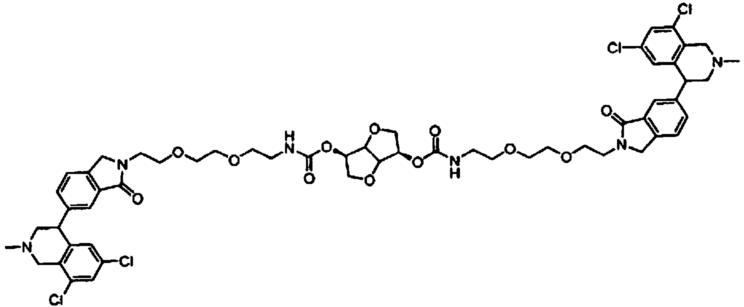 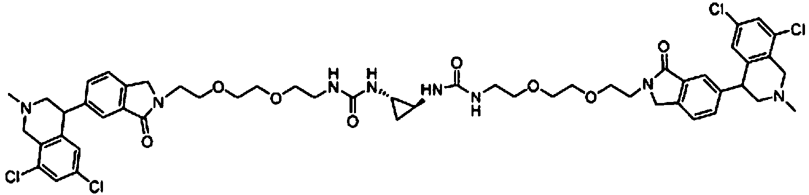 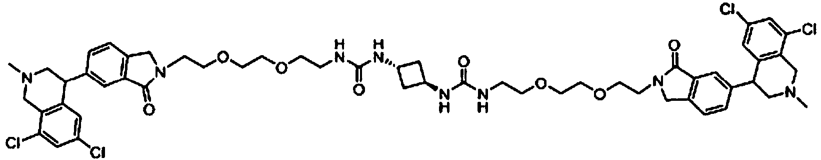 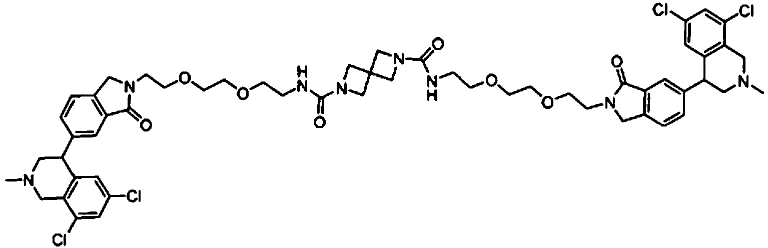 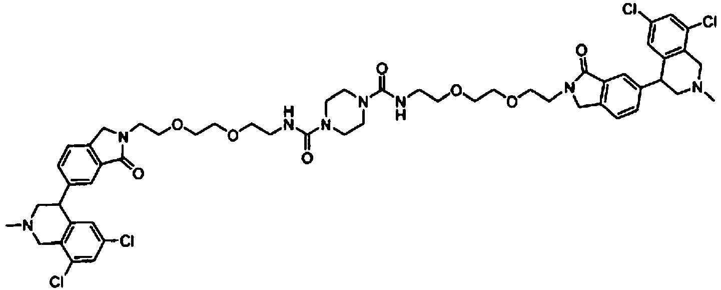 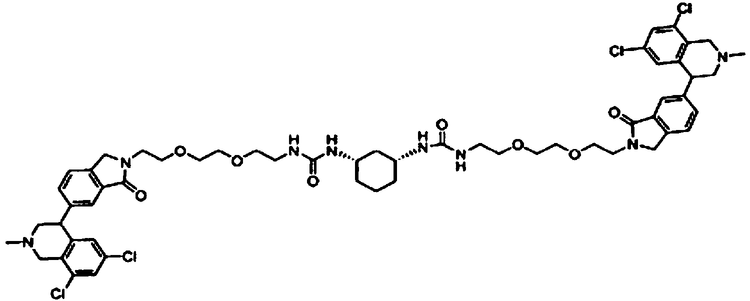 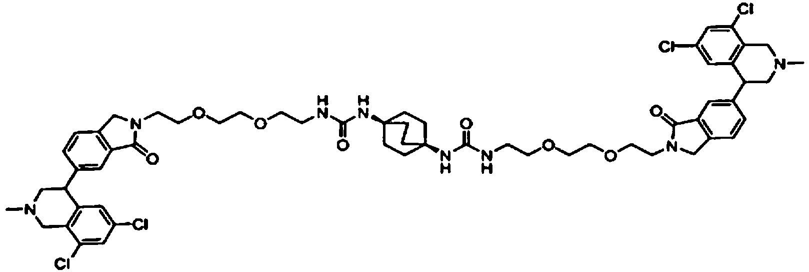 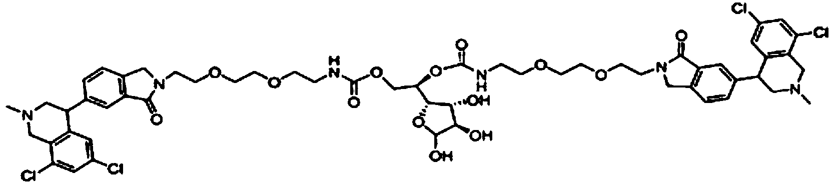 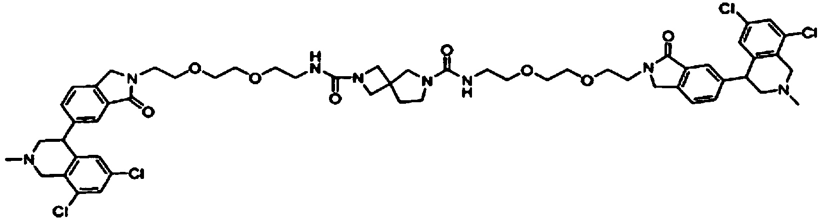 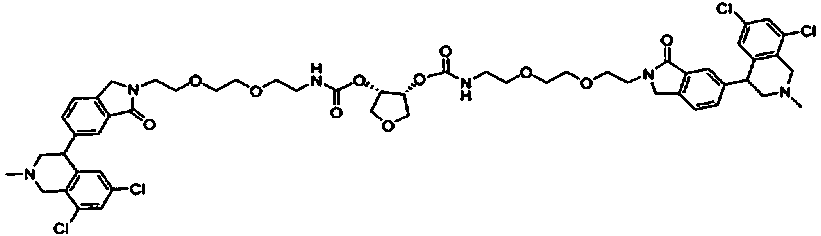 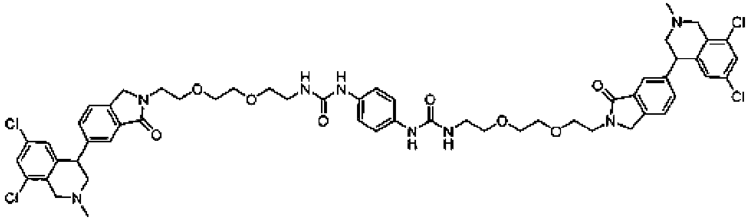 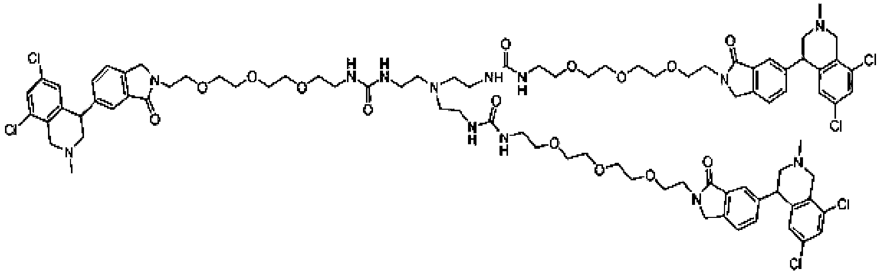 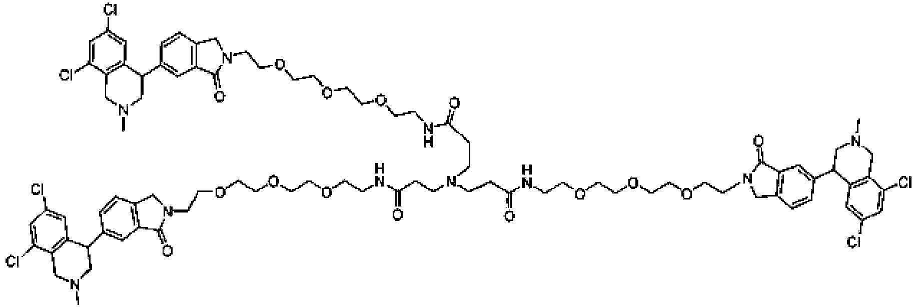 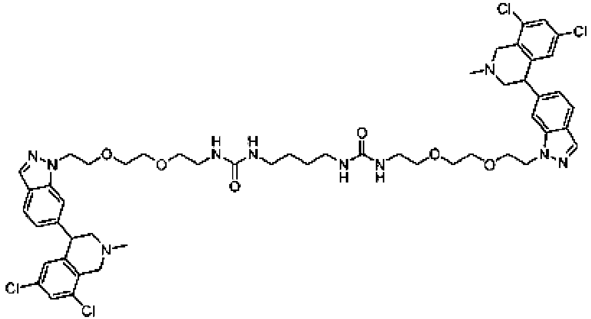 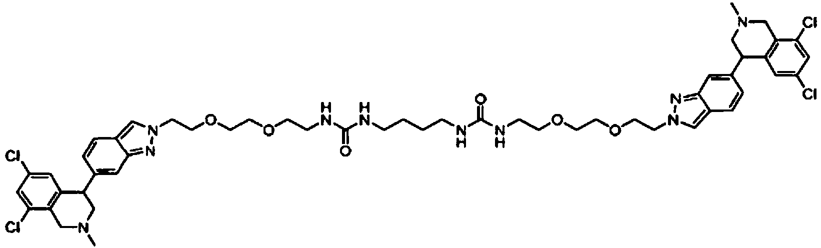 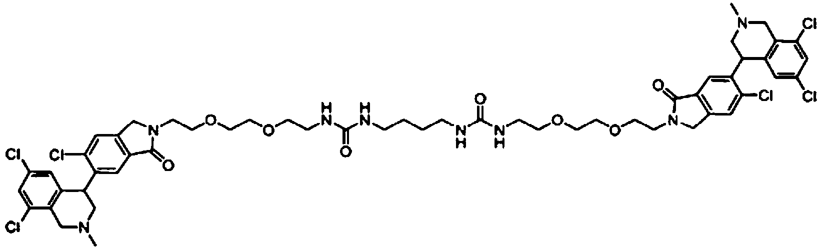 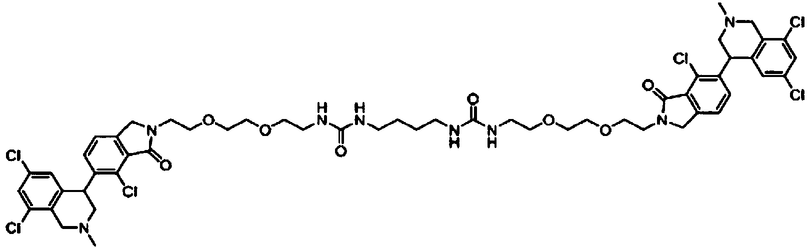 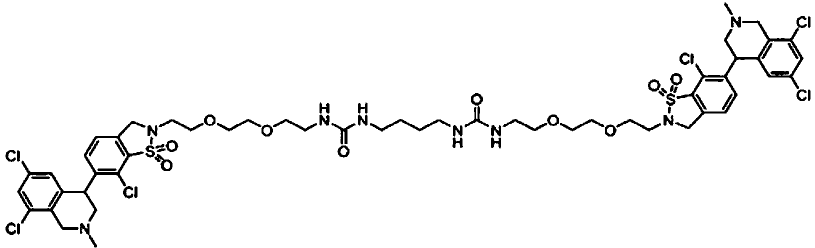 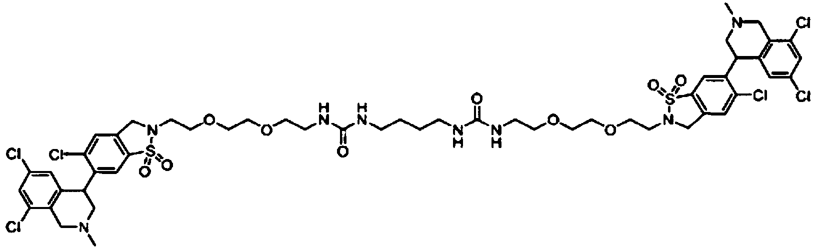 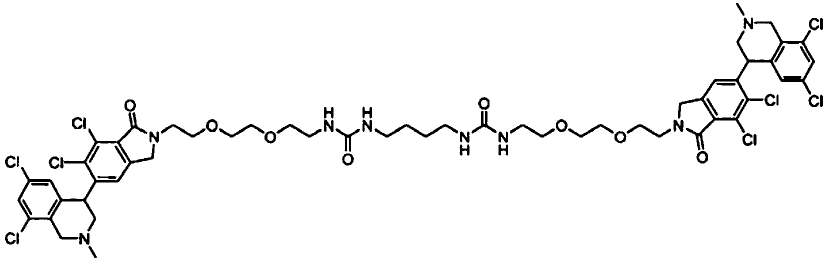 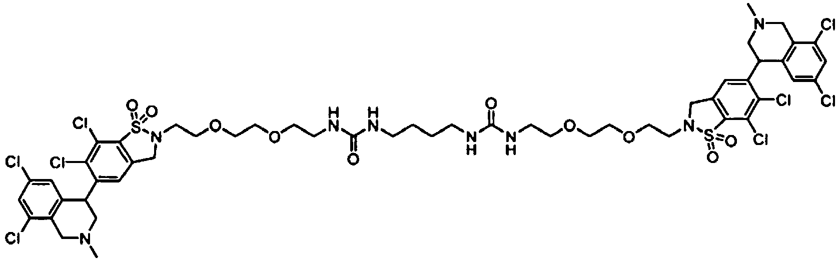 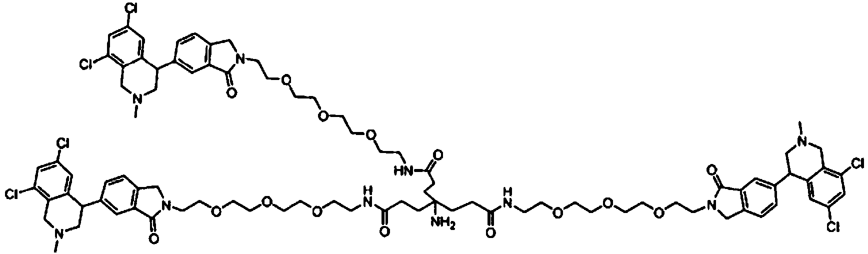 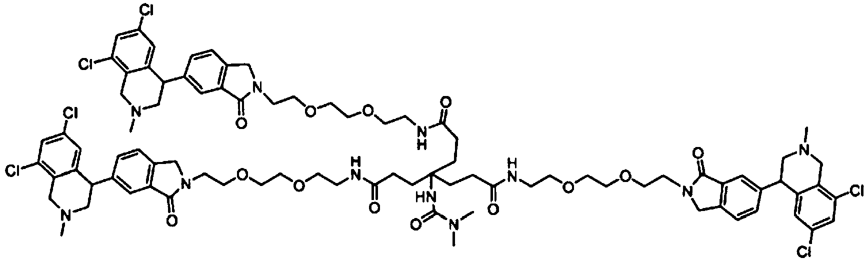 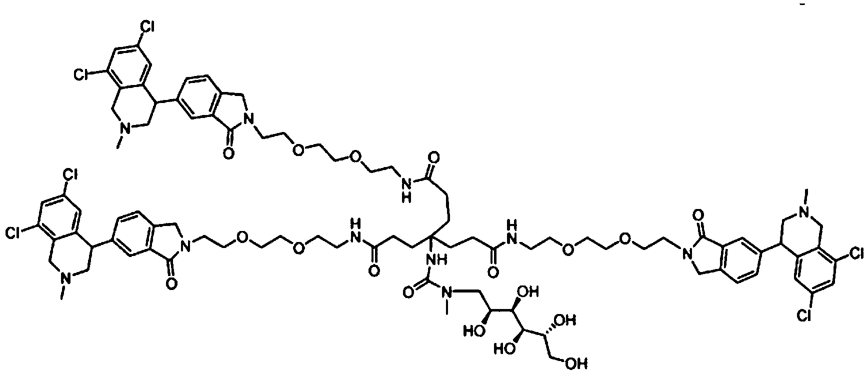 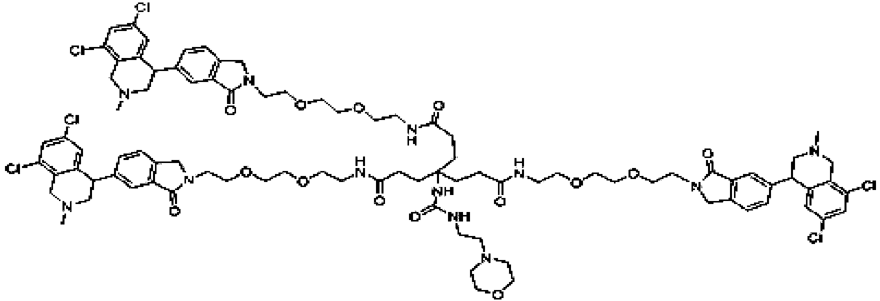 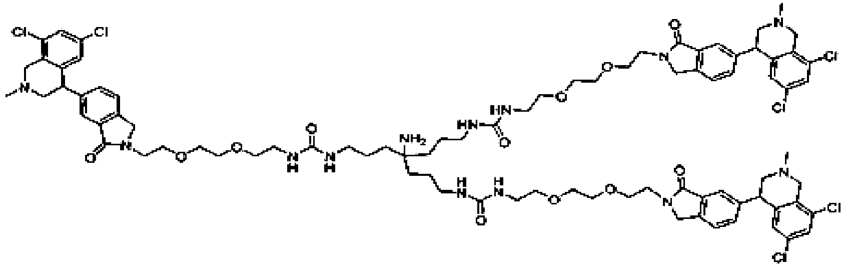 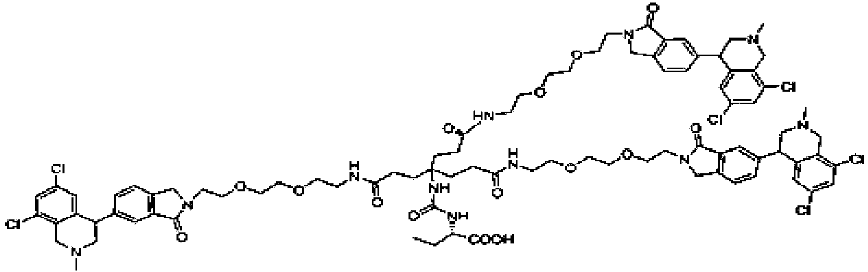 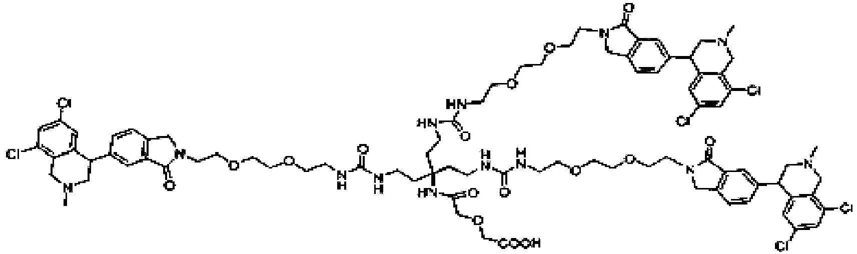 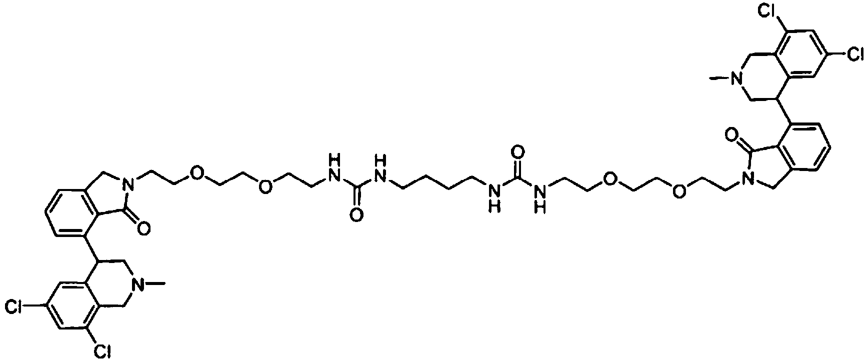 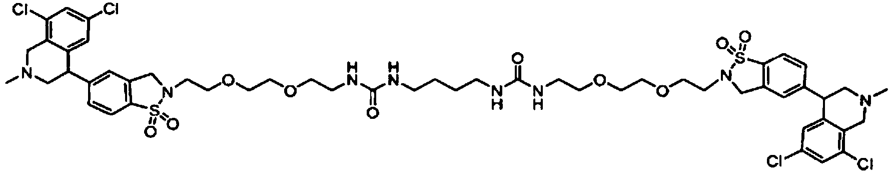 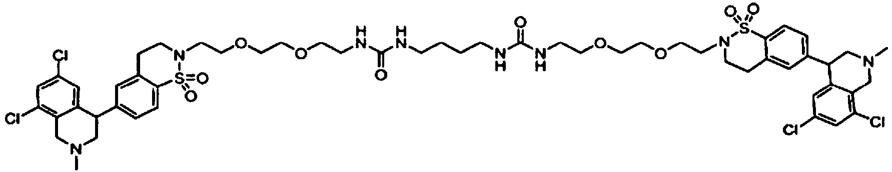 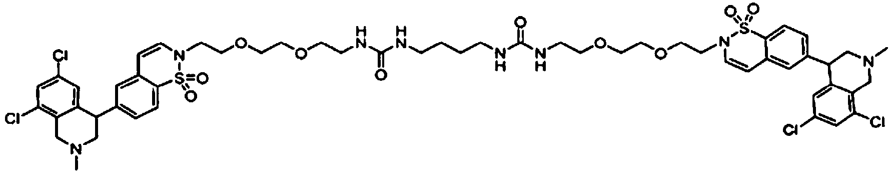 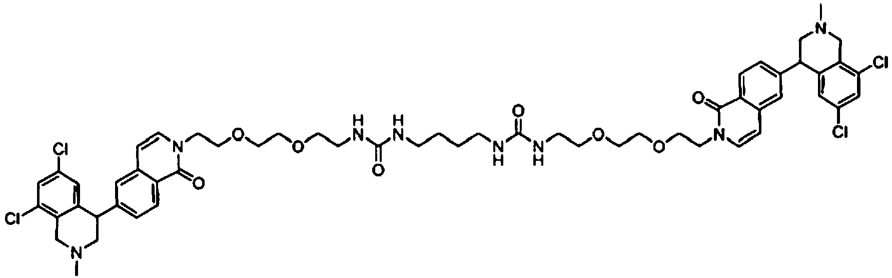 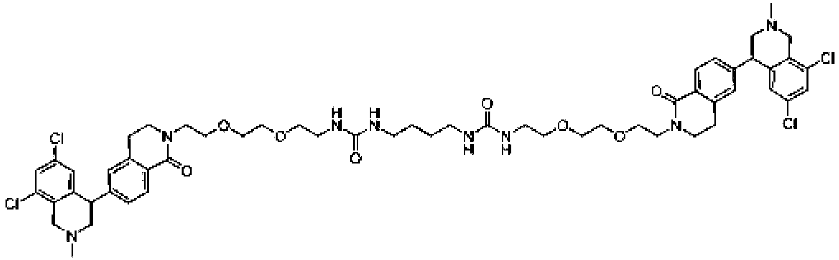 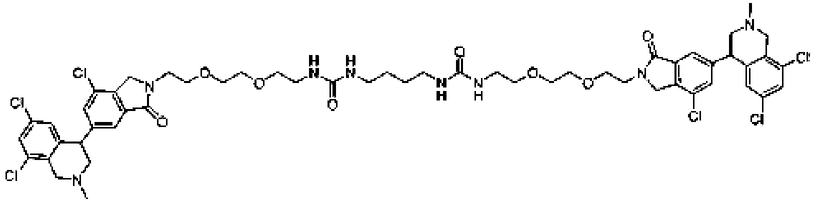 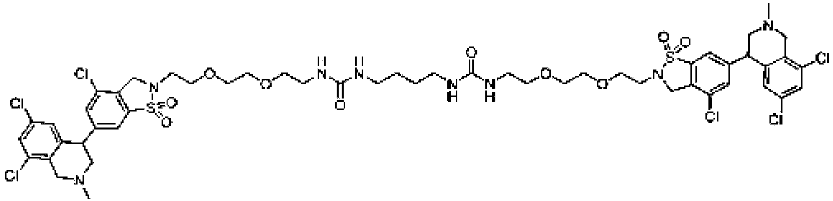 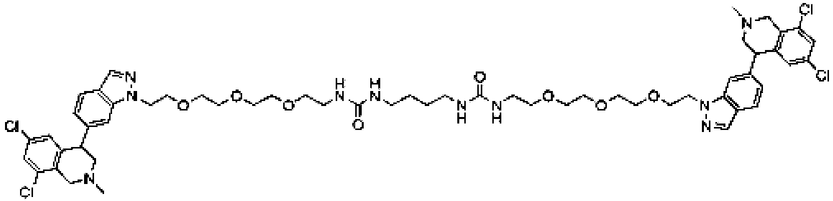 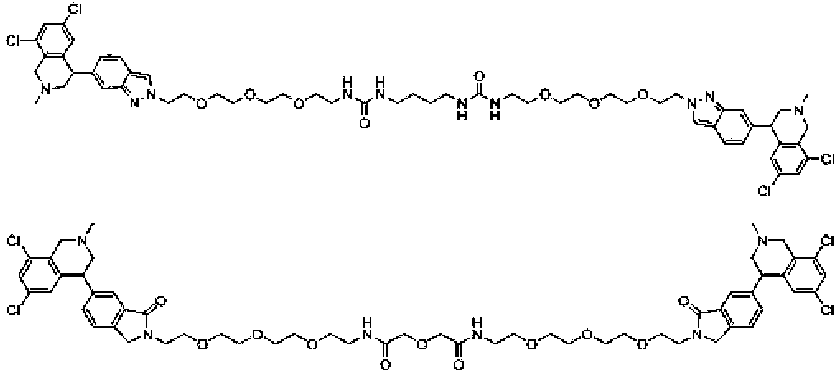 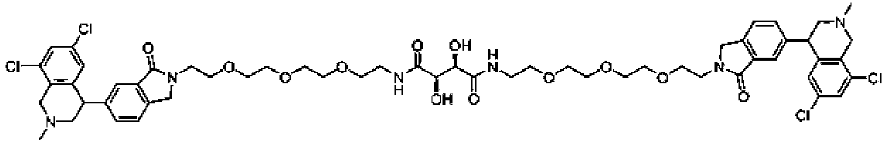 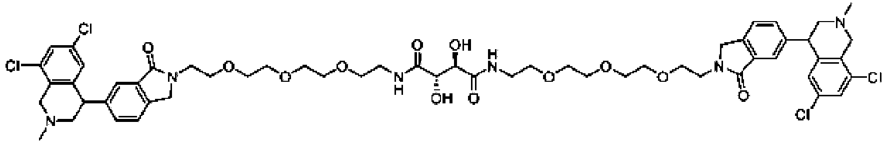 et

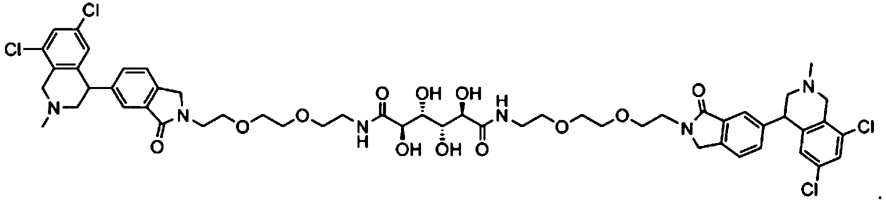

3. Composé, le stéréoisomère de celui-ci et le sel pharmaceutiquement acceptable selon l'une quelconque des revendications 1 et 2 pour être utilisé dans le traitement du syndrome du côlon irritable, de l'insuffisance cardiaque, de la maladie rénale chronique, de l'insuffisance rénale terminale ou de la maladie du foie.
